# EUROPEAN PATENT SPECIFICATION

(11) **EP 1 165 555 B1**
(45) Date of publication and mention of the grant of the patent: **16.07.2003**
(21) Application number: 00919382.2
(22) Date of filing: 10.03.2000
(51) Int. Cl.: C07D 471/00

(54) **ARYL FUSED 2,4-DISUBSTITUTED PYRIDINES: NK3 RECEPTOR LIGANDS**
ARYL-ANNELLIERTE DISUBSTITUIERTE PYRIDINE : NK3 REZEPTOR-LIGANDEN
PYRIDINES 2,4-DISUBSTITUEES FUSIONNEES AVEC UN ARYLE: LIGANDS DU RECEPTEUR NK-3

(30) Priority: 11.03.1999 US 123802 P
(43) Date of publication of application: 02.01.2002
(73) Proprietor: NEUROGEN CORPORATION, Branford, CT 06405 (US)
(72) Inventor: YUAN, Jun, Guilford, CT 06437 (US); MAYNARD, George, D., Clinton, CT 06413 (US); HUTCHISON, Alan, J., Madison, CT 06443 (US)
(74) Representative: Smaggasgale, Gillian Helen
(86) International application number: US0006371
(87) International publication number: WO00058307

(56) References cited:
- WO-A-97/19926
- GIARDINA, G. A. M. ET AL: "Replacement of the quinoline system in 2-phenyl-4- quinolinecarboxamide NK-3 receptor antagonists" FARMACO (1999), 54(6), 364-374 , XP000939369
- WAGNER G. ET AL: 'Synthese neuer Pyrido[3',2':4,5]thieno[3,2-d]1,2,3-triazin -Derivate als Antianaphylaktika' PHARMAZIE vol. 48, 1993, pages 514 - 518

## Description

### Field of the Invention

This invention relates to aryl fused 2,4-disubstituted pyridines that bind to cell surface receptors, particularly neurokinin-3 (NK-3) receptors. More specifically, the invention relates to such compounds that selectively bind to such receptors. This invention also relates to pharmaceutical compositions comprising such compounds. These novel NK-3 receptor ligands are useful in the treatment of a broad array of disorders and diseases of the central nervous system (CNS) and peripheral nervous systems in mammals that are associated with pathogenic NK-3 receptor activation.

### Description of the Related Art

The tachykinins represent a family of structurally related peptides originally isolated based upon their smooth muscle contractile and sialogogic activity. These mammalian peptides include substance P (SP), neurokinin A (NKA) and neurokinin β (NKB). Tachykinins are synthesized in the central nervous system (CNS), as well as in peripheral tissues, where they exert a variety of biological activities. Substance P can be produced from three different mRNAs (α-, β- and γ-preprotachykinin mRNAs) that arise from a single gene as a result of alternative RNA splicing, whereas NKA can be generated from either the β- or the γ-preprotachykinin mRNA through posttranslationally processed precursor polypeptides. These precursors can also be differentially processed so that amino terminally extended forms of NKA (known as neuropeptide K and neuropeptide γ) are produced. NKB is produced from a separate mRNA arising from a second gene known as preprotachykinin B.

Three receptors for the tachykinin peptides have been moleculary characterized and are referred to as neurokinin-1 (NK-1), neurokinin-2 (NK-2) and neurokinin-3 (NK-3) receptors. The NK-1 receptor has a natural agonist potency profile of SP>NKA>NKB. The NK-2 receptor agonist potency profile is NKA>NKB>SP, and the NK-3 receptor agonist potency profile is NKB>NKA>SP. These receptors mediate the variety of tachykinin-stimulated biological effects that generally include 1) modulation of smooth muscle contractile activity, 2) transmission of (generally) excitatory neuronal signals in the CNS and periphery (e.g. pain signals), 3) modulation of immune and inflammatory responses, 4) induction of hypotensive effects via dilation of the peripheral vasculature, and 5) stimulation of endocrine and exocrine gland secretions. These receptors transduce intracellular signals via the activation of pertussis toxin-insensitive (G_{αq/11}) G proteins, resulting in the generation of the intracellular second messengers inositol 1,4,5-trisphosyphate and diacylglycerol. NK-1 receptors are expressed in a wide variety of peripheral tissues and in the CNS. NK-2 receptors are expressed primarily in the periphery, while NK-3 receptors are primarily (but not exclusively) expressed in the CNS. Recent work confirms the presence of NK-3 receptor binding sites in the human brain.

Studies measuring the localization of NKB and NK-3 receptor mRNAs and proteins, along with studies performed using peptide agonists and non-peptide NK-3 receptor antagonists, provide a rationale for using NK-3 receptor antagonists in treating a variety of disorders in both the CNS and the periphery. In the CNS, activation of NK-3 receptors has been shown to modulate dopamine and serotonin release, indicating therapeutic utility in the treatment of a variety of disorders including anxiety, depression, schizophrenia and obesity. Further, studies in primate brain detect the presence of NK-3 mRNA in a variety of regions relevant to these disorders. With regard to obesity, it has also been shown that NK-3 receptors are located on MCH-containing neurons in the rat lateral hypothalamus and zona incerta. In the periphery, administration of NKB into the airways is known to induce mucus secretion and bronchoconstriction, indicating therapeutic utility for NK-3 receptor antagonists in the treatment of patients suffering from airway diseases such as asthma and chronic obstructive pulmonary disease (COPD). Localization of NK-3 receptors in the gastrointestinal (GI) tract and the bladder indicates therapeutic utility for NK-3 receptor antagonists in the treatment of GI and bladder disorders including inflammatory bowel disease and urinary incontinence.

Both peptide and nonpeptide antagonists have been developed for each of the tachykinin receptors. The first generation of peptide antagonists for the tachykinin receptors had problems with low potency, partial agonism, poor metabolic stability and toxicity, whereas the current generation of non-peptide antagonists display more drug-like properties. Unfortunately, previous non-peptide NK-3 receptor antagonists suffer from a number of problems such as species selectivity (which limits the potential to evaluate these compounds in many appropriate disease models). New non-peptide NK-3 receptor antagonists are therefore being sought, both as therapeutic agents and as tools to further investigate the anatomical and ultrastructural distribution of NK-3 receptors, as well as the physiologic and pathophysiologic consequences of NK-3 receptor activation.

Examples of alternative aryl-fused compounds can be found in WO 97/ 19926 A.

### SUMMARY OF THE INVENTION

This invention provides novel compounds of Formula I (below), which interact with the NK3 binding site (the NK-3 receptor). The invention provides pharmaceutical compositions containing compounds described by Formula I. These novel tachykinin NK-3 receptor ligands act as receptor antagonists. As such, they useful in the treatment of a broad array of disorders and diseases of the central nervous system (CNS) and periphery in mammals in which pathogenic activation of NK-3 receptors may occur. These include anxiety, panic disorder, depression, psychosis, obsessive compulsive disorder, dementia, Huntington's disease, schizophrenia, stress related somatic disorders, reflex sympathetic dystrophy, dysthmic disorders, Parkinson's disease, movement disorders, obesity, eating disorders, addiction, convulsive disorders such as epilepsy, neurodegenerative diseases such as Alzheimer's disease, Multiple sclerosis and other demyelinating diseases, AIDS related neuropathy, chemotherapy-induced neuropathy and neuralgia, diabetic or peripheral neuropathy, neurogenic inflammation, inflammatory pain and other types of chronic or acute pain, migraine, Reynaud's disease, vasodilation, vasospasm, angina, asthma, chronic obstructive pulmonary diseases, airway hyperreactivity, cough, allergic rhinitis, bronchospasm, bronchopneumonia, ocular inflammation, inflammatory bowel disease, Crohn's disease, ulcerative colitis, skin disorders and itch, hypersensitivity disorders, atopic dermatitis, contact dermatitis, cutaneous wheal and flare, psoriasis, renal disorders, urinary incontinence, immune system disorders and adverse immunological reactions, fibrositis, osteoarthritis, eosinophilic fascioliasis, and scleroderma.

These compounds are also useful for the diagnosis of disorders involving activation of tachykinin NK-3 receptors and as probes for detecting NK-3 receptors in cultured cells and tissue samples.

Accordingly, a broad embodiment of the invention is directed to compounds of Formula I: 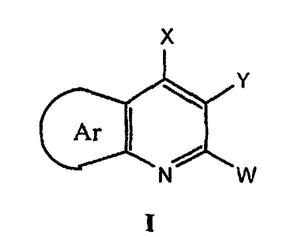 and the pharmaceutically acceptable non-toxic salts or pharmaceutically acceptable solvates thereof wherein:
Y is
   hydrogen, straight or branched chain lower alkyl having 1-6 carbon atoms, halogen, amino, hydroxyl, or lower alkoxy having 1-6 carbon atoms; or
Y is
   straight or branched chain lower alkyl having 1-6 carbon atoms or lower alkoxy having 1-6 carbon atoms which is substituted on the alkyl chain with an amino or mono or dialkylamino group;
W is
   phenyl, thienyl, or pyridyl, each of which may be mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms; or
W is
   phenyl, thienyl or pyridyl straight or branched chain lower alkyl having 1-6 carbon atoms with all aryl groups being either unsubstituted or mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms; or
W is
   piperidino, morpholino, thiomorpholino, pyrrolindino, piperazino, homopiperazino, azabicyclo[3.2.2]nonano, isoindolino, as well as any other nitrogen-containing heterocycle attached on nitrogen so as to form a tertiary amine in structure I wherein the heterocycle is unsubstituted, mono or disubstituted with alkyl or aryl groups, or fused to an aromatic ring; or
W is
   nitrogen that is mono or disubstituted with a hydrogen, a straight or branched alkyl chain consisting of 1 to 8 carbons, a cycloalkyl containing 3 to 8 carbon atoms, an alkyl chain having 1to 6 carbon atoms that is attached at any position to an aryl ring, an alkyl chain having 1 to 6 carbon atoms that is attached at any position to a cycloalkyl containing 3 to 8 carbon atoms, phenyl, thienyl, pyridyl, imidazolyl, benzimidazolyl, or any heterocycle having aromatic character; or
W is
   oxygen that is substituted,with a straight or branched alkyl chain having 1 to 8 carbon atoms, a cycloalkyl having 3 to 8 carbon atoms, or a phenyl ring that is mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms; 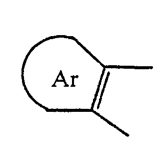
represents 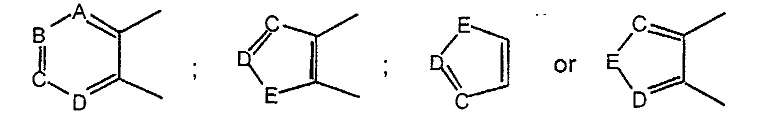 wherein:
A represents nitrogen or C-R₁
B represents nitrogen or C-R₂;
C represents nitrogen or C-R₃;
D represents nitrogen or C-R₄ with the proviso that if all four of A, B, C and D are present, at least one but not more than 2 of the group A, B, C, D are nitrogen; and
E represents oxygen, sulfur or N-R₅;
R₁ through R₄ are the same or different and represent
   hydrogen, halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms:
R₅ is
   hydrogen or straight or branched chain lower alkyl having 1-6 carbon atoms, or phenyl, thienyl, or pyridyl, each of which may be mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms;
X is 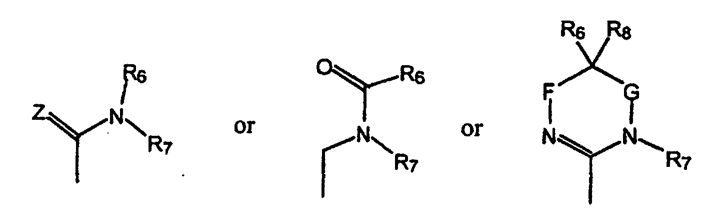
F is
   carbonyl, methyene or ethylene
G is
   methylene or a carbon-carbon single bond with the proviso that G is a carbon-carbon single bond when F is ethylene
Z is
   oxygen, sulfur, N-C≡N, or two hydrogen atoms;
R₆ is
   phenyl, thienyl, or pyridyl, each of which may be mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms; or
R₆ is
   straight or branched alkyl consisting of 1-8 carbons or cycloalkyl containing 3 to 8 carbon atoms wherein these groups may be mono or disubstituted with an aromatic ring such as phenyl or pyridyl and the aromatic groups can be mono or disubstituted with halogen, cyano, trifluoromethyl, trifluoromethoxy, hydroxy, straight or branched chain lower alkyl having 1-6 carbon atoms, amino, mono or disubstituted amino where the substituent on nitrogen is either a straight or branched chain having 1-6 carbon atoms, or straight or branched chain lower alkoxy having 1-6 carbon atoms; or
R₆ is
   is cycloalkyl containing 5 to 8 carbon atoms wherein the cycloalkyl is fused to an aromatic ring at adjacent positions;
R₇ and R₈ are the same or different and represent
   hydrogen or straight or branched chain lower alkyl having 1-6 carbon atoms; or
R₆ and R₇ taken together may form a 5, 6, 7 or 8 membered ring which is mono or disubstituted with an aryl group or contains a fused aryl ring as part of the 5,6, 7 or 8 membered ring described above with the proviso that R₆ and R₇ taken together do not form a ring when G is a methylene; or
R₆ and R₈ taken together may form a 5, 6, 7 or 8 membered ring which is mono or disubstituted with an aryl group or contains a fused aryl ring as part of the 5,6, 7 or 8 membered ring.

Compounds of formula I may have one or more asymmetric centers and may therefore exist in more than one stereoisomeric form. All stereoisomeric forms and mixtures thereof are encompassed in this application.

This invention also includes methods for using compounds of formula I to treat diseases and disorders in mammals in which activation of NK-3 receptors is of importance.

These compounds are highly selective agonists or, preferably, antagonists at NK3 receptors.

### DETAILED DESCRIPTION OF THE INVENTION

The novel compounds encompassed by the instant invention are described by general formula I as set forth above. Specific compounds of the invention include those of Formula IIa: 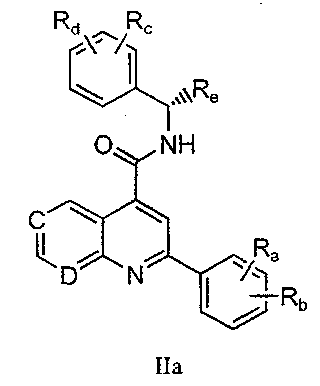 where
C and D are independently CH or N, provided that not both C and D are N simultaneously;
Rₐ and R_{b} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl;
R_{c} and R_{d} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl; and
Rₑ is C₁-C₆ alkyl, preferably C₁-C₄ alkyl, more preferably methyl, ethyl or propyl.

Preferred compounds of Formula IIa are those where D is CH, C is nitrogen, and Rₐ, R_{b}, R_{c}, and R_{d} are hydrogen. Other preferred compounds of of Formula IIa are those where C is CH, D is nitrogen, Rₐ and R_{b} are hydrogen and Rₑ is ethyl. Other preferred compounds of Formula IIa are those where both C and D are CH, Rₐ and R_{b} are hydrogen and R_{c} is ethyl.

Further specific compounds of the invention include those of Formula IIb: 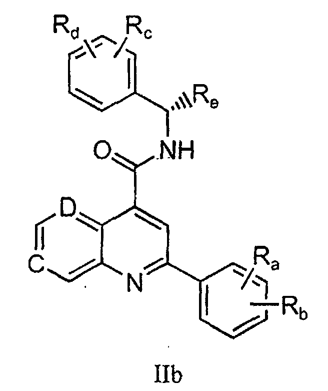 where
C and D are independently CH or N, provided that not both C and D are N simultaneously;
Rₐ and R_{b} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl;
R_{c} and R_{d} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl; and
Rₑ is C₁-C₆ alkyl, preferably C₁-C₄ alkyl, more preferably methyl, ethyl or propyl.

Preferred compounds of Formula IIb are those where D is CH, C is nitrogen, and Rₐ, R_{b}, R_{c}, and R_{d} are hydrogen. Other preferred compounds of of Formula IIb are those where C is CH, D is nitrogen, Rₐ and R_{b} are hydrogen and Rₑ is ethyl. Other preferred compounds of Formula IIb are those where both C and D are CH, Rₐ and R_{b} are hydrogen and Rₑ is ethyl.

Other specific compounds of the invention include those of Formula III: 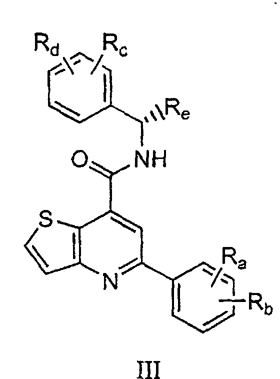 where
Rₐ and R_{b} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl;
R_{c} and R_{d} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl; and
Rₑ is C₁-C₆ alkyl, preferably C₁-C₄alkyl, more preferably methyl, ethyl or propyl.

Preferred compounds of Formula III are those where Rₐ and R_{b} are hydrogen and Rₑ is ethyl.

Other specific compounds of the invention include those of Formula IVa: 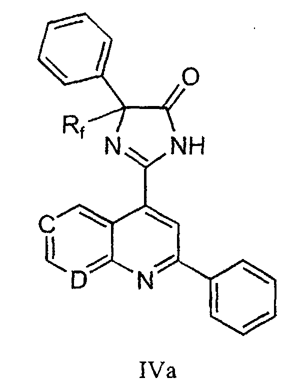 where
C and D are independently CH or N, provided that not both C and D are N simultaneously;
R_{f} is hydrogen or C₁-C₆ alkyl, preferably methyl or ethyl;
Rₐ and R_{b} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl; and
R_{c} and R_{d} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl.

Preferred compounds of Formula IVa are those where Rₐ and R_{b} are hydrogen and R_{f} is ethyl.

Still other specific compounds of the invention include those of Formula IVb: 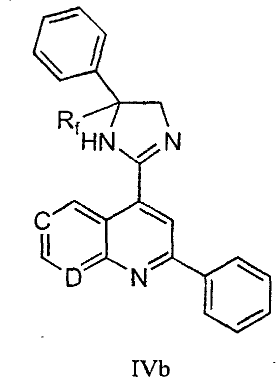 where
C and D are independently CH or N, provided that not both C and D are N simultaneously;
R_{f} is hydrogen or C₁-C₆ alkyl, preferably methyl or ethyl;
Rₐ and R_{b} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl; and
R_{c} and R_{d} are independently hydrogen, halogen, cyano, trifluoromethyl, trifluoromethoxy, hydroxy, C₁-C₆ alkyl, C₁-C₆ alkoxy, amino, or mono or dialkylamino where each alkyl independently C₁-C₆ alkyl.

Preferred compounds of Formula IVb are those where Rₐ and R_{b} are hydrogen and R_{f} is ethyl.

Compounds of formula I may have one or more asymmetric centers and may therefore exist in more than one stereoisomeric form. All stereoisomeric forms and mixtures thereof are encompassed in this application.

This invention also includes methods for using compounds of formula I to treat diseases and disorders in mammals in which activation of NK-3 receptors is of importance.

Non-toxic pharmaceutical salts include salts of acids such as hydrochloric, phosphoric, hydrobromic, sulfuric, sulfinic, formic, toluene sulfonic, hydroiodic, acetic and the like. Those skilled in the art will recognize a wide variety of non-toxic pharmaceutically acceptable addition salts.

Representative compounds of the present invention are those of Formula I and their pharmaceutically acceptable salts. The present invention also encompasses the acylated prodrugs of the compounds of Formula I. Those skilled in the art will recognize various synthetic methodologies that may be employed to prepare non-toxic pharmaceutically acceptable addition salts and acylated prodrugs of the compounds encompassed by Formula I.

By "lower alkyl" and "alkyl" in the present invention is meant straight or branched chain alkyl groups having 1-6 carbon atoms, such as, for example, methyl, ethyl, propyl, isopropyl, n-butyl, sec-butyl, tert-butyl, pentyl, 2-pentyl, isopentyl, neopentyl, hexyl, 2-hexyl, 3-hexyl, and 3-methylpentyl.

By "lower alkoxy" and "alkoxy" in the present invention is meant straight or branched chain alkoxy groups having 1-6 carbon atoms, such as, for example, methoxy, ethoxy, propoxy, isopropoxy, n-butoxy, sec-butoxy, tert-butoxy, pentoxy, 2-pentyl, isopentoxy, neopentoxy, hexoxy, 2-hexoxy, 3-hexoxy, and 3-methylpentoxy.

By halogen in the present invention is meant fluorine, bromine, chlorine, and iodine.

Where a substituent, such as, for example, W, represents groups such as piperidinyl, morpholinyl, thiomorpholinyl, pyrrolindinyl, piperazinyl, homopiperazinyl, azabicyclo[3.2.2]nonanyl, and isoindolinyl, those groups are preferably covalently coupled to the parent structure by a nitrogen atom in the group. Further, the specific groups recited in this paragraph may unsubstituted, or mono or disubstituted with alkyl or aryl groups, or fused to an aromatic ring. Where they are fused to an aromatic ring, the aromatic ring is preferably a benzo, pyridino, pyrimidino, pyridazino, or pyrazino group.

By "aromatic" as used herein is meant both aryl and heteroaryl groups.

By "hetroaryl" (aromatic heterocycle) as used herein is meant one or more aromatic ring systems of 5-, 6-, or 7-membered rings containing at least one and up to four hetero atoms selected from nitrogen, oxygen, or sulfur. Such heteroaryl groups include, for example, thienyl, furanyl, thiazolyl, imidazolyl, (is)oxazolyl, pyridyl, pyrimidinyl, (iso)quinolinyl, naphthyridinyl, benzimidazolyl, and benzoxazolyl.

By "heterocycle having aromatic character" as used herein is meant the heteroaryl groups described above and also bicyclic ring systems where at least one of the rings is aromatic. Examples of such bicyclic ring systems include pyridazinomorpholinyl, pyridinomorpholinyl, 2-2,3,4-trihydropyranopyridinyl, 5,6,7,8-tetrahydropyridinopyrimidinyl, 5,6,7,8-tetrahydroquinazolinyl, 5,6,7,8-tetrahydroisoquinolyl, 5,6,7,8,9-pentahydrocycloheptapyridinyl, and 1H,2H,3H,4H,5H-pyridinoazepinyl.

By aryl is meant an aromatic carbocyclic group having a single ring (e. g., phenyl), multiple rings (e. g., biphenyl), or multiple condensed rings in which at least one is aromatic, (e. g., 1,2,3,4-tetrahydronaphthyl, naphthyl, anthryl, or phenanthryl), which is optionally mono-, di-, or trisubstituted with, e. g., halogen, lower alkyl, lower alkoxy, trifluoromethyl, hydroxy, mono- or di-alkylamino. Preferred aryl groups are optionally substituted phenyl groups.

Representative compounds of the invention are shown below in Table 1.

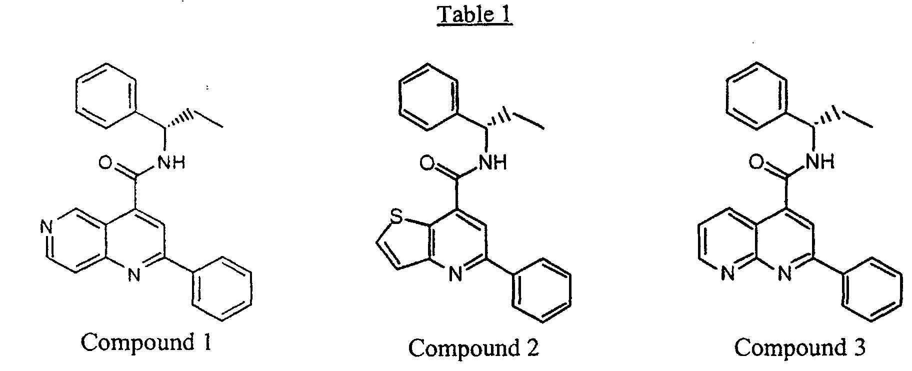

The compounds of general Formula I may be administered orally, topically, parenterally, by inhalation or spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition, there is provided a pharmaceutical formulation comprising a compound of general Formula I and a pharmaceutically acceptable carrier. One or more compounds of general Formula I may be present in association with one or more non-toxic pharmaceutically acceptable carriers and/or diluents and/or adjuvants and if desired other active ingredients. The pharmaceutical compositions containing compounds of general Formula I may be in a form suitable for oral use, for example, as tablets, troches. lozenges, aqueous or oily suspensions, dispersible powders or granules, emulsion, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydropropylmethylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents arc exemplified by those already mentioned above. Additional excipients, for example sweetening, flavoring and coloring agents, may also be present.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monoleate, and condensation products of the said partial esters with ethylene oxide, for example polyoxyethylene sorbitan monoleate. The emulsions may also contain sweetening and flavoring agents.

Syrups and elixirs may be formulated with sweetening agents, for example glycerol, propylene glycol. sorbitol or sucrose. Such formulations may also contain a demulcent, a preservative and flavoring and coloring agents. The pharmaceutical compositions may be in the form of a sterile injectable aqueous or oleaginous suspension. This suspension may be formulated according to the known art using suitable dispersing or wetting agents and suspending agents, such as those mentioned above. The sterile injectable preparation may also be a solution or suspension in a non-toxic parentally acceptable diluent or solvent, for example as a solution in 1,3-butanediol. Among the acceptable vehicles and solvents that may be employed are water, isotonic aqueous sodium chloride solutions, Ringer's solution and other buffered isotonic salt solutions. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compounds of general Formula I may also be administered in the form of suppositories for rectal administration of the drug. These compositions can be prepared by mixing the drug with a suitable non-irritating excipient, which is solid at ordinary temperatures but liquid at the body temperature and will therefore melt internally to release the drug. Such materials include cocoa butter and polyethylene glycols.

Compounds of general Formula I may be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of the order of from about 0.1 mg to about 140 mg, preferably between about 1 mg to about 70 mg, per kilogram of body weight per day are typically useful in the treatment of the above-indicated conditions (preferably between about 5 mg to about 5 g per patient per day). The amount of active ingredient that may be combined with the carrier materials to produce a single dosage form will vary depending upon the host treated and the particular mode of administration. Dosage unit forms will generally contain between from about 1 mg to about 1 g of an active ingredient.

Frequency of dosage may also vary depending on the compound used and the particular disease treated. However, for treatment of most CNS disorders, a dosage regimen of 4 times daily or less is preferred. For the treatment of schizoprenia and depression a dosage regimen of 1 or 2 times daily is particularly preferred.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

Preferred compounds of the invention will have certain pharmacological properties. Such properties include, but are not limited to oral bioavailability, low toxicity, low serum protein binding and desirable *in vitro* and *in vivo* half-lifes. Penetration of the blood brain barrier for compounds used to treat CNS disorders is necessary, while low brain levels of compounds used to treat periphereal disorders are often preferred.

Assays may be used to predict these desirable pharmacological properties. Assays used to predict bioavailability include transport across human intestinal cell monolayers, including Caco-2 cell monolayers. Toxicity to cultured hepatocyctes may be used to predict compound toxicity. Penetration of the blood brain barrier of a compound in humans may be predicted from the brain levels of the compound in laboratory animals given the compound intravenously.

Serum protein binding may be predicting from albumin binding assays. Such assays are described in a review by Oravcová, et al. (Journal of Chromatography B (1996) volume 677, pages 1-27).

Compound half-life is inversely proportional to the frequency of dosage of a compound. *In vitro* half-lifes of compounds may be predicted from assays of microsomal half-life as described by Kuhnz and Gieschen (Drug Metabolism and Disposition, (1998) volume 26, pages 1120-1127).

The present invention also pertains to packaged pharmaceutical compositions for treating disorders responsive to NK-3 receptor modulation, e.g., schizoprenia or depression. The packaged pharmaceutical compositions include a container holding a therapeutically effective amount of at least one NK-3 receptor ligand as described supra and instructions (e.g., labeling) indicating that the contained NK-3 receptor ligand is to be used for treating a disorder responsive to NK-3 receptor modulation in a patient.

The present invention also pertains to methods of inhibiting the binding of a neurokinin to the NK-3 receptor which methods involve contacting a compound of the invention with cells expressing NK-3 receptors, wherein the compound is present at a concentration sufficient to inhibit neurokinin binding to cells expressing a cloned human NK-3 receptor *in vitro* and to methods for altering the signal-transducing activity of NK-3 receptors, said method comprising exposing cells expressing such receptors to an effective amount of a compound of the invention.

An illustration of the preparation of compounds of the present invention is given in Schemes 1, 2, and 3. The various substituents in Schemes 1, 2, and 3 are defined as above for Formula I.

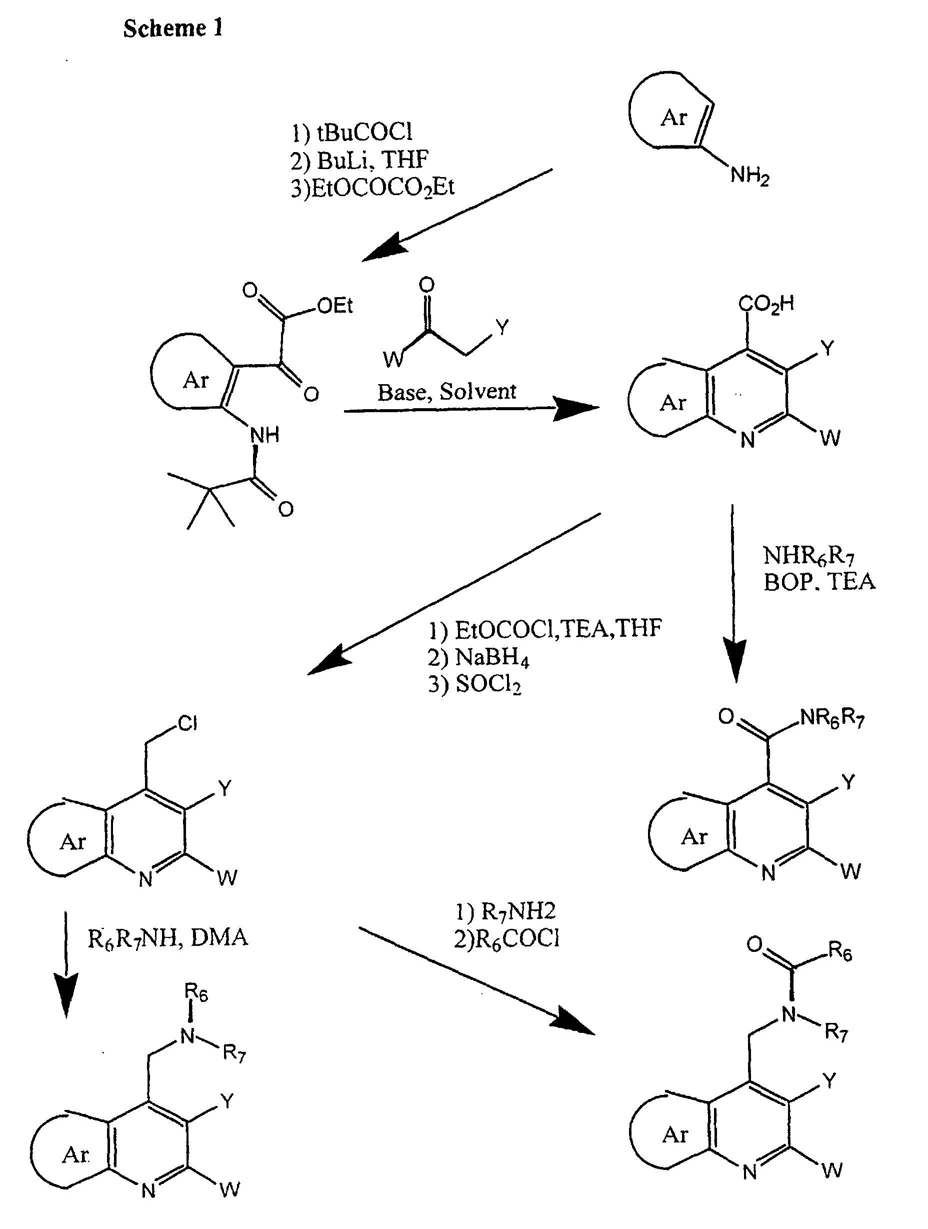

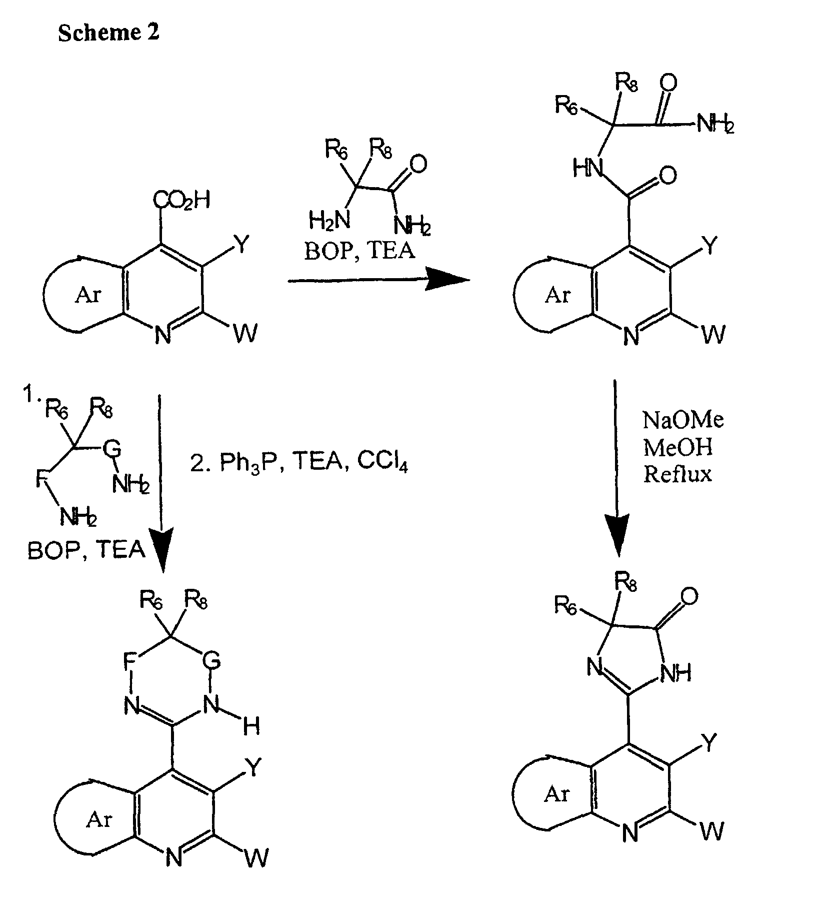

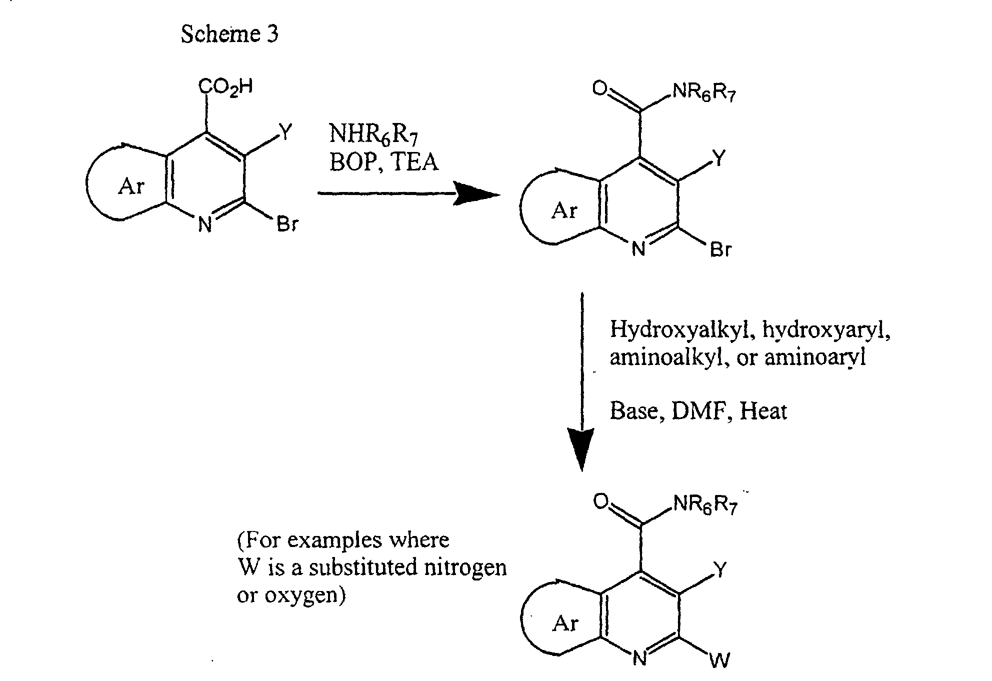

Those having skill in the art will recognize that the starting materials may be varied and additional steps employed to produce compounds encompassed by the present invention, as demonstrated by the following examples. In some cases protection of certain reactive functionalities may be necessary to achieve some of the above transformations. In general the need for such protecting groups will be obvious to those skilled in the art of organic synthesis as well as the conditions necessary to attach and remove such groups.

The invention is illustrated further by the following examples, which are not to be construed as limiting the invention in scope or spirit to the specific procedures described in them.

### Example I

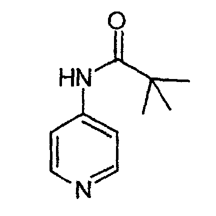

To a mixture of 20g of 4-Aminopyridine and 37 mL of triethylamine in 380 mL of methylene chloride is added 28.8 mL of pivaloyl chloride in a dropwise fashion. After 1 hour at room temperature the reaction mixture is washed with water and sodium bicarbonate solution, dried over magnesium sulfate and the solvent is removed in vacuo. The residue is triturated with hexane to afford 4-trimethylacetamidopyridine as a white solid.

### Example II

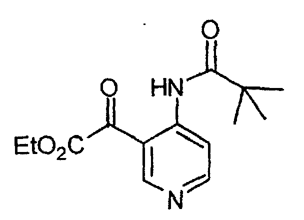

A solution of 10g of 4-trimethylacetamido pyridine in 150mL of dry tetrahydrofuran is cooled to -78°C and 88 mL of 1.6M n-butyllithium in hexane is added in a dropwise fashion. The mixture is then stirred at 0°C for 3 hours, cooled again to -78°C and 21.3g of diethyl oxalate dissolved in 190mL of tetrahydrofuran is added in a dropwise fashion, the reaction mixture was then gradually allowed to come to room temperature. The reaction mixture is diluted with water and the product is extracted with ether. After drying over magnesium sulfate the solvent is removed in vacuo. The residue is chromatagraphed on silica gel with hexane/ether 2:1 as the eluent to afford ethyl 4-trimethylacetamido-3-pyridylpyruvate as an orange oil.

### Example III

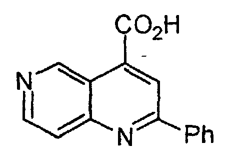

A mixture of 3.59g of ethyl 4-trimethylacetamido-3-pyridylpyruvate, 2.89g of potassium hydroxide in 10mL of ethanol and 40 mL of water is heated at reflux for 2 hours. At this time 3.1g of acetophenone is added and refluxing is continued for 6 hours. The ethanol is removed in vacuo, the resulting aqueous solution is washed with ether and the aqueous layer is acidified to pH5. The product is collected washed with water and dried to afford (2-phenylpyridino[4,3-b]pyridin-4-yl) carboxylic acid as a white solid.

### Example IV

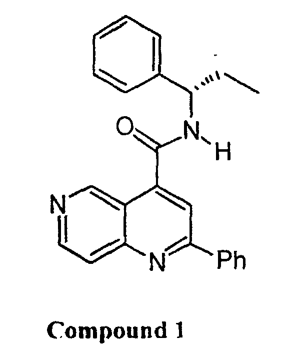

To a solution of 250mg of 2-phenylpyridino[4,3-b]pyridin-4-yl carboxylic acid dissolved in 5mL of dimethylacetamide and 200uL of triethylamine is added 500mg of BOP in 5ml. of dichloroethane followed by 150mg of(S)-1-phenylpropylamine. The reaction mixture is stirred for 16 hours at room temperature. The reaction mixture is poured onto 50mL of 1N NaOH and the product is extracted with 25mL of ethyl acetate. After drying over magnesium sulfate the solvent is removed in vacuo to afford N-[(S)-1-phenyl-1-propyl)]-(2-phenylpyridino[4.3-b]pyridin-4-yl)carboxamide as a solid.

### Example V

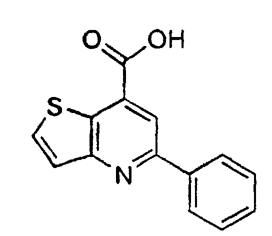

Prepare 2-phenylthieno[3,2-b]pyridin-4-yl chloride by the method described in PCT application WO 9943682. A mixture containing 400 mg of 2-phenylthieno[3,2-b]pyridin-4-yl chloride, 400 mg of *p*-toluenesulfonic acid and 200 mg of potassium cyanide in anhydrous DMF is heated at 160 °C in an oil bath for 16 h under nitrogen. The mixture is cooled to ambient temperature, poured into a mixture of ice and water. The resulting mixture is extracted with ethyl acetate and the ethyl acetate layer is washed with water followed by brine. The ethyl acetate solution is dried over sodium sulfate, filtered and evaporated at reduced pressure to obtain a tan-colored residue. This material is dissolved in 10 ml of ethanol, treated with 1 g of sodium hydroxide and heated at reflux for 2 h. The ethanol solution is evaporated and the resulting residue is dissolved in water. The pH of this solution is adjusted to slightly acidic by addition of 1 N HCl to yield 2-phenylthieno[3,2-b]pyridin-4-yl carboxylic acid as a red-orange solid. After drying *in vacu* this material is pure according to proton NMR and LC/MS.

### Example VI

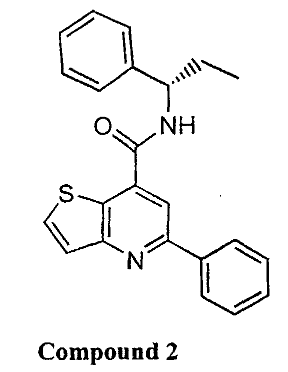

A mixture containing 100 mg of 2-phenylthieno[3,2-b]pyridin-4-yl carboxylic acid, 66 mg of (S)-1-phenylpropylamine, 43 µl of N-methylmorpholine and 79 mg of hydroxybenzotriazole (HOBT) in 8 ml of 7:3 tetrahydrofuran/acetonitrile is cooled to -5 °C. Add 580 µl of 1 mM solution of dicyclohexylcarbodiimide (DCC) in tetrahydrofuan. Stir at room temperature for 17 h. Filter to remove solids and evaporate the filtrate at reduced pressure to obtain an orange solid. Purify by chromatography on silica gel eluting with 20% ethyl acetate in hexane to obtain a cream-colored solid. TLC (20% ethyl acetate/hexane) Rf = 0.29. LC/MS m/z M+1 = 373.

### Example VII

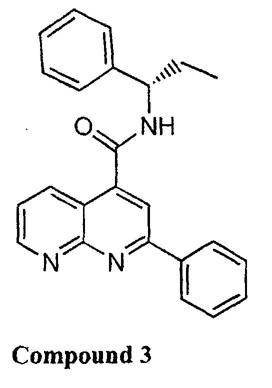

The required 2-phenylpyridino[2,3-b]pyridin-4-yl carboxylic acid is prepared from 3-aminopyridine according to the procedures outlined in Examples I-III. A mixture containing 100 mg of 2-phenylpyridino[2,3-b]pyridin-4-yl carboxylic acid, 68 mg of (S)-1-phenylpropylamine, 44 µl of N-methylmorpholine and 81 mg of hydroxybenzotriazole (HOBT) in 8 ml of 7:3 tetrahydrofuran/acetonitrile is cooled to -5 °C. Add 600 µl of 1 mM solution of dicyclohexylcarbodiimide (DCC) in tetrahydrofuan. Stir at room temperature for 17 h. Filter to remove solids and evaporate the filtrate at reduced pressure. Dilute with-10 ml of dichloromethane, let stand 1 hour and again remove solids by filtration. Evaporate the filtrate and purify by chromatography on silica gel eluting with 3% methanol in dichloromethane with 0.5% ammonium hydroxide as additive to obtain a white solid. TLC (5% methanol/dichloromethane/0.5% ammonium hydroxide) Rf = 0.45. LC/MS m/z M+1 = 368.

### Example VIII

Additional compounds of the invention, prepared as described in Schemes 1-3 are shown in Table 2a, Table 2b and Table 2c.

The compounds in TABLES 2a and 2b are prepared according to Scheme I using the following conditions: A quantity of 0.1mL of a 0.2 M acid solution in DMA:TEA is added to 0.1 mL of a 0.2M amine solution in Toluene:NMM, and 0.15 mL of a 0.2 M BOP reagent solution in dichloroethane. The mixture is heated for 3 hrs at 50° C. At which time the solution is cooled to room temperature. The mixture is partitioned between 1N NaOH and ethyl acetate. The ethyl acetate layer is chromatographed on silica gel with ethyl acetate to afford the desired compound.

Analysis is performed on a Hewlett Packard 6890 GC, equipped with a dual cooLon-column inlets and flame ionization detectors or mass spectrometer detectors. All gas flows are regulated via electronic pneumatic control. The analytical column used is a Supelco PTE-5 QTM, 15 m x 0.53 mm ID x 0.50 µm film. GC instrument control and data collection are handled using a Perkin Elmer TurboChrom Client/Server data system. GC conditions: On-column injector 163 °C for 2.5 min., ramp at 40 °C/min to 323 °C. Oven program 100 °C for 1 minute, ramp at 40 °C/min to 320 °C. Detector temperature is set at 325 °C.

In Table 2a, X₁ is a group of the Formula:

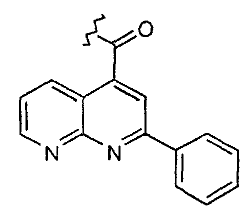

In Table 2b, X₁ represents a group of the formula:

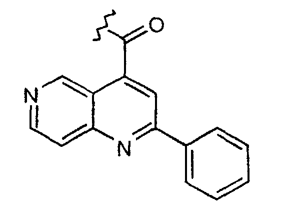

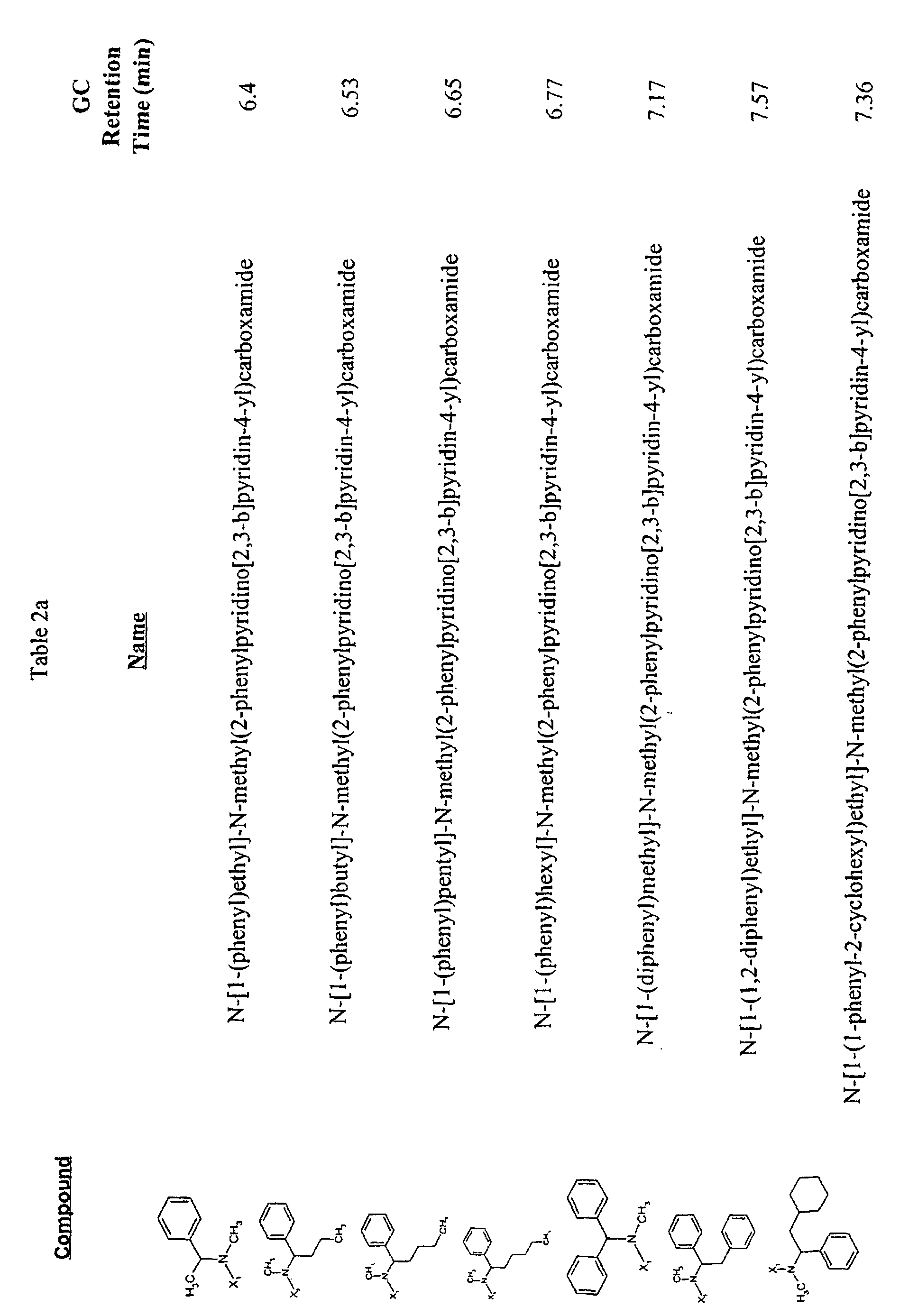

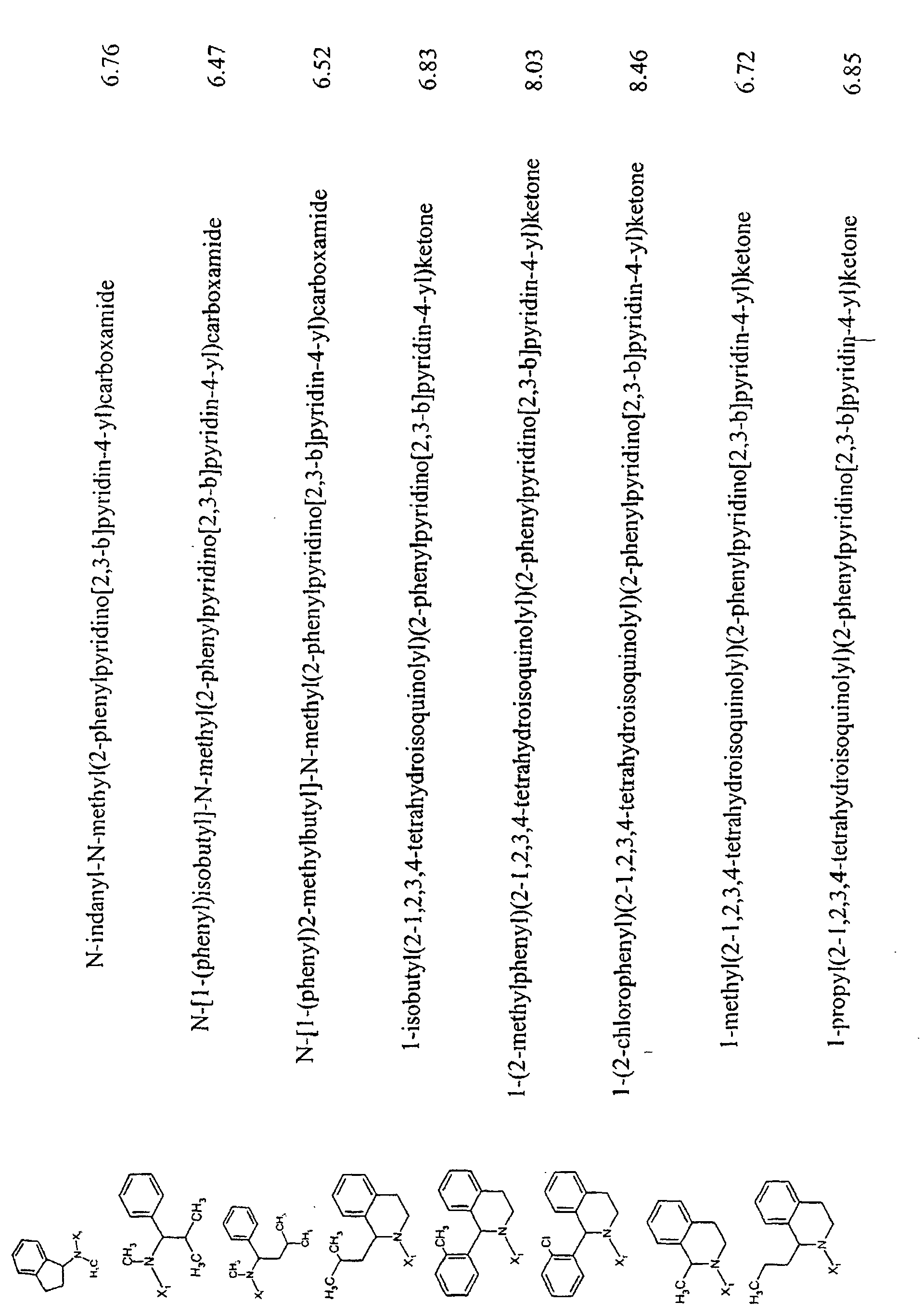

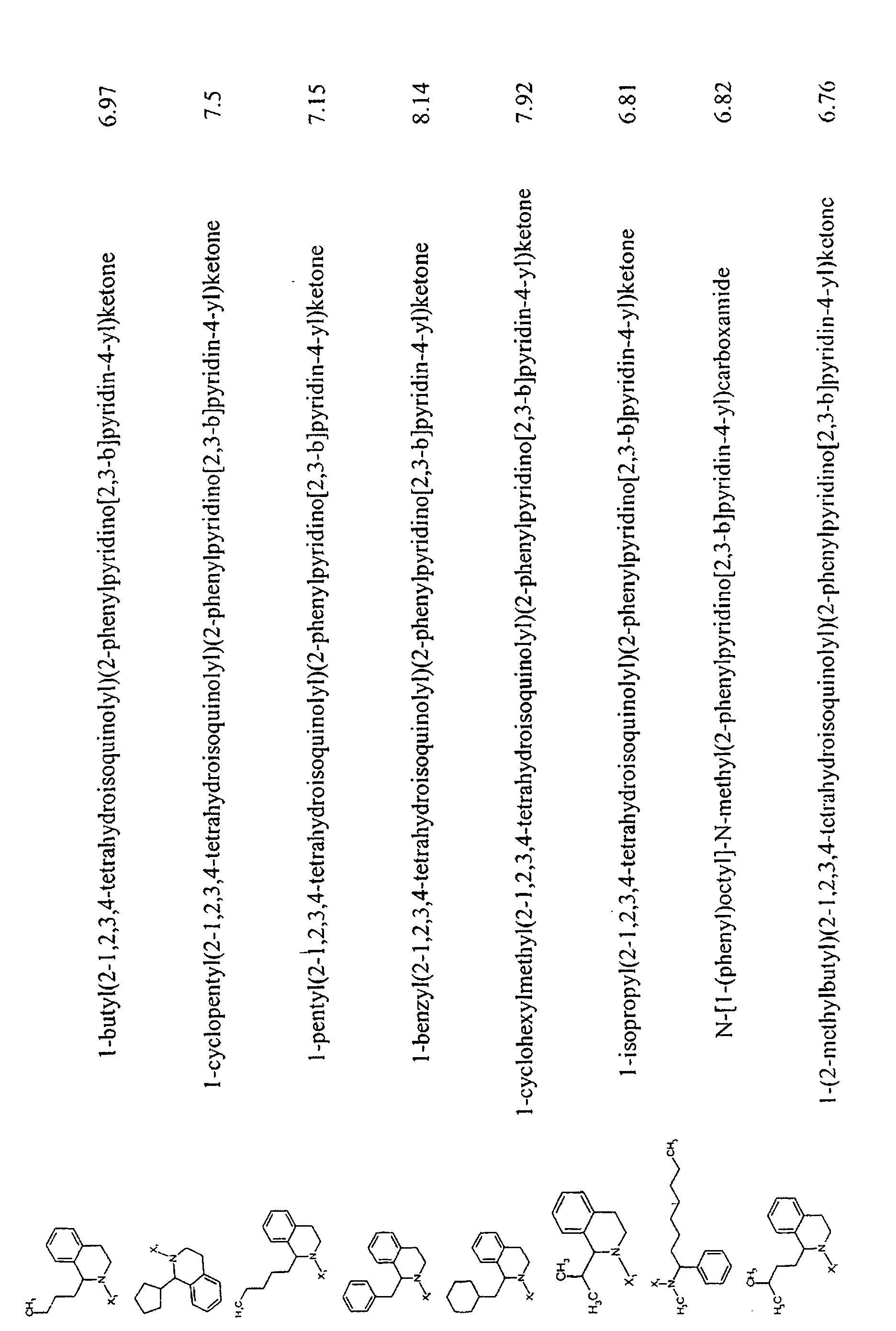

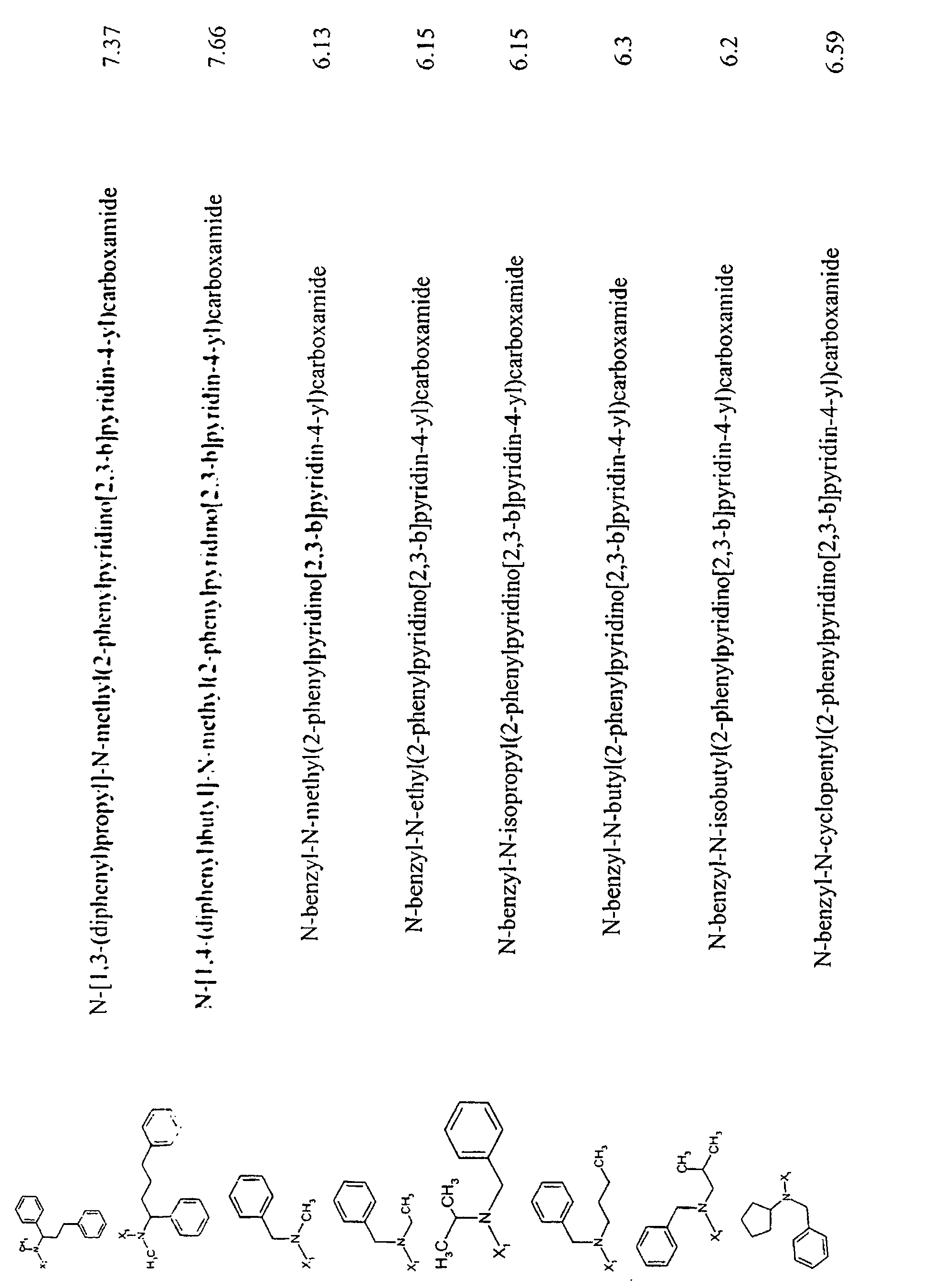

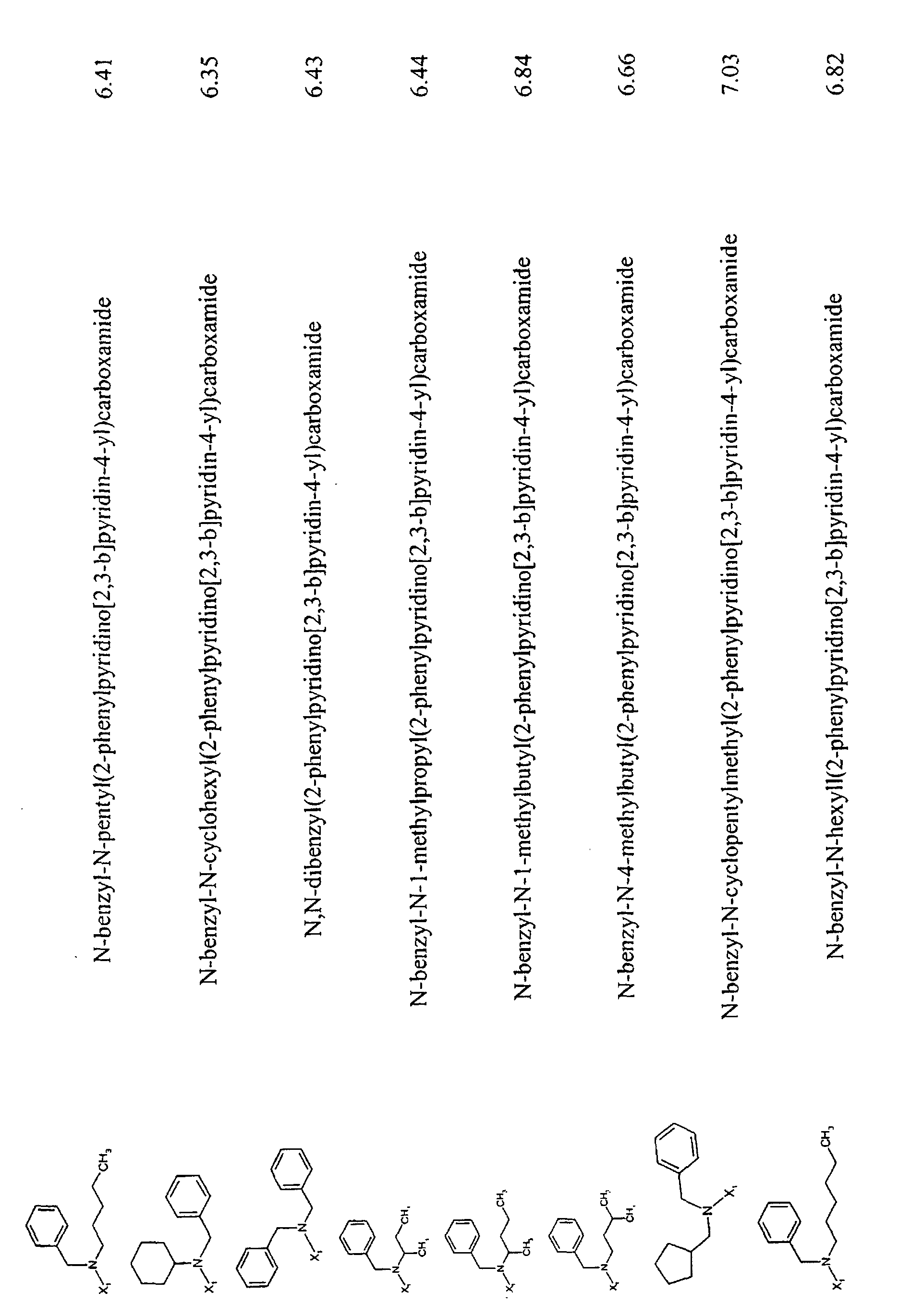

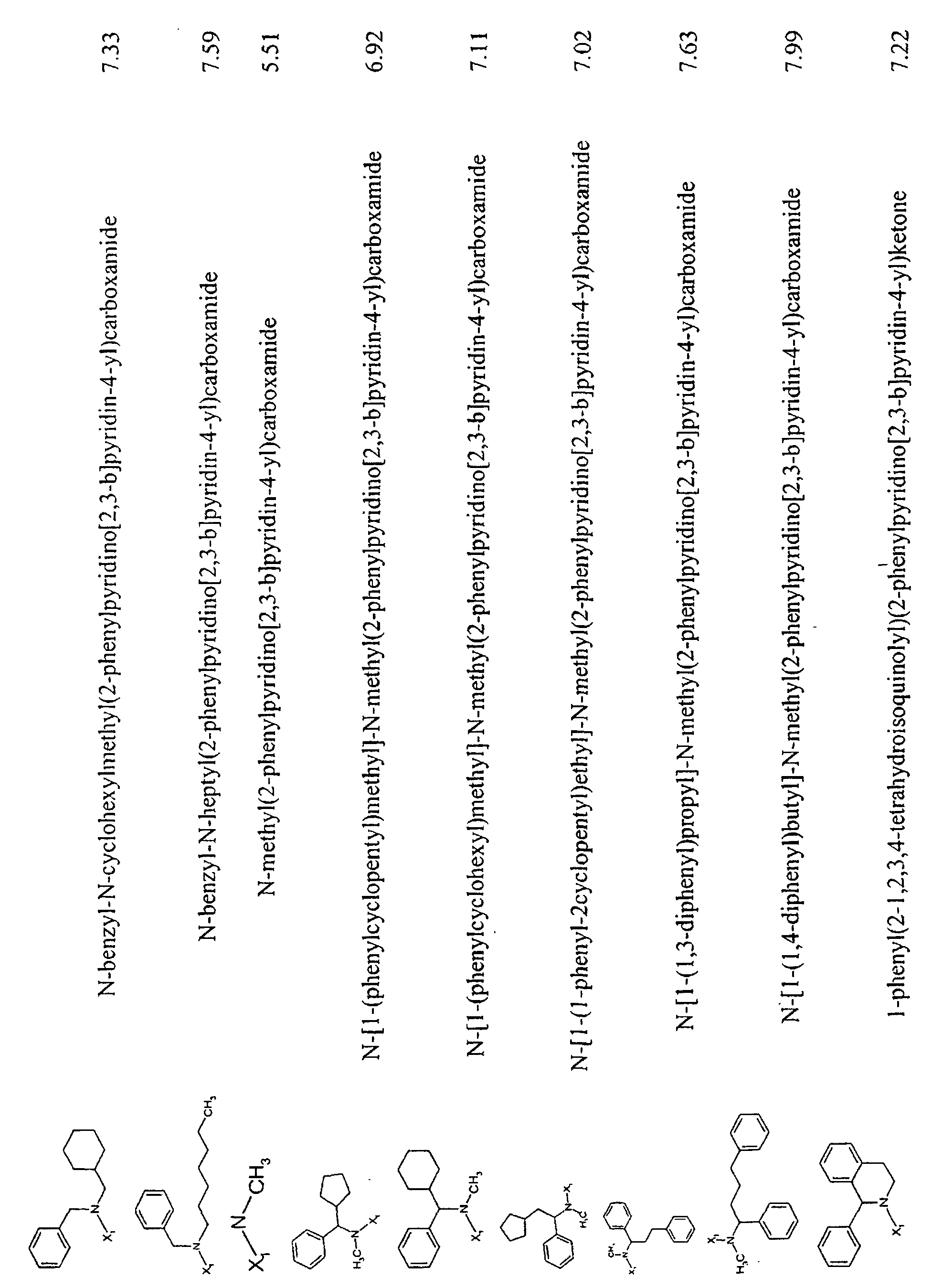

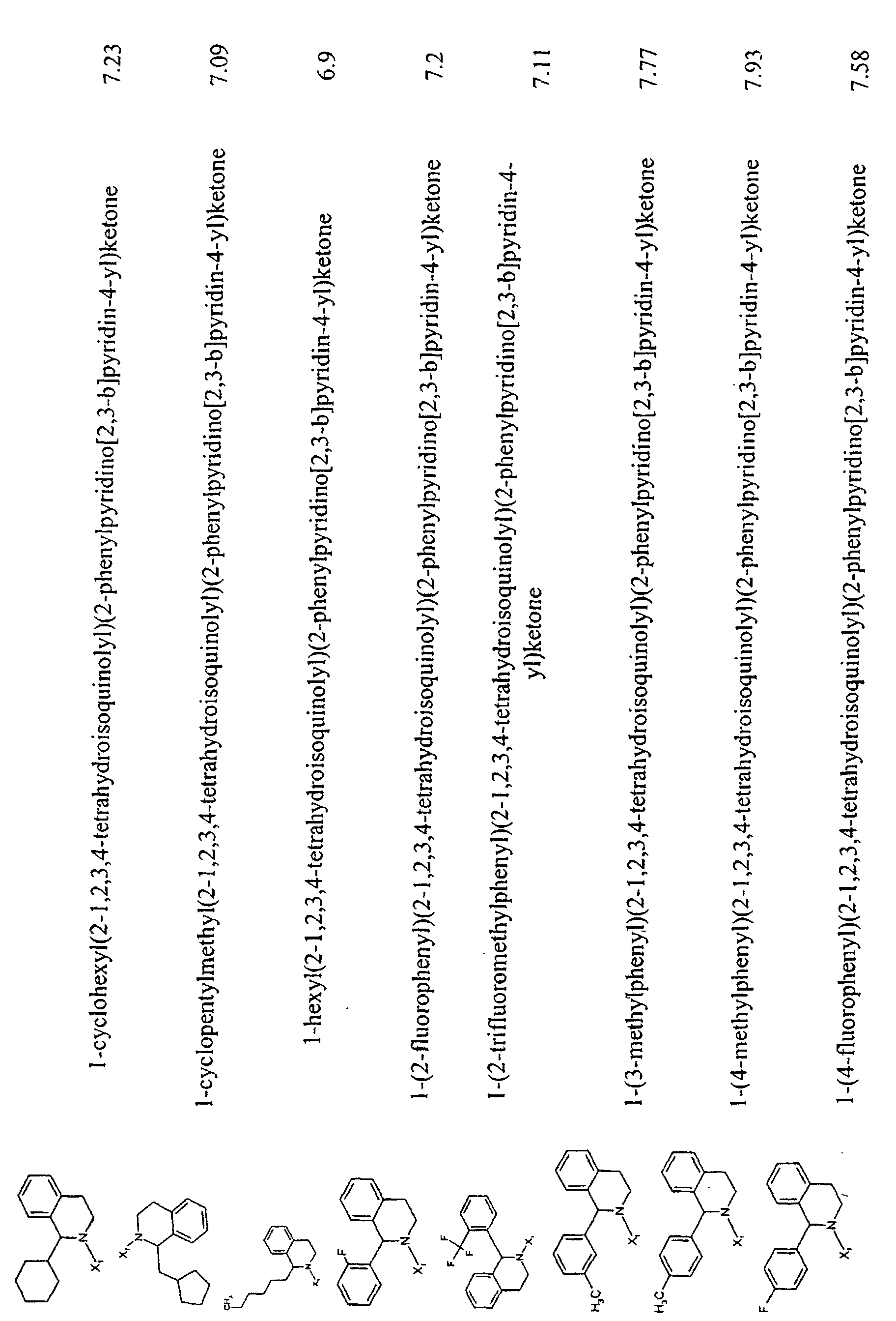

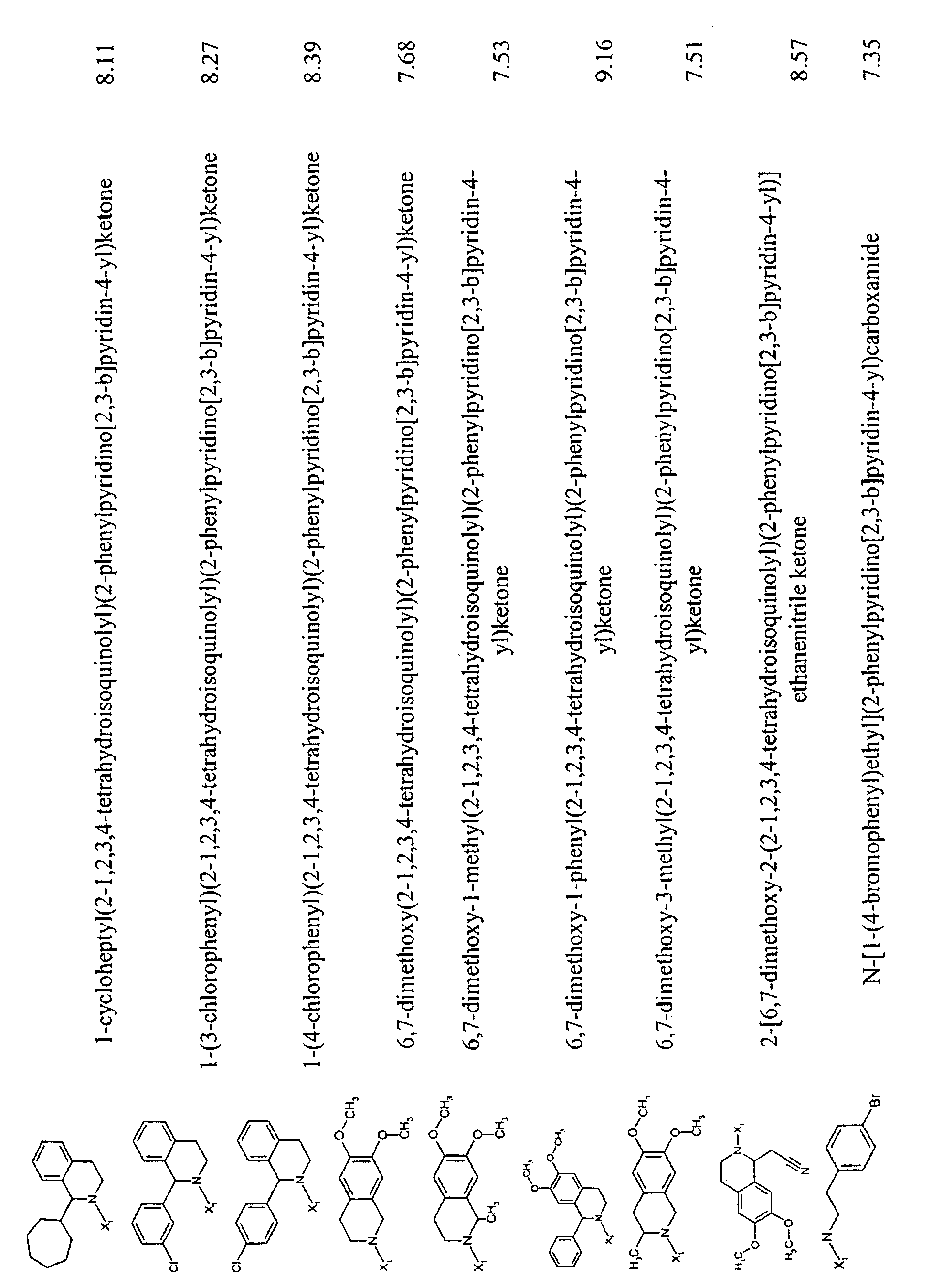

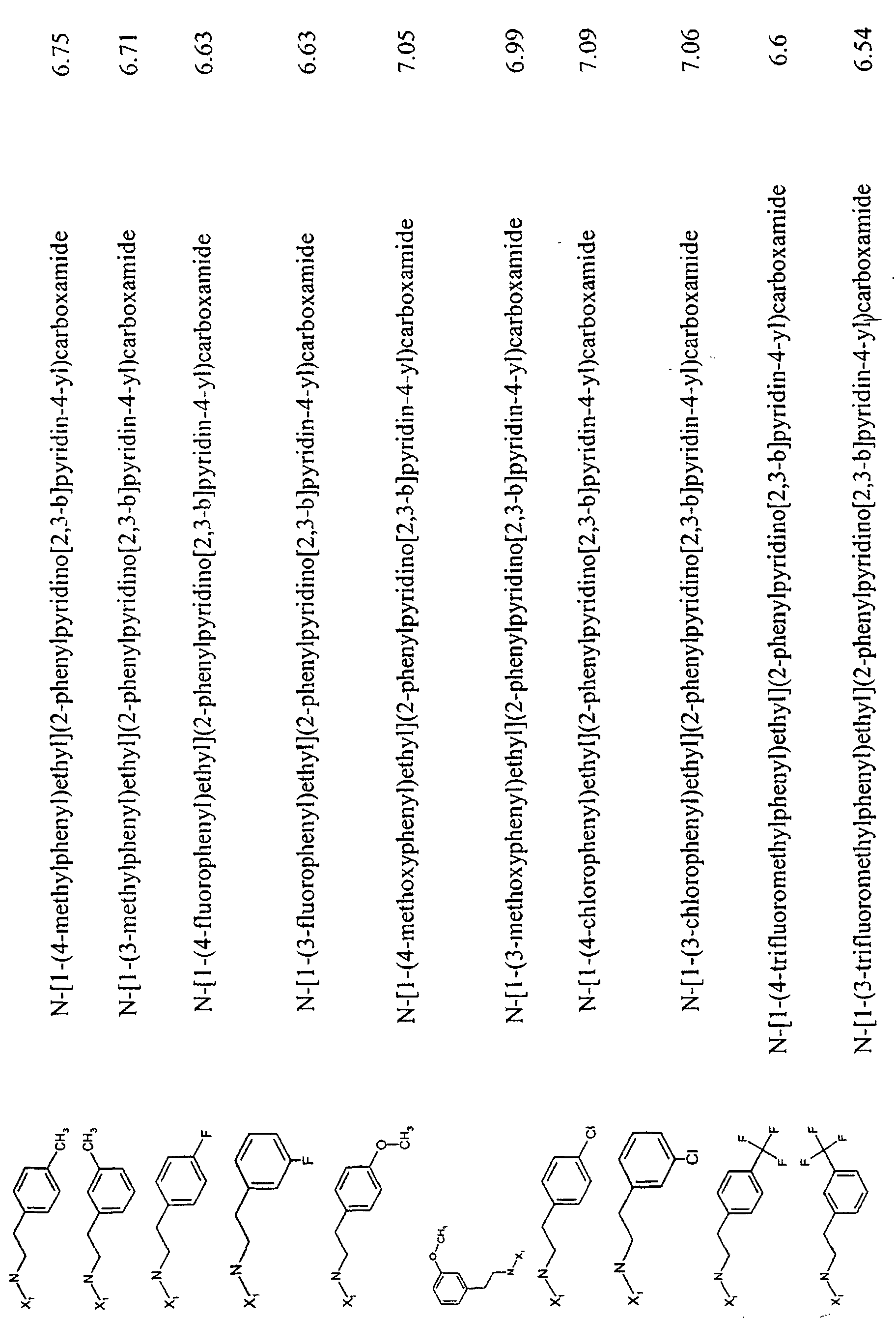

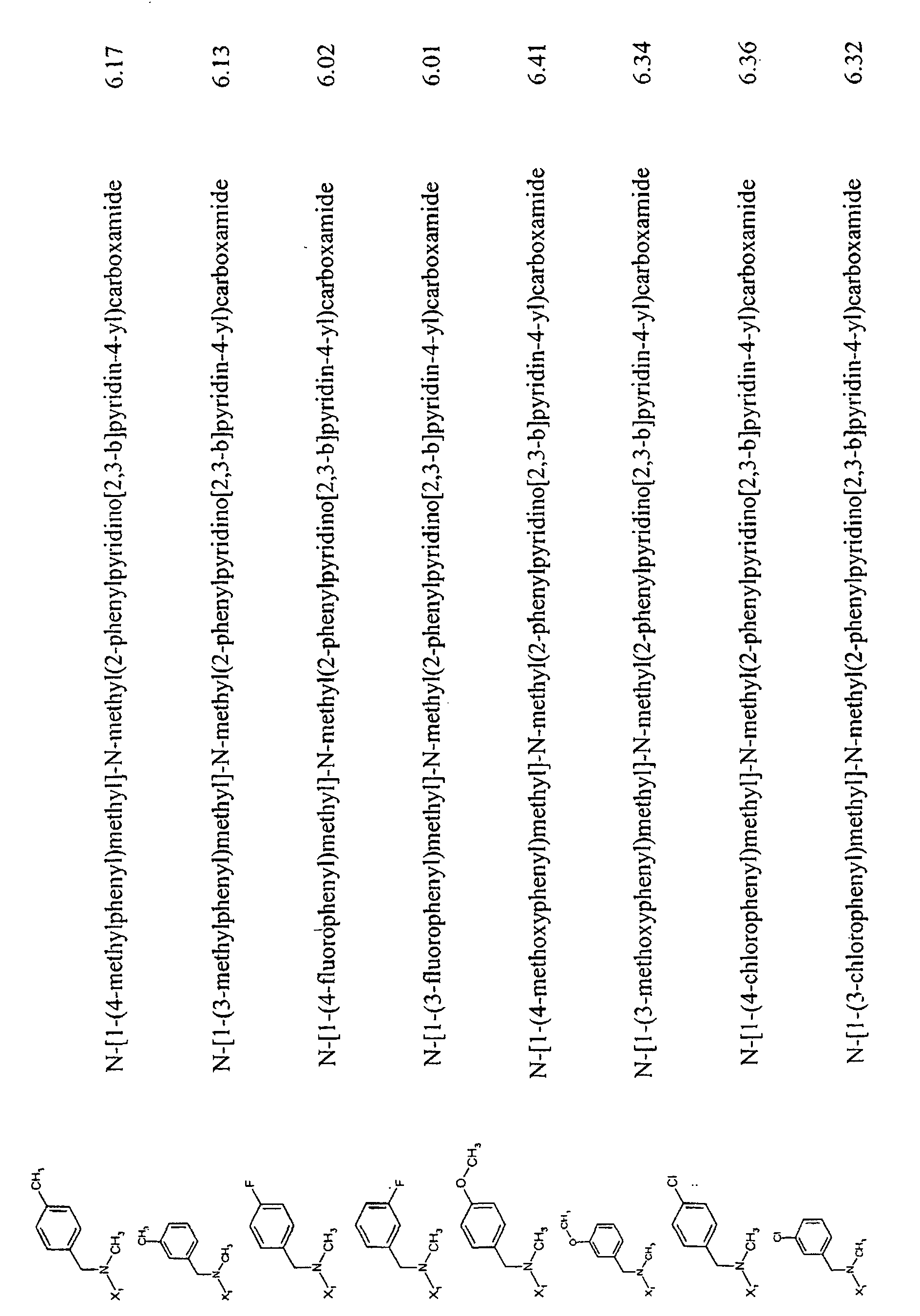

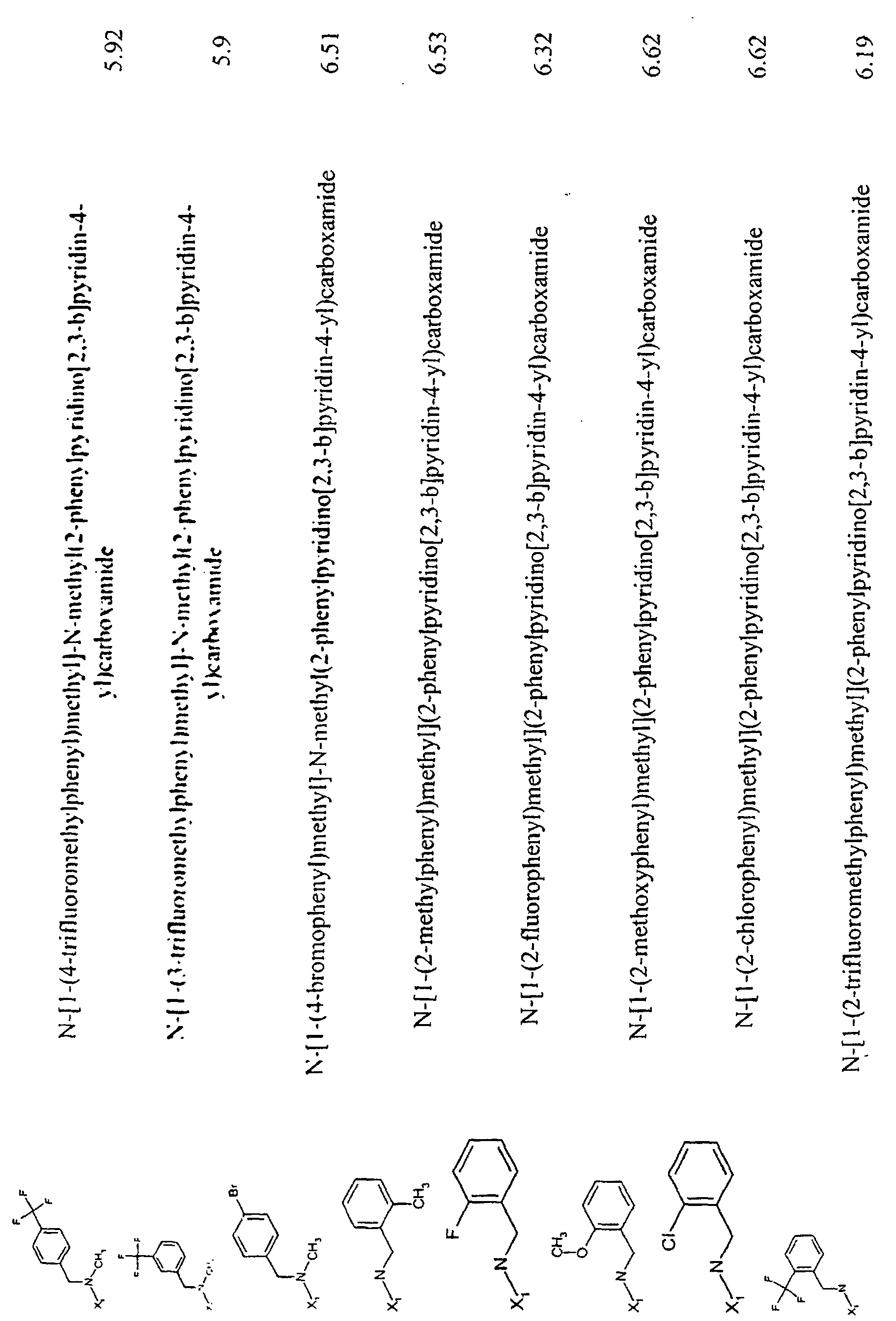

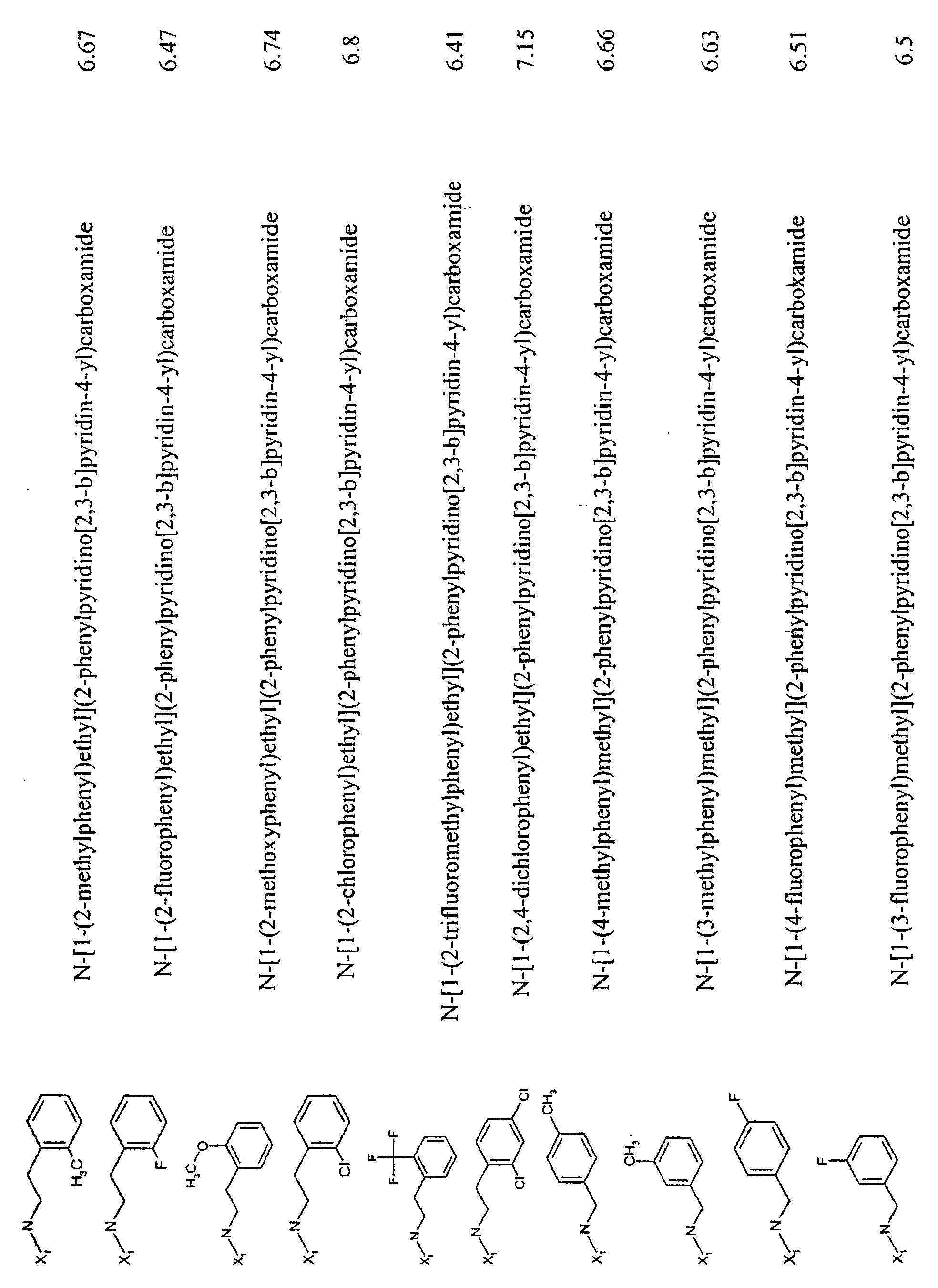

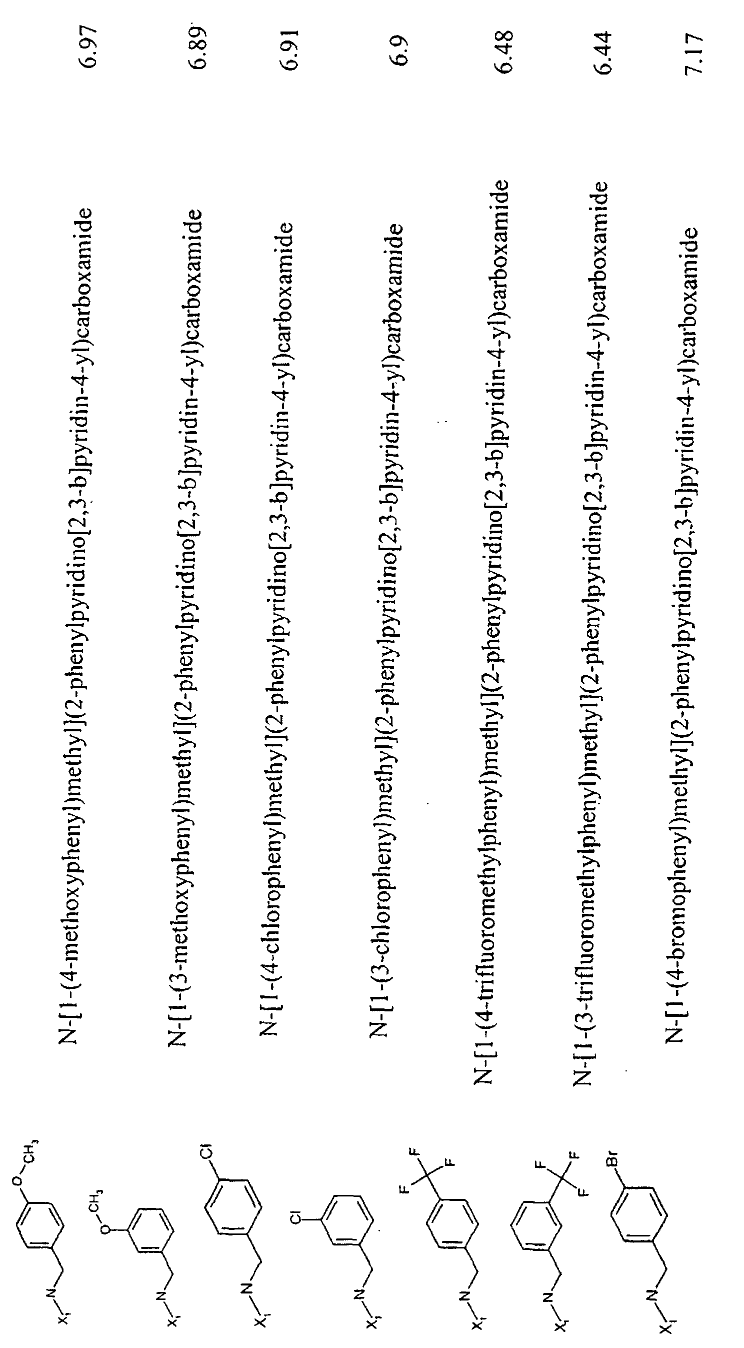

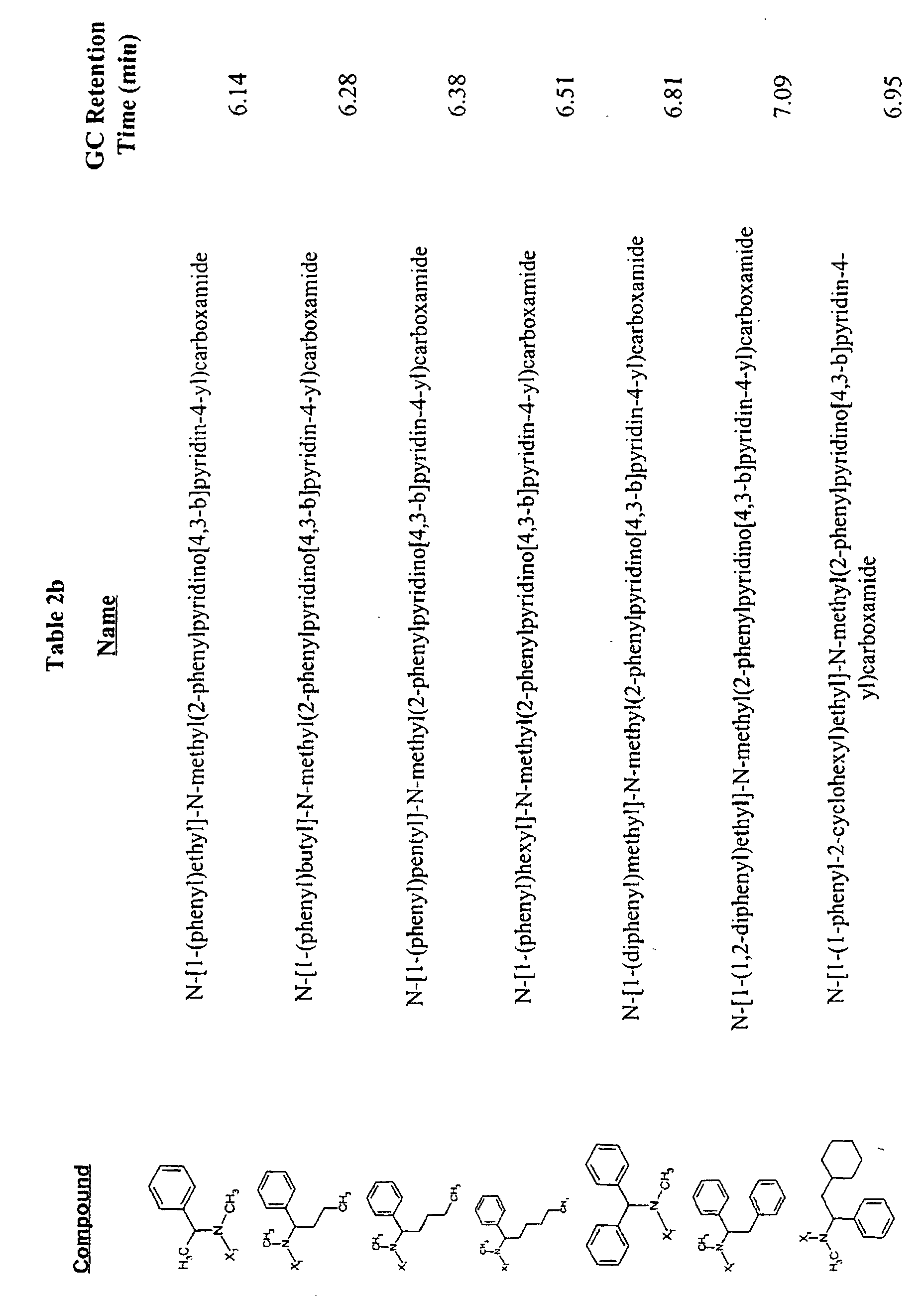

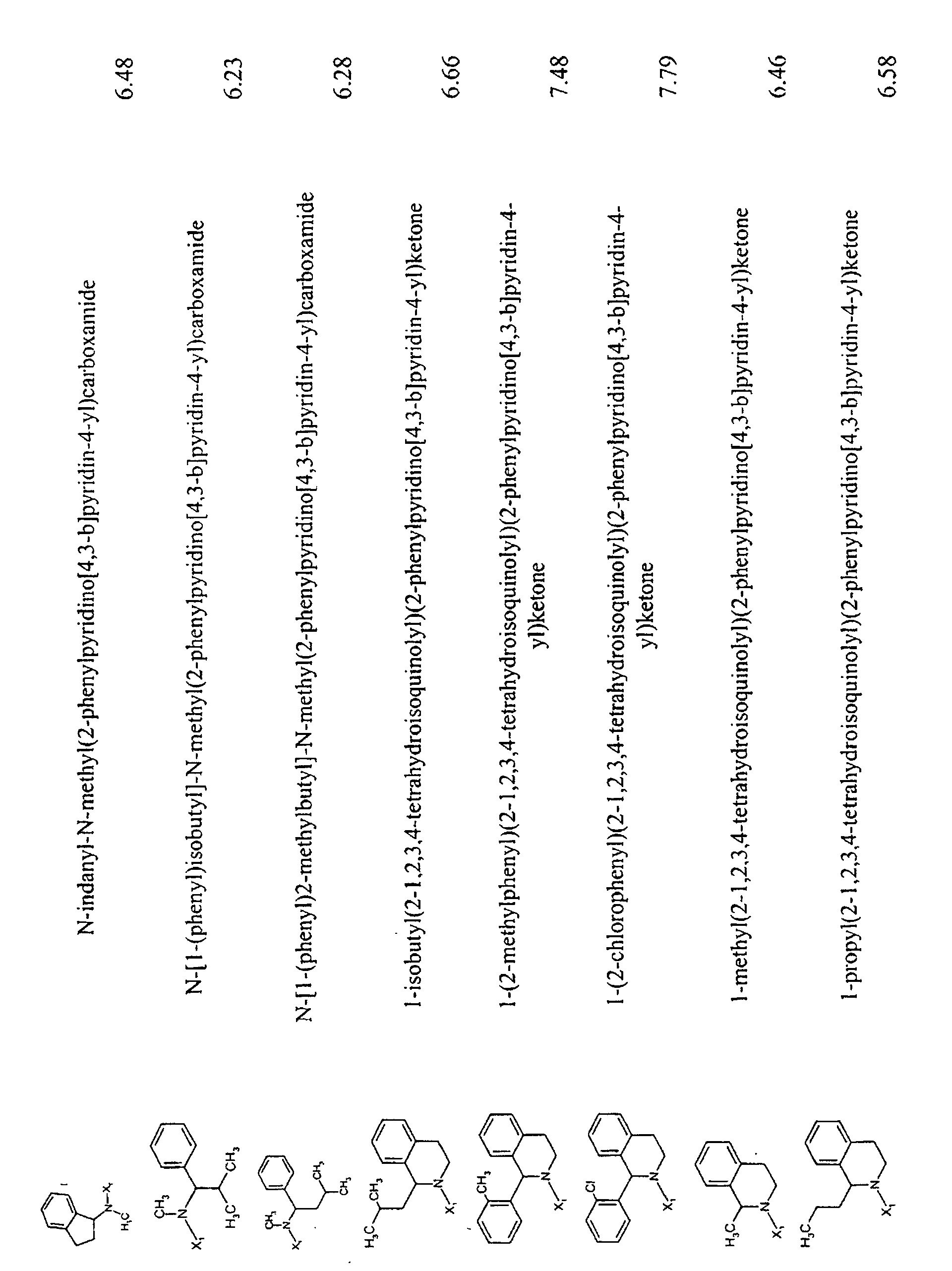

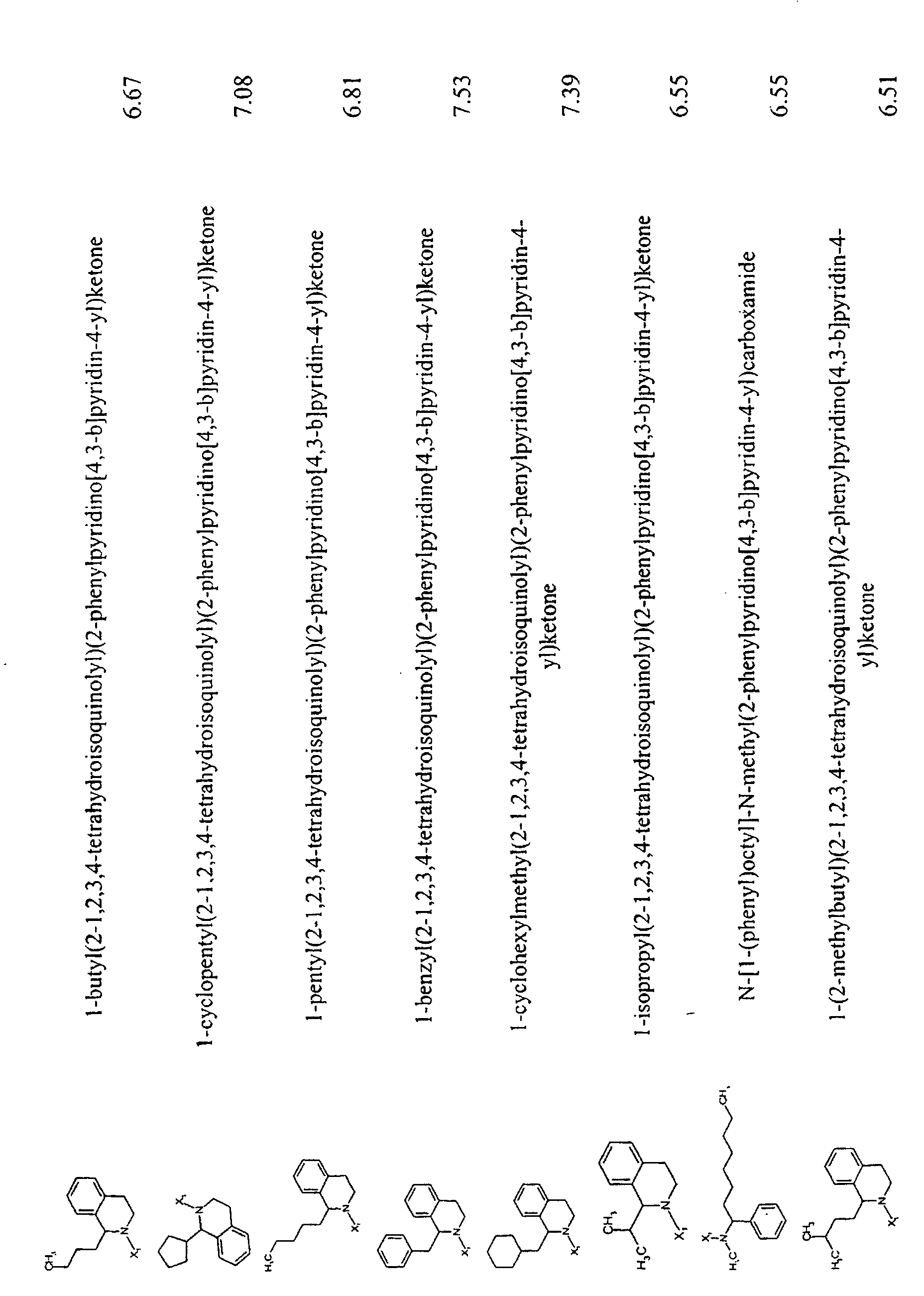

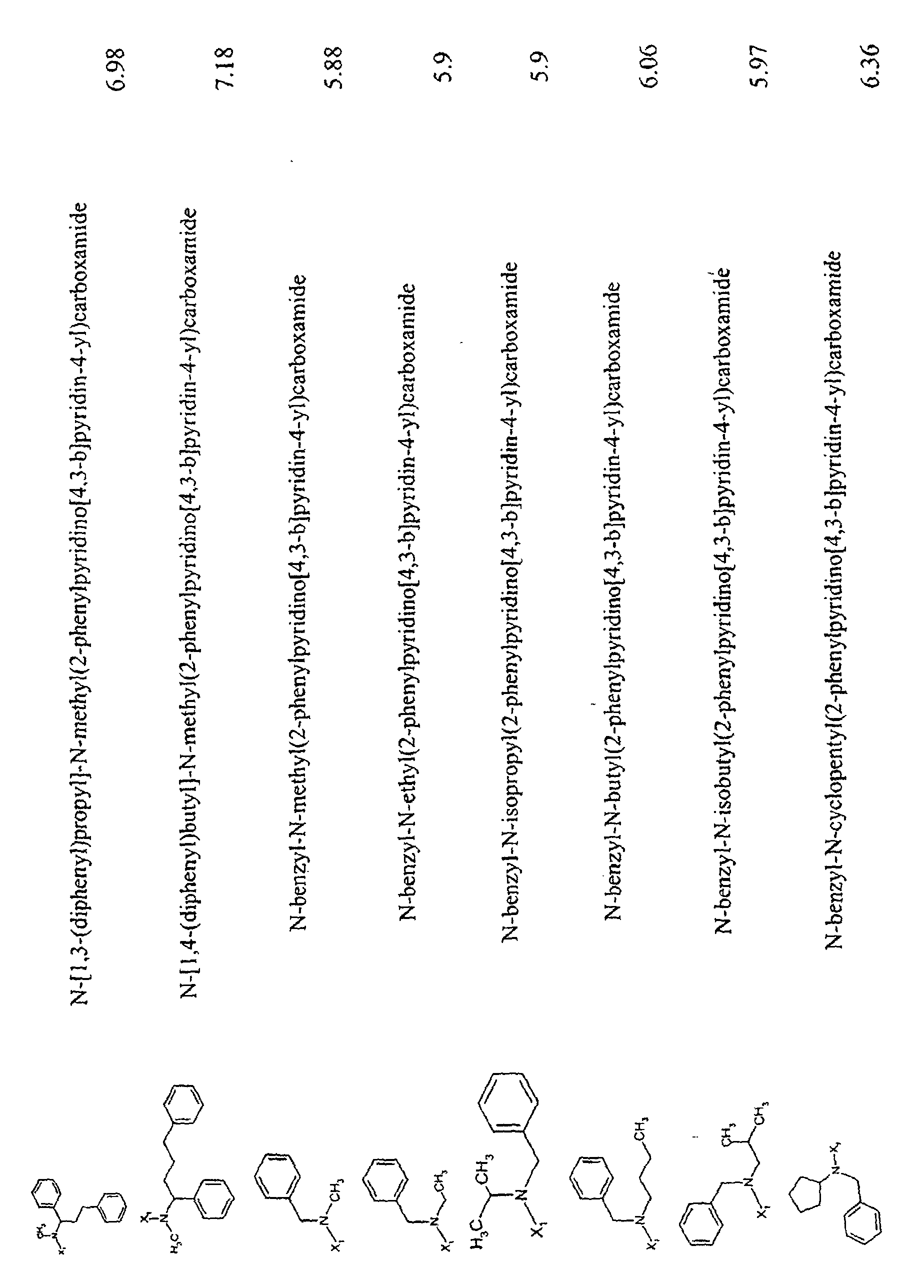

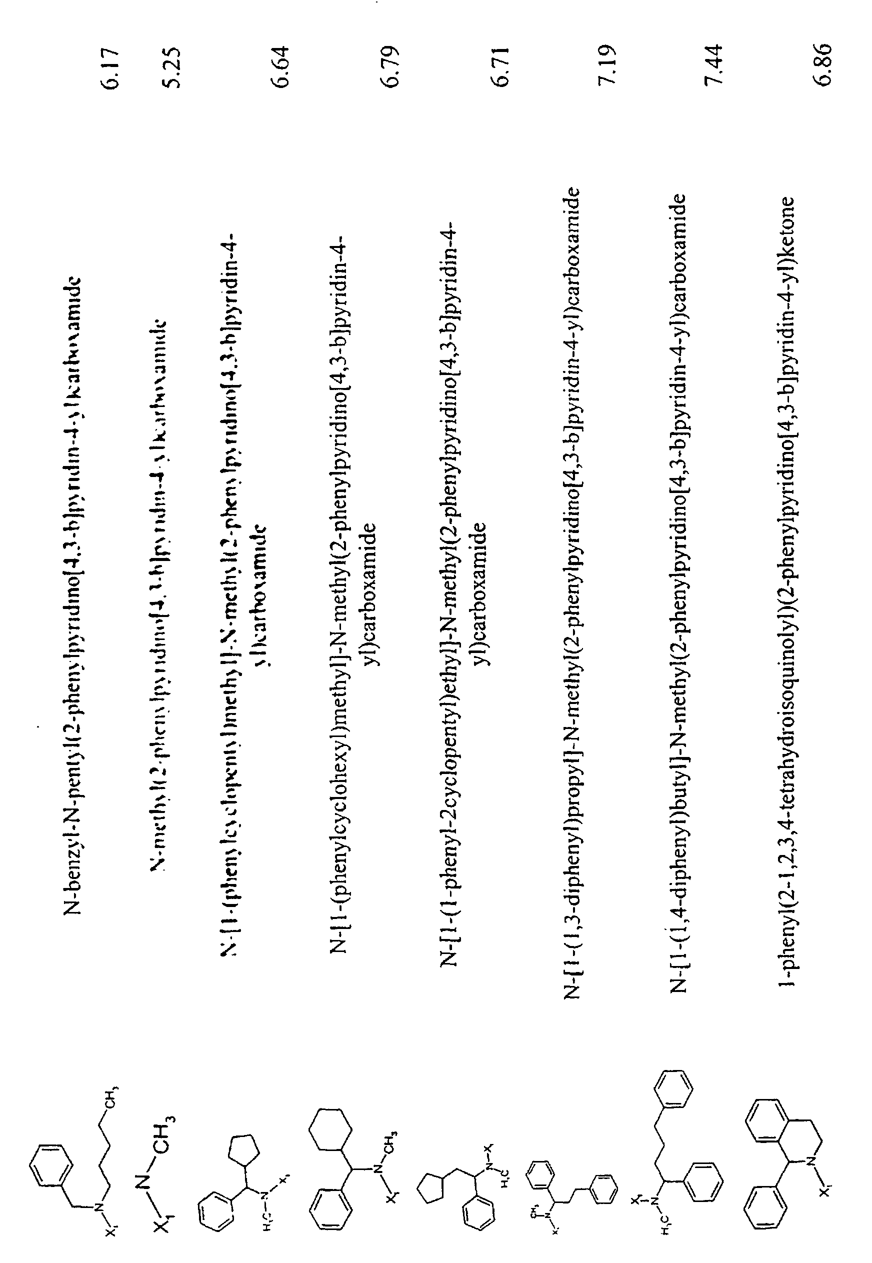

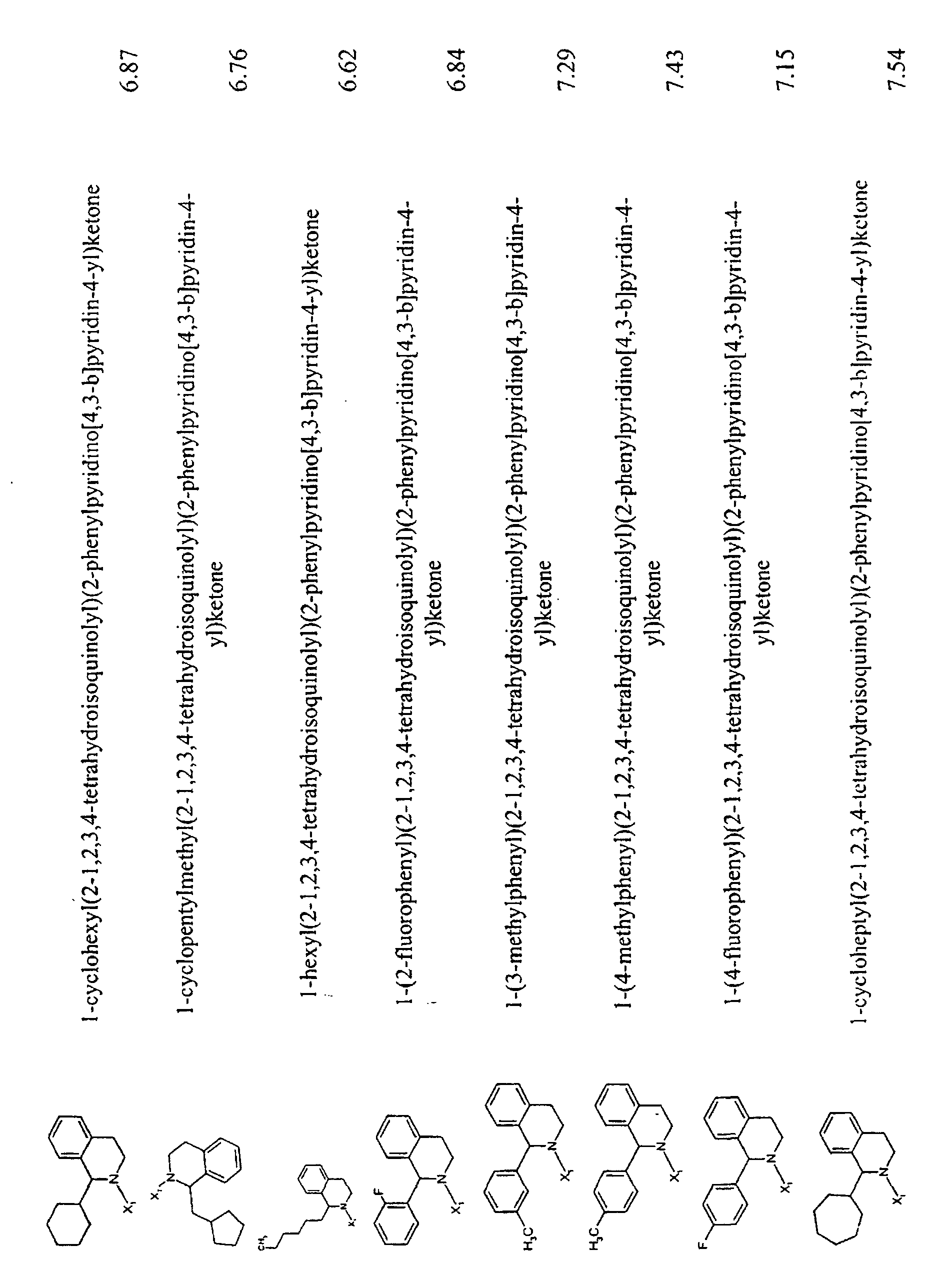

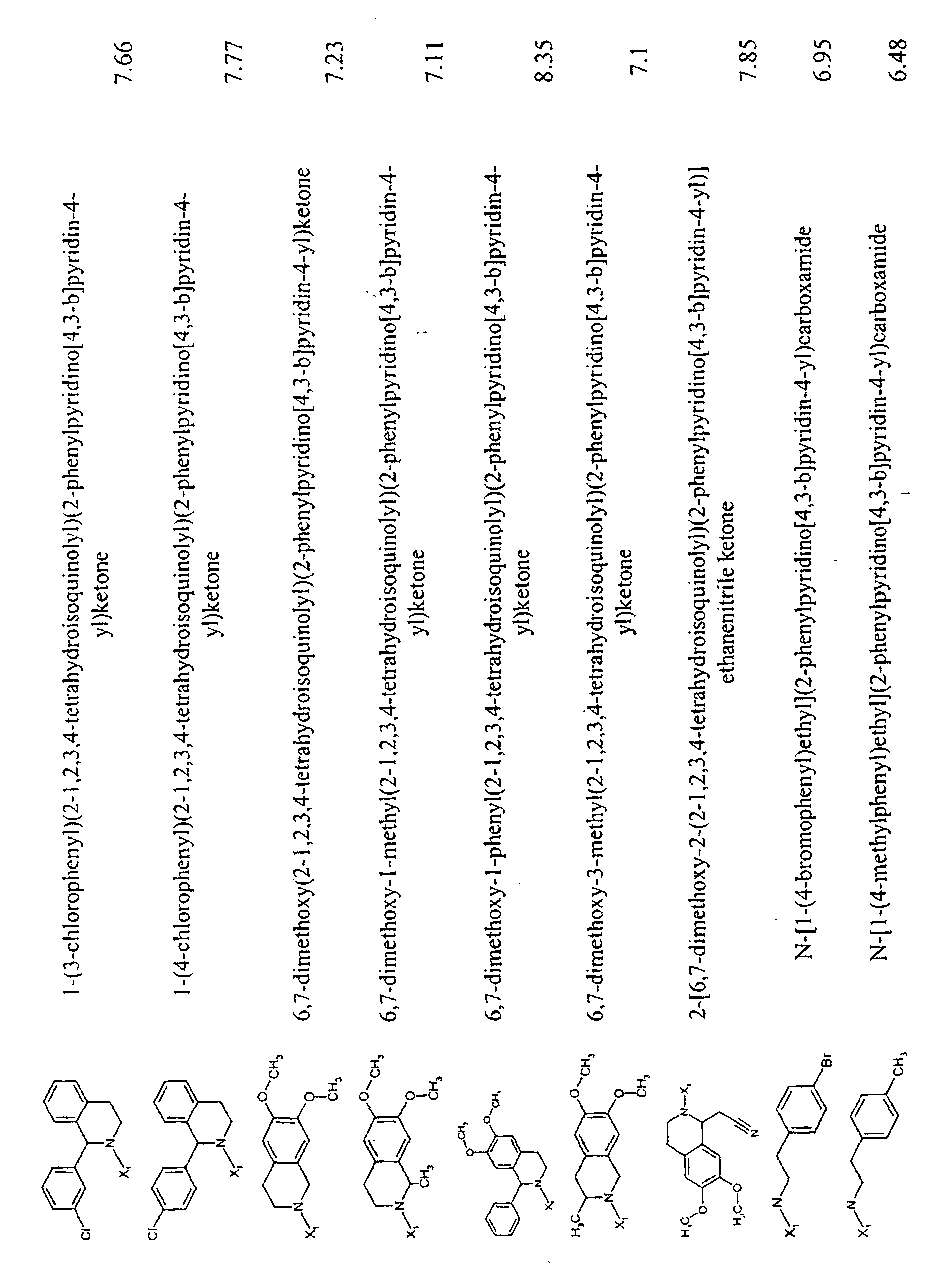

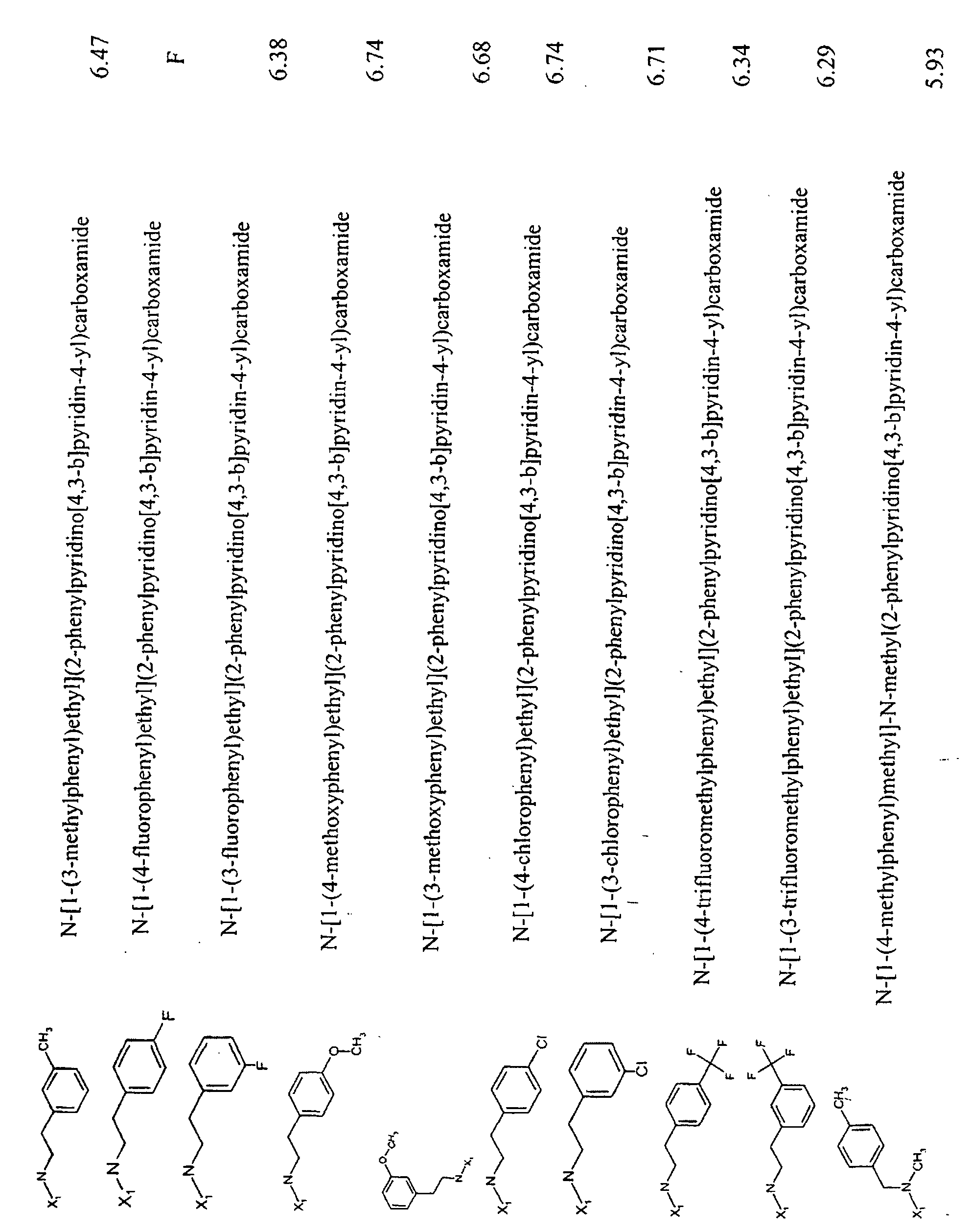

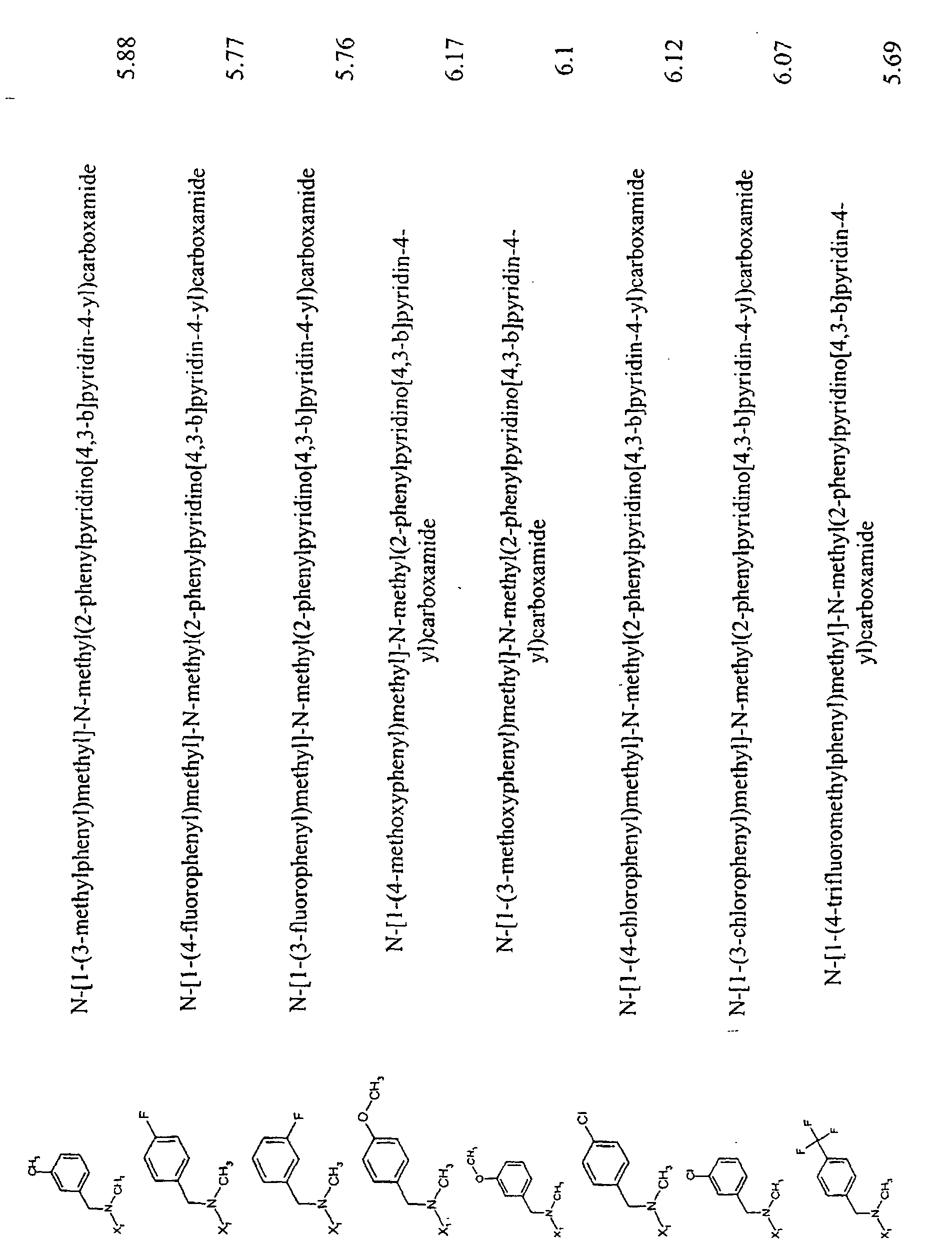

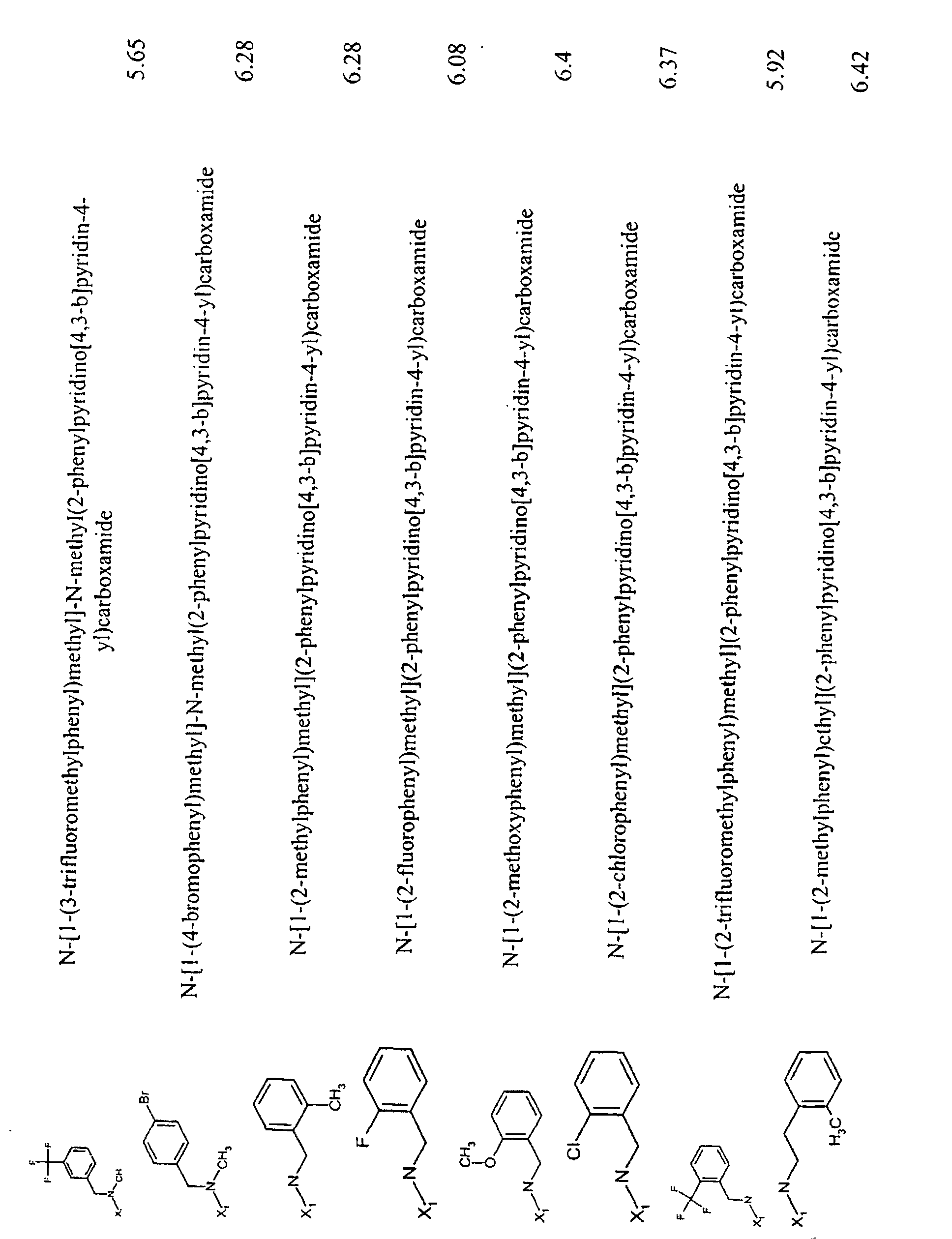

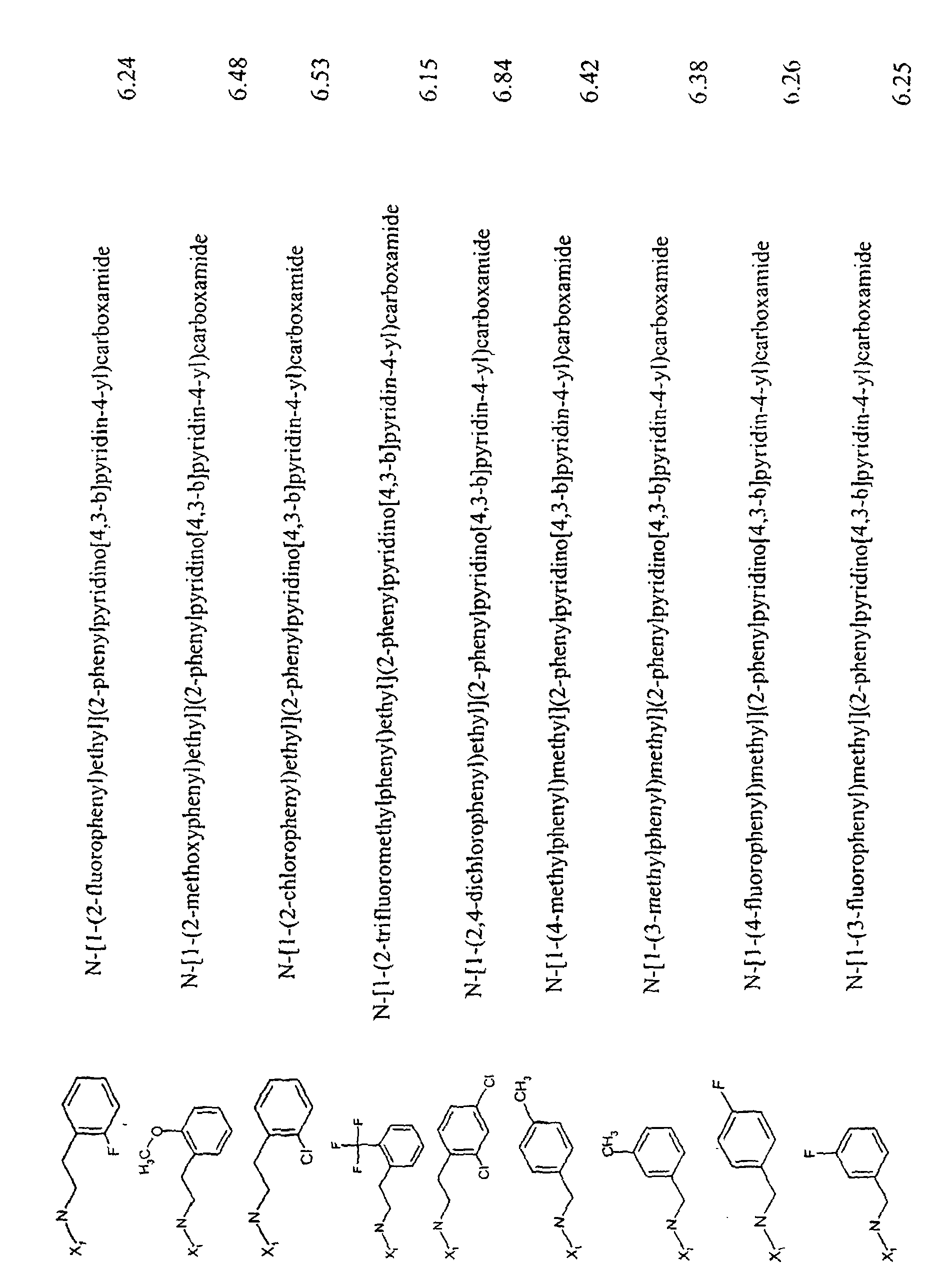

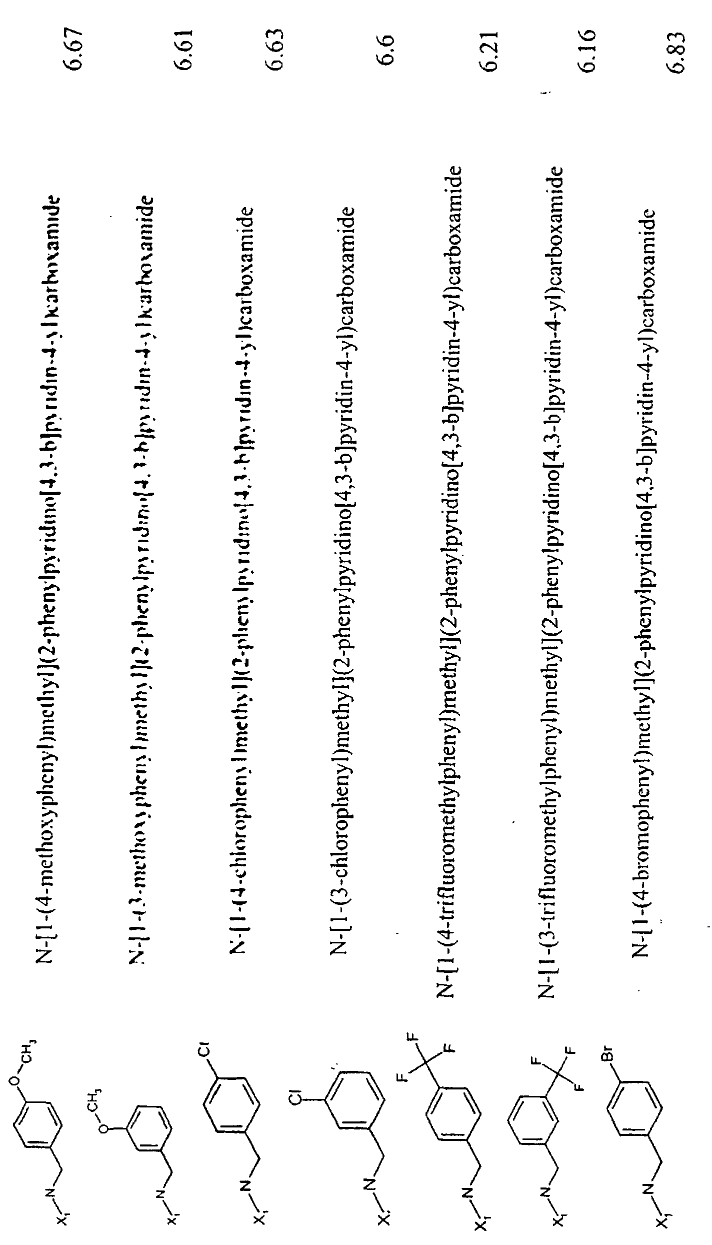

The compounds in Table 2c may be prepared according to Scheme 2. For example, the appropriate acid is treated with diamine in the presence of BOP coupling reagent and base to form an aminoamide derivative. In a subsequent step, this material is treated with carbon tetrachloride in the presence of triphenylphosphine and triethylamine at room temperature to reflux over 2-18 h.

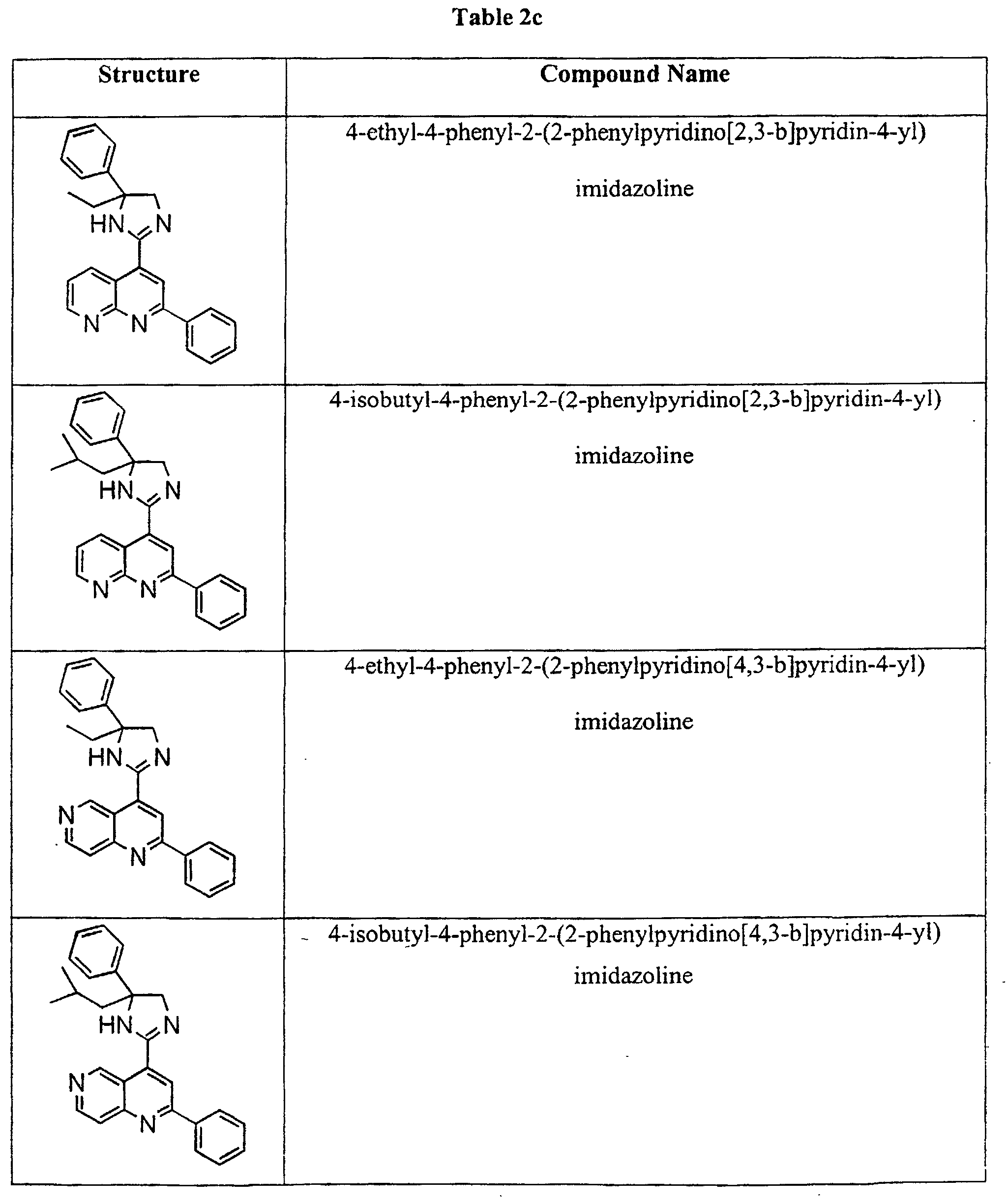

### Example IX

The following assay is a standard assay of NK3 receptor binding, which is used to determine the NK-3 receptor binding affinity of compounds.

Assays are performed as described in Krause et al (Proc. Natl. Acad. Sci. USA 94: 310-315, 1997). The NK-3 receptor complementary DNA was cloned from human hypothalamic RNA using standard procedures. The receptor cDNA was inserted into the expression vector pM² to transfect the mammalian Chinese hamster ovary cell line, and a stably expressing clonal cell line was isolated, characterized and used for the current experiments. Cells are grown in minimal essential medium alpha containing 10% fetal bovine serum and 0.8 mg G418 per ml. Cells were liberated from cell culture plates with No-zyme (PBS base, JRH Biosciences), and harvested by low speed centrifugation. The cell pellet was homogenized in TBS (0.05 m TrisHCl, 120 mM NaCI, pH 7.4) with a Polytron homogenizer at setting 5 for 20 seconds, and total cellular membranes were isolated by centrifugation at 47,500 x g for 10 minutes. The membrane pellet was resuspended by homogenization with the Polytron as above, and the membranes were isolated by centrifugation at 47,500 x g for 10 minutes. This final membrane pellet was resuspended in TBS at a protein concentration of 350 µg/ml.

Receptor binding assays contain a total volume of 200 µl containing 50 µg membrane protein, 0.15 nM ¹²⁵I-methylPhe⁷-neurokinin B, drug or blocker in TBS containing 1.0 mg/ml bovine serum albumen, 0.2 mg/ml bacitracin, 20 ug/ml leupeptin and 20 µg/ml chymostatin. Incubations are carried out for 2 hours at 4°C, and the membrane proteins are harvested by passing the incubation mixture by rapid filtration over presoaked GF/B filters to separate bound from free ligand. The filters are presoaked in TBS containing 2% BSA and 0.1% Tween 20. After filtration of the incubation mixture, filters are rinsed 4 times with ice-cold TBS containing 0.01% sodium dodecyl sulfate and counted in a β-plate scintillation counter. One µM methylPhe⁷-neurokinin B is added to some tubes to determine nonspecific binding. Data are collected in duplicate determinations, averaged, and the percent inhibition of total specific binding is calculated. The total specific binding is the total binding minus the nonspecific binding. In many cases, the concentration of unlabeled drug is varied and total displacement curves of binding is carried out. Data are converted to a form for the calculation of IC₅₀ and Hill coefficient (nH). Data for compounds of this invention are listed in Table 3.

**TABLE 3**

| **Compound Number**^{**1**} | **IC**_{**50**}**(uM)** |
|---|---|
| 1 | 0.05 |
| 2 | 0.08 |
| 3 | 0.10 |

| | |
|---|---|
| ¹Compound numbers correspond to the compounds presented in Table 1 above. | |

### Example X

### Preparation of radiolabeled probe compounds of the invention

The compounds of the invention are prepared as radiolabeled probes by carrying out their synthesis using precursors comprising at least one atom that is a radioisotope. The radioisotope is preferably selected from of at least one of carbon (preferably ¹⁴C), hydrogen (preferably ³H), sulfur (preferably ³⁵S), or iodine (preferably ¹²⁵I). A commercial laboratory specializing in custom synthesis of radiolabeled probe compounds conveniently carries out synthesis of such radiolabeled probes. Such laboratories include Amersham Corporation, Arlington Heights, IL; Cambridge Isotope Laboratories, Inc. Andover, MA; SRI International, Menlo Park, CA; Wizard Laboratories, West Sacramento, CA; ChemSyn Laboratories, Lexena, KS; American Radiolabeled Chemicals, Inc., St. Louis, MO; and Moravek Biochemicals Inc., Brea, CA.

Tritium-labeled probe compounds are also conveniently prepared catalytically-via platinum-catalyzed exchange in tritiated acetic acid, acid-catalyzed exchange in tritiated trifluoroacetic acid, or heterogeneous-catalyzed exchange with tritium gas. Such preparations are also conveniently carried out as a custom radiolabeling by any of the suppliers listed in the preceding paragraph using the compound of the invention as substrate. In addition, certain precursors may be subjected to tritium-halogen exchange with tritium gas, tritium gas reduction of unsaturated bonds, or reduction using sodium borotritide, as appropriate.

### Example XI

### Use of compounds of the invention as probes for detecting NK-3 receptors in cultured cells and tissue samples (e.g., tissue sections)

Receptor autoradiography (receptor mapping) of NK-3 receptors in cultured cells or tissue samples is carried out in vitro as described by Kuhar in sections 8.1.1 to 8.1.9 of Current Protocols in Pharmacology (1998) John Wiley & Sons, New York, using radiolabeled compounds of the invention prepared as described in the preceding Example.

The invention and the manner and process of making and using it, are now described in such full, clear, concise and exact terms as to enable any person skilled in the art to which it pertains, to make and use the same. It is to be understood that the foregoing describes preferred embodiments of the present invention and that modifications may be made therein without departing from the spirit or scope of the present invention as set forth in the claims. To particularly point out and distinctly claim the subject matter regarded as invention, the following claims conclude this specification.

## Claims

1. A compound of the formula: 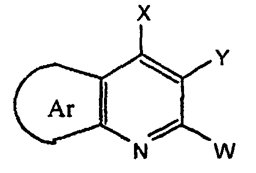 or pharmaceutically acceptable non-toxic salts or pharmaceutically acceptable solvates thereof wherein:
Y is
hydrogen, straight or branched chain lower alkyl having 1-6 carbon atoms, halogen, amino, hydroxyl, or lower alkoxy having 1-6 carbon atoms; or
Y is
straight or branched chain lower alkyl having 1-6 carbon atoms or lower alkoxy having 1-6 carbon atoms each of which is substituted on the alkyl chain with an amino or mono or dialkylamino group where each alkyl is independently lower alkyl;
W is
phenyl, thienyl, or pyridyl, each of which may be mono or disubstituted with halogen, cyano, trifluoromethyl, trifluoromethoxy, hydroxy, straight or branched chain lower alkyl having 1-6 carbon atoms, straight or branched chain lower alkoxy having 1-6 carbon atoms, amino, or mono or dialkylamino where each alkyl is straight or branched chain lower alkyl having 1-6 carbon atoms; or
W is
phenyl(C₁-C₆)alkyl, thienyl(C₁-C₆)alkyl, or pyridyl(C₁-C₆)alkyl, where the aromatic portion of each is optionally mono or disubstituted with halogen, cyano, trifluoromethyl, trifluoromethoxy, hydroxy, straight or branched chain lower alkyl having 1-6 carbon atoms, straight or branched chain lower alkoxy having 1-6 carbon atoms, amino, or mono or dialkylamino where each alkyl is straight or branched chain lower alkyl having 1-6 carbon atoms; or
W is
piperidinyl, morpholinyl, thiomorpholinyl, pyrrolindinyl, piperazinyl, homopiperazinyl, azabicyclo[3.2.2]nonanyl, isoindolinyl, each of which is unsubstituted, or mono or disubstituted with alkyl or aryl groups, or fused to an aromatic ring; or
W is
nitrogen that is mono or disubstituted with hydrogen, straight or branched alkyl chain consisting of 1 to 8 carbons, cycloalkyl containing 3 to 8 carbon atoms, alkyl chain having 1 to 6 carbon atoms that is attached at any position to an aryl ring, alkyl chain having 1 to 6 carbon atoms that is attached at any position to a cycloalkyl containing 3 to 8 carbon atoms, phenyl, thienyl, pyridyl, imidazolyl, benzimidazolyl, or any heterocycle having aromatic character; or
W is
oxygen that is substituted with
straight or branched alkyl chain having 1 to 8 carbon atoms, cycloalkyl having 3 to 8 carbon atoms, or phenyl mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms; 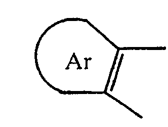 represents 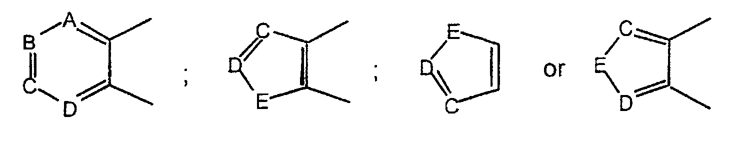
wherein:
A represents nitrogen or C-R₁
B represents nitrogen or C-R₂;
C represents nitrogen or C-R₃;
D represents nitrogen or C-R₄ with the proviso that if all four of A, B, C and D are present, at least one but not more than 2 of A, B, C, and D are nitrogen; and
E represents oxygen, sulfur or N-R₅;
R₁ through R₄ are the same or different and represent
hydrogen, halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms:
R₅ is
hydrogen or straight or branched chain lower alkyl having 1-6 carbon atoms; or
phenyl, thienyl, or pyridyl, each of which may be mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms;
X is 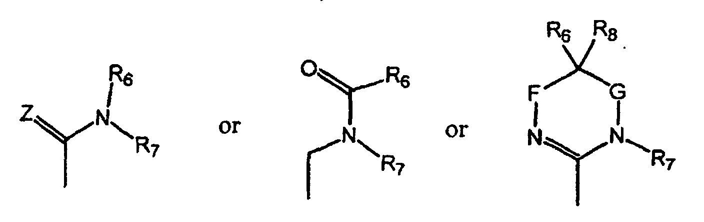
where
F is carbonyl, methylene or ethylene;
G is methylene or a bond with the proviso that G is a bond when F is ethylene; and
Z is oxygen, sulfur, N-C≡N, or two hydrogen atoms;
R₆ is
phenyl, thienyl, or pyridyl, each of which may be mono or disubstituted with halogen, cyano, trifluoromethyl, trifluoromethoxy, hydroxy, straight or branched chain lower alkyl having 1-6 carbon atoms, amino, mono or dialkylamino where each alkyl is straight or branched chain lower alkyl having 1-6 carbon atoms, or straight or branched chain lower alkoxy having 1-6 carbon atoms; or
R₆ is
straight or branched alkyl having 1-8 carbons or cycloalkyl containing 3 to 8 carbon atoms each of which is optionally mono or disubstituted with an aromatic ring where each aromatic ring is optionally mono or disubstituted independently with halogen, cyano, trifluoromethyl, trifluoromethoxy, hydroxy, straight or branched chain lower alkyl having 1-6 carbon atoms, amino, mono or disubstituted amino where the substituent on nitrogen is either straight or branched chain alkyl having 1-6 carbon atoms or straight or branched chain alkoxy having 1-6 carbon atoms; or
R₆ is
cycloalkyl containing 5 to 8 carbon atoms wherein the cycloalkyl is fused to an aromatic ring at adjacent positions;
R₇ and R₈ are the same or different and represent
hydrogen or straight or branched chain lower alkyl having 1-6 carbon atoms; or
R₆ and R₇ taken together form a 5, 6, 7 or 8 membered ring which is mono or disubstituted with an aryl group or contains a fused aryl ring as part of the 5,6, 7 or 8 membered ring described above with the proviso that R₆ and R₇ taken together do not form a ring when G is a methylene; or
R₆ and R₈ taken together may form a 5, 6, 7 or 8 membered ring which is mono or disubstituted with an ary] group or contains a fused aryl ring as part of the 5,6, 7 or 8 membered ring.

2. A compound according to Claim 1 , which is N-[(S)-1-phenyl-1-propyl)(2-phenylpyridino[4,3-b]pyridin-4-yl)carboxamide;
N-[(R)-1-phenyl-1-propyl)(2-phenylpyridino[4,3-b]pyridin-4-yl)carboxamide;
N-((S)- 1-phenyl-1-propyl)(2-phenylpyridino[2,3-b]pyridin-4-yl)carboxamide;
N-((R)-1-phenyl-1-propyl)(2-phenylpyridino[2,3-b]pyridin-4-yl)carboxamide;
N-(Diphenylmethyl)N-methyl(2-phenylpyridino[2,3-b]pyridin-4-yl)carboxamide.
N-(Diphenylmethyl)N-methyl(2-phenylpyridino[4,3-b]pyridin-4-yl)carboxamide;
N-((S)-1-phenyl-1-propyl)(2-phenylthieno[3,2-b]pyridin-4-yl)carboxamide;
N-[1-(phenyl)ethyl)-N-methyl(2-phenylpyridino[2,3-b]pyriden-4-yl)carboxamide;
N-[1-(phenyl)butyl]-N-methyl(2-phenylpyridino[2,3-b]pyridin-4-yl)carboxamide;
N-[1-(phenyl)pentyl]-N-methyl(2-phenylpyridino[2,3-b]pyridin)-4-yl)carboxamide;
N-[1-(phenyl)hexyl]-N-methyl(2-phenylpyridino[2,3-b]pyridin-4-yl)carboxamide;
N-[1-(diphenyl)methyl]-N-methyl(2-phenylpyridino[2,3-b]pyridin-4-yl)carboxamide;
N-[1-(1,2-diphenyl)ethyl]-N-methyl(2-phenylpyridino[2,3-b]pyridin-4-yl)carboxamide;
N-[1-(1-phenyl-2-cyclohexyl)ethyl]-N-methyl(2-phenylpyridino[2,3-b]pyridin-4-yl)carboxamide;
N-indanyl-N-methyl(2-phenylpyridino[2,3-b]pyridin-4-yl)carboxaniide;
N-[1-(phenyl)isobutyl]-N-methyl(2-phenylpyridino[2,3-b]pyridin-4-yl)carboxamide;
N-[1-(phenyl)2-methylbutyl]-N-methyl(2-phenylpyridino[2,3-b]pyridin-4-yl)carboxamide;
1-isobutyl(2-1,2,3,4-tetrahydroisoquinolyl)(2-phenylpyridino[2,3-b]pyridin-4-yl)ketone;
1-(2-methylphenyl)(2-1,2,3,4-tetrahydroisoquinolyl)(2-phenylpyridino[2,3-b]pyridin-4-yl)ketone;
1-(2-chlorophenyl)(2-1,2,3,4-tetrahydroisoquinolyl)(2-phenylpyridino[2,3-b]pyridin-4-yl)ketone;
1-methyl(2-1,2,3,4-tetrahydroisoquinolyl)(2-phenylpyridino[2,3-b]pyridin-4-yl)ketone;
1-propyl(2-1,2,3,4-tetrahydroisoquinolyl)(2-phenylpyridino[2,3-b]pyridin-4-yl)ketone;
1-butyl(2-1,2,3,4-tetrahydroisoquinolyl)(2-phenylpyridino[2,3-b]pyridin-4-yl)ketone;
1-cyclopentyl(2-1,2,3,4-tetrahydroisoquinolyl)(2-phenylpyridino[2,3-b]pyridin-4-yl)ketone;
1-pentyl(2-1,2,3,4-tetrahydroisoquinolyl)(2-phenylpyridino[2,3-b]pyridin-4-yl)ketone;
1-benzyl(2-1,2,3,4-tetrahydroisoquinolyl)(2-phenylpyridino[2,3-b]pyridin-4-yl)ketone;
1-cyclohexylmethyl(2-1,2,3,4-tetrahydroisoquinolyl)(2-phenylpyridino[2,3-b]pyridin-4-yl)ketone;
1-isopropyl(2-1,2,3,4-tetrahydroisoquinolyl)(2-phenylpyridino[2,3-b]pyridin-4-yl)ketone;
N-[1-(phenyl)octyl]-N-methyl(2-phenylpyridino[2,3-b]pyridin-4-yl)carboxamide;
1-(2-methylbutyl)(2-1,2,3,4-tetrahydroisoquinolyl)(2-phenylpyridino[2,3-b]pyridin-4-yl)ketone;
N-[1,3-(diphenyl)propyl]-N-methyl(2-phenylpyridino[2,3-b]pyridin-4-yl)carboxamide;
N-[1,4-(diphenyl)butyl]-N-methyl(2-phenylpyridino[2,3-b]pyridin-4-yl)carboxamide;
N-benzyl-N-methyl(2-phenylpyridino[2,3-b]pyridin-4-yl)carboxamide;
N-benzyl-N-ethyl(2-phenylpyridino[2,3-b]pyridin-4-yl)carboxamide;
N-benzyl-N-isopropyl(2-phenylpyridino[2,3-b]pyridin-4-yl)carboxamide;
N-benzyl-N-butyl(2-phenylpyridino[2,3-b]pyridin-4-yl)carboxamide;
N-benzyl-N-isobutyl(2-phenylpyridino[2,3-b]pyridin-4-yl)carboxamide;
N-benzyl-N-cyclopentyl(2-phenylpyridino[2,3-b]pyridin-4-yl)carboxamide;
N-benzyl-N-pentyl(2-phenylpyridino[2,3-b]pyridin-4-yl)carboxamide;
N-benzyl-N-cyclohexyl(2-phenylpyridino[2,3-b]pyridin-4-yl)carboxamide.
N,N-dibenzyl(2-phenylpyridino[2,3-b]pyridin-4-yl)carboxamide;
N-benzyl-N-1-methylpropyl(2-phenylpyridino[2,3-b]pyridin-4-yl)carboxamide;
N-benzyl-N-1-methylbutyl(2-phenylpyridino[2,3-b]pyridin-4-yl)carboxamide;
N-benzyl-N-4-methylbutyl(2-phenylpyridino[2,3-b]pyridin-4-yl)carboxamide;
N-benzyl-N-cyclopentylmethyl(2-phenylpyridino[2,3-b]pyridin-4-yl)carboxamide;
N-benzyl-N-hexyll(2-phenylpyridino[2,3-b]pyridin-4-yl)carboxamide;
N-benzyl-N-cyclohexylmethyl(2-phenylpyridino[2,3-b]pyridin-4-yl)carboxamide;
N-benzyl-N-heptyl(2-phenylpyridino[2,3-b]pyridin-4-yl)carboxamide;
N-methyl(2-phenylpyridino[2,3-b]pyridin-4-yl)carboxamide;
N-[1-(phenylcyclopentyl)methyl]-N-methyl(2-phenylpyridino[2,3-b]pyridin-4-yl)carboxamide;
N-[1-(phenylcyclohexyl)methyl]-N-methyl(2-phenylpyridino[2,3-b]pyridin-4-yl)carboxamide;
N-[1-(1-phenyl-2cyclopentyl)ethyl]-N-methyl(2-phenylpyridino[2,3-b]pyridin-4-yl)carboxamide;
N-[1-(1,3-diphenyl)propyl]-N-methyl(2-phenylpyridino[2,3-b]pyridin-4-yl)carboxamide;
N-[1-(1,4-diphenyl)butyl]-N-methyl(2-phenylpyridino[2,3-b]pyridin-4-yl)carboxamide;
1-phenyl(2-1,2,3,4-tetrahydroisoquinolyl)(2-phenylpyridino[2,3-b]pyridin-4-yl)ketone;
1-cyclohexyl(2-1,2,3,4-tetrahydroisoquinolyl)(2-phenylpyridino[2,3-b]pyridin-4-yl)ketone;
I -cyclopentylmethyl(2-1,2,3,4-tetrahydroisoquinolyl)(2-phenylpyridino[2,3-b]pyridin-4-yl)ketone;
1-hexyl(2-1,2,3,4-tetrahydroi soquinolyl)(2-phenylpyridino[2,3-b]pyridin-4-yl)ketone;
1-(2-fluorophenyl)(2-1,2,3,4-tetrahydroisoquinolyl)(2-phenylpyridino[2,3-b]pyridin-4-yl)ketone;
1-(2-trifluoromethylphenyl)(2-1,2,3,4-tetrahydroisoquinolyl)(2-phenylpyridino[2,3-b]pyridin-4-yl)ketone.
1-(3-methylphenyl)(2-1,2,3,4-tetrahydroisoquinolyl)(2-phenylpyridino[2,3-b]pyridin-4-yl)ketone;
1-(4-methylphenyl)(2-1,2,3,4-tetrahydroisoquinolyl)(2-phenylpyridino[2,3-b]pyridin-4-yl)ketone;
1-(4-fluorophenyl)(2-1,2,3,4-tetrahydroisoquinolyl)(2-phenylpyridino[2,3-b]pyridin-4-yl)ketone;
1-cycloheptyl(2-1,2,3,4-tetrahydroisoquinolyl)(2-phenylpyridino[2,3-b]pyridin-4-yl)ketone;
1-(3-chlorophenyl)(2-1,2,3,4-tetrahydroisoquinolyl)(2-phenylpyridino[2,3-b]pyridin-4-yl)ketone;
1-(4-chlorophenyl)(2-1,2,3,4-tetrahydroisoquinolyl)(2-phenylpyridino[2,3-b]pyridin-4-yl)ketone;
6,7-dimethoxy(2-1,2,3,4-tetrahydroisoquinolyl)(2-phenylpyridino[2,3-b]pyridin-4-yl)ketone;
6,7-dimethoxy-1-methyl(2-1,2,3,4-tetrahydroisoquinolyl)(2-phenylpyridino[2,3-b]pyridin-4-yl)ketone;
6,7-dimethoxy-1-phenyl(2-1,2,3,4-tetrahydroisoquinolyl)(2-phenylpyridino[2,3-b]pyridin-4-yl)ketone;
6,7-dimethoxy-3-methyl(2-1,2,3,4-tetrahydroisoquinolyl)(2-phenylpyridino[2,3-b]pyridin-4-yl)ketone.
2-[6,7-dimethoxy-2-(2-1,2,3,4-tetrahydroisoquinolyl)(2-phenylpyridino[2,3-b]pyridin-4-yl)] ethanenitrile ketone;
N-[1-(4-bromophenyl)ethyl](2-phenylpyridino[2,3-b]pyridin-4-yl)carboxamide;
N-[1-(4-methylphenyl)ethyl](2-phenylpyridino[2,3-b]pyridin-4-yl)carboxamide;
N-[1-(3-methylphenyl)ethyl](2-phenylpyridino[2,3-b]pyridin-4-yl)carboxamide;
N-[1-(4-fluorophenyl)ethyl](2-phenylpyridino[2,3-b]pyridin-4-yl)carboxamide;
N-[1-(3-fluorophenyl)ethyl](2-phenylpyridino[2,3-b]pyridin-4-yl)carboxamide;
N-[1-(4-methoxyphenyl)ethyl](2-phenylpyridino[2,3-b]pyridin-4-yl)carboxamide;
N-[1-(3-methoxyphenyl)ethyl](2-phenylpyridino[2,3-b]pyridin-4-yl)carboxamide;
N-[1-(4-chlorophenyl)ethyl](2-phenylpyridino[2,3-b]pyridin-4-yl)carboxamide;
N-[1-(3-chlorophenyl)ethyl](2-phenylpyridino[2,3-b]pyridin-4-yl)carboxamide;
N-[1-(4-trifluoromethylphenyl)ethyl](2-phenylpyridino[2,3-b]pyridin-4-yl)carboxamide;
N-[1-(3-trifluoromethylphenyl)ethyl](2-phenylpyridino[2,3-b]pyridin-4-yl)carboxamide;
N-[1-(4-methylphenyl)methyl]-N-methyl(2-phenylpyridino[2,3-b]pyridin-4-yl)carboxamide;
N-[1-(3-methylphenyl)methyl]-N-methyl(2-phenylpyridino[2,3-b]pyridin-4-yl)carboxamide;
N-[1-(4-fluorophenyl)methyl]-N-methyl(2-phenylpyridino[2,3-b]pyridin-4-yl)carboxamide;
N-[1-(3-fluorophenyl)methyl]-N-methyl(2-phenylpyridino[2,3-b]pyridin-4-yl)carboxamide;
N-[1-(4-methoxyphenyl)methyl]-N-methyl(2-phenylpyridino[2,3-b]pyridin-4-yl)carboxamide;
N-[1-(3-methoxyphenyl)methyl]-N-methyl(2-phenylpyridino[2.3-b]pyridin-4-yl)carboxamide;
N-[1-(4-chlorophenyl)methyl]-N-methyl(2-phenylpyridino[2,3-b]pyridin-4-yl)carboxamide;
N-[1-(3-chlorophenyl)methyl]-N-methyl(2-phenylpyridino[2,3-b]pyridin-4-yl)carboxamide.
N-[1-(4-trifluoromethylphenyl)methyl]-N-methyl(2-phenylpyridino[2,3-b]pyridin-4-yl)carboxamide;
N-[1-(3-trifluoromethylphenyl)methyl]-N-methyl(2-phenylpyridino[2,3-b]pyridin-4-yl)carboxamide;
N-[ 1-(4-bromophenyl)methyl]-N-methyl(2-phenylpyridino[2,3-b]pyridin-4-yl)carboxamide;
N-[1-(2-methylphenyl)methyl](2-phenylpyridino[2,3-b]pyridin-4-yl)carboxamide;
N-[1-(2-fluorophenyl)methyl](2-phenylpyridino[2,3-b]pyridin-4-yl)carboxamide;
N-[1-(2-methoxyphenyl)methyl](2-phenylpyridino[2,3-b]pyridin-4-yl)carboxamide;
N-[1-(2-chlorophenyl)methyl](2-phenylpyridino[2,3-b]pyridin-4-yl)carboxamide;
N-[1-(2-trifluoromethylphenyl)methyl](2-phenylpyridino[2,3-b]pyridin-4-yl)carboxamide;
N-[1-(2-methylphenyl)ethyl](2-phenylpyridino[2,3-b]pyridin-4-yl)carhoxamide;
N-[1-(2-fluorophenyl)ethyl](2-phenylpyridino[2,3-b]pyridin-4-yl)carboxamide;
N-[1-(2-methoxyphenyl)ethyl](2-phenylpyridino[2,3-b]pyridin-4-yl)carboxamide;
N-[1-(2-chlorophenyl)ethyl](2-phenylpyridino[2,3-b]pyridin-4-yl)carboxamide;
N-[1-(2-trifluoromethylphenyl)ethyl](2-phenylpyridino[2,3-b]pyridin-4-yl)carboxamide;
N-[1-(2,4-dichlorophenyl)ethyl](2-phenylpyridino[2,3-b]pyridin-4-yl)carboxamide;
N-[1-(4-methylphenyl)methyl](2-phenylpyridino[2,3-b]pyridin-4-yl)carboxamide;
N-[1-(3-methylphenyl)methyl](2-phenylpyridino[2,3-b]pyridin-4-yl)carboxamide;
N-[1-(4-fluorophenyl)methyl](2-phenylpyridino[2,3-b]pyridin-4-yl)carboxamide;
N-[1-(3-fluorophenyl)methyl](2-phenylpyridino[2,3-b]pyridin-4-yl)carboxamide;
N-[1-(4-methoxyphenyl)methyl](2-phenylpyridino[2,3-b]pyridin-4-yl)carboxamide;
N-[1-(3-methoxyphenyl)methyl](2-phenylpyridino[2,3-b]pyridin-4-yl)carboxamide;
N-[1-(4-chlorophenyl)methyl](2-phenylpyridino[2,3-b]pyridin-4-yl)carboxamide;
N-[1-(3-chlorophenyl)methyl](2-phenylpyridino[2,3-b]pyridin-4-yl)carboxamide;
N-[1-(4-trifluoromethylphenyl)methyl](2-phenylpyridino[2,3-b]pyridin-4-yl)carboxamide;
N-[1-(3-trifluoromethylphenyl)methyl](2-phenylpyridino[2,3-b]pyridin-4-yl)carboxamide;
N-[1-(4-bromophenyl)methyl](2-phenylpyridino[2,3-b]pyridin-4-yl)carboxamide;
N-[1-(phenyl)ethyl]-N-methyl(2-phenylpyridino[4,3-b]pyridin-4-yl)carboxamide;
N-[1-(phenyl)butyl]-N-methyl(2-phenylpyridino[4,3-b]pyridin-4-yl)carboxamide;
N-[1-(phenyl)pentyl]-N-methyl(2-phenylpyridino[4,3-b]pyridin-4-yl)carboxamide;
N-[1-(phenyl)hexyl]-N-methyl(2-phenylpyridino[4,3-b]pyridin-4-yl)carboxamide;
N-[1-(diphenyl)methyl]-N-methyl(2-phenylpyridino[4,3-b]pyridin-4-yl)carboxamide;
N-[1-(1,2-diphenyl)ethyl)-N-methyl(2-phenylpyridino[4,3-b]pyridin-4-yl)carboxamide;
N-[1-(1-phenyl-2-cyclohexyl)ethyl]-N-methyl(2-phenylpyridino[4,3-b]pyridin-4-yl)carboxamide;
N-indanyl-N-methyl(2-phenylpyridino[4,3-b]pyridin-4-yl)carboxamide;
N-[ 1-(phenyl)isobutyl]-N-methyl(2-phenylpyridino[4,3-b]pyridin-4-yl)carboxamide;
N-[1-(phenyl)2-methylbutyl]-N-methyl(2-phenylpyridino[4,3-b]pyridin-4-yl)carboxamide;
1-isobutyl(2-1,2,3,4-tetrahydroisoquinolyl)(2-phenylpyridino[4,3-b]pyridin-4-yl)ketone;
1-(2-methylphenyl)(2-1,2,3,4-tetrahydroisoquinolyl)(2-phenylpyridino[4,3-b]pyridin-4-yl)ketone;
1 -(2-chlorophenyl)(2-1,2,3,4-tetrahydroisoquinolyl)(2-phenylpyridino[4,3-b]pyridin-4-yl)ketone;
1-methyl(2-1,2,3,4-tetrahydroisoquinolyl)(2-phenylpyridino[4,3-b]pyridin-4-yl)ketone;
1-propyl(2-1,2,3,4-tetrahydroisoquinolyl)(2-phenylpyridino[4,3-b]pyridin-4-yl)ketone;
1-butyl(2-1,2,3,4-tetrahydroisoquinolyl)(2-phenylpyridino[4,3-b]pyridin-4-yl)ketone;
1-cyclopentyl(2-1,2,3,4-tetrahydroisoquinolyl)(2-phenylpyridino[4,3-b]pyridin-4-yl)ketone;
1-pentyl(2-1,2,3,4-tetrahydroisoquinolyl)(2-phenylpyridino[4,3-b]pyridin-4-yl)ketone;
1-benzyl(2-1,2,3,4-tetrahydroisoquinolyl)(2-phenylpyridino[4,3-b]pyridin-4-yl)ketone;
1-cyclohexylmethyl(2-1,2,3,4-tetrahydroisoquinolyl)(2-phenylpyridino[4,3-b]pyridin-4-yl)ketone;
1-isopropyl(2-1,2,3,4-tetrahydroisoquinolyl)(2-phenylpyridino[4,3-b]pyridin-4-yl)ketone; and
N-[1-(phenyl)octyl]-N-methyl(2-phenylpyridino[4,3-b]pyridin-4-yl)carboxamide;
1-(2-methylbutyl)(2-1,2,3,4-tetrahydroisoquinolyl)(2-phenylpyridino[4,3-b]pyridin-4-yl)ketone;
N-[1,3-(diphenyl)propyl]-N-methyl(2-phenylpyridino[4,3-b]pyridin-4-yl)carboxamide;
N-[1,4-(diphenyl)butyl]-N-methyl(2-phenylpyridino[4,3-b]pyridin-4-yl)carboxamide;
N-benzyl-N-methyl(2-phenylpyridino[4,3-b]pyridin-4-yl)carboxamide;
N-benzyl-N-ethyl(2-phenylpyridino[4,3-b]pyridin-4-yl)carboxamide;
N-benzyl-N-isopropyl(2-phenylpyridino[4,3-b]pyridin-4-yl)carboxamide;
N-benzyl-N-butyl(2-phenylpyridino[4,3-b]pyridin-4-yl)carboxamide;
N-benzyl-N-isobutyl(2-phenylpyridino[4,3-b]pyridin-4-yl)carboxamide;
N-benzyl-N-cyclopentyl(2-phenylpyridino[4,3-b]pyridin-4-yl)carboxamide;
N-benzyl-N-pentyl(2-phenylpyridino[4,3-b]pyridin-4-yl)carboxamide,
N-methyl(2-phenylpyridino[4,3-b]pyridin-4-yl)carboxamide;
N-[ 1-(phenylcyclopentyl)methyl]-N-methyl(2-phenylpyridino[4,3-b]pyridin-4-yl)carboxamide;
N-[1-(phenylcyclohexyl)methyl]-N-methyl(2-phenylpyridino[4,3-b]pyridin-4-yl)carboxamide;
N-[1-(1-phenyl-2cyclopentyl)ethyl]-N-methyl(2-phenylpyridino[4,3-b]pyridin-4-yl)carboxamide;
N-[1-(1,3-diphenyl)propyl]-N-methyl(2-phenylpyridino[4,3-b]pyridin-4-yl)carboxamide;
N-[1-(1,4-diphenyl)butyl]-N-methyl(2-phenylpyridino[4,3-b]pyridin-4-yl)carboxamide;
1-phenyl(2-1,2,3,4-tetrahydroisoquinolyl)(2-pheny]pyridino[4,3-b]pyridin-4-yl)ketone;
1-cyclohexyl(2-1,2,3,4-tetrahydroisoquinolyl)(2-phenylpyridino[4,3-b]pyridin-4-yl)ketone;
1-cyclopentylmethyl(2-1,2,3,4-tetrahydroisoquinolyl)(2-phenylpyridino[4,3-b]pyridin-4-yl)ketone;
1-hexyl(2-1,2,3,4-tetrahydroisoquinolyl)(2-phenylpyridino[4,3-b]pyridin-4-yl)ketone;
1-(2-fluorophenyl)(2-1,2,3,4-tetrahydroisoquinolyl)(2-phenylpyridino[4,3-b]pyridin-4-yl)ketone;
1-(3-methylphenyl)(2-1,2,3,4-tetrahydroisoquinolyl)(2-phenylpyridino[4,3-b]pyridin-4-yl)ketone;
1-(4-methylphenyl)(2-1,2,3,4-tetrahydroisoquinolyl)(2-phenylpyridino[4,3-b]pyridin-4-yl)ketone;
1-(4-fluorophenyl)(2-1,2,3,4-tetrahydroisoquinolyl)(2-phenylpyridino[4,3-b]pyridin-4-yl)ketone;
1-cycloheptyl(2-1,2,3,4-tetrahydroisoquinolyl)(2-phenylpyridino[4,3-b]pyridin-4-yl)ketone;
1-(3-chlorophenyl)(2-1,2,3,4-tetrahydroisoquinolyl)(2-phenylpyridino[4,3-b]pyridin-4-yl)ketone;
1-(4-chlorophenyl)(2-1,2,3,4-tetrahydroisoquinolyl)(2-phenylpyridino[4,3-b]pyridin-4-yl)ketone;
6,7-dimethoxy(2-1,2,3,4-tetrahydroisoquinolyl)(2-phenylpyridino[4,3-b]pyridin-4-yl)ketone;
6,7-dimethoxy-1-methyl(2-1,2,3,4-tetrahydroisoquinolyl)(2-phenylpyridino[4,3-b]pyridin-4-yl)ketone;
6,7-dimethoxy-1-phenyl(2-1,2,3,4-tetrahydroisoquinolyl)(2-phenylpyridino[4,3-b]pyridin-4-yl)ketone;
6,7-dimethoxy-3-methyl(2-1,2,3,4-tetrahydroisoquinolyl)(2-phenylpyridino[4,3-b]pyridin-4-yl)ketone;
2-[6,7-dimethoxy-2-(2-1,2,3,4-tetrahydroisoquinolyl)(2-phenylpyridino[4,3-b]pyridin-4-yl)] ethanenitrile ketone;
N-[1-(4-bromophenyl)ethyl](2-phenylpyridino[4,3-b]pyridin-4-yl)carboxamide;
N-[1-(4-methylphenyl)ethyl](2-phenylpyridino[4,3-b]pyridin-4-yl)carboxamide;
N-[1-(3-methylphenyl)ethyl](2-phenylpyridino[4,3-b]pyridin-4-yl)carboxamide;
N-[1-(4-fluorophenyl)ethyl](2-phenylpyridino[4,3-b]pyridin-4-yl)carboxamide;
N-[1-(3-fluorophenyl)ethyl](2-phenylpyridino[4,3-b]pyridin-4-yl)carboxamide;
N-[ 1-(4-methoxyphenyl)ethyl](2-phenylpyridino[4,3-b]pyridin-4-yl)carboxamide;
N-[1-(3-methoxyphenyl)ethyl](2-phenylpyridino[4,3-b]pyridin-4-yl)carboxamide;
N-[1-(4-chlorophenyl)ethyl](2-phenylpyridino[4,3-b]pyridin-4-yl)carboxamide;
N-[1-(3-chlorophenyl)ethyl](2-phenylpyridino[4,3-b]pyridin-4-yl)carboxamide;
N-[1-(4-trifluoromethylphenyl)ethyl](2-phenylpyridino[4,3-b]pyridin-4-yl)carboxamide;
N-[1-(3-trifluoromethylphenyl)ethyl](2-phenylpyridino[4,3-b]pyridin-4-yl)carboxamide;
N-[1-(4-methylphenyl)methyl]-N-methyl(2-phenylpyridino[4,3-b]pyridin-4-yl)carboxamide;
N-[ 1-(3-methylphenyl)methyl]-N-methyl(2-phenylpyridino[4,3-b]pyridin-4-yl)carboxamide;
N-[1-(4-fluorophenyl)methyl]-N-methyl(2-phenylpyridino[4,3-b]pyridin-4-yl)carboxamide;
N-[1-(3-fluorophenyl)methyl]-N-methyl(2-phenylpyridino[4,3-b]pyridin-4-yl)carboxamide;
N-[1-(4-methoxyphenyl)methyl]-N-methyl(2-phenylpyridino[4,3-b]pyridin-4-yl)carboxamide;
N-[1-(3-methoxyphenyl)methyl]-N-methyl(2-phenylpyridino[4,3-b]pyridin-4-yl)carboxamide;
N-[1-(4-chlorophenyl)methyl)-N-methyl(2-phenylpyridino[4,3-b]pyridin-4-yl)carboxamide;
N-[1-(3-chlorophenyl)methyl]-N-methyl(2-phenylpyridino[4,3-b]pyridin-4-yl)carboxamide;
N-[ 1-(4-trifluoromethylphenyl)methyl]-N-methyl(2-phenylpyridino[4,3-b]pyridin-4-yl)carboxamide;
N-[1-(3-trifluoromethylphenyl)methyl]-N-methyl(2-phenylpyridino[4,3-b]pyridin-4-yl)carboxamide;
N-[1-(4-bromophenyl)methyl]-N-methyl(2-phenylpyridino[4,3-b]pyridin-4-yl)carboxamide;
N-[1-(2-methylphenyl)methyl](2-phenylpyridino[4,3-b]pyridin-4-yl)carboxamide;
N-[1-(2-fluorophenyl)methyl](2-phenylpyridino[4,3-b]pyridin-4-yl)carboxamide;
N-[1-(2-methoxyphenyl)methyl](2-phenylpyridino[4,3-b]pyridin-4-yl)carboxamide;
N-[1-(2-chlorophenyl)methyl](2-phenylpyridino[4,3-b]pyridin-4-yl)carboxamide;
N-[1-(2-trifluoromethylphenyl)methyl](2-phenylpyridino[4,3-b]pyridin-4-yl)carboxamide;
N-[1-(2-methylphenyl)ethyl](2-phenylpyridino[4,3-b]pyridin-4-yl)carboxamide;
N-[1-(2-fluorophenyl)ethyl](2-phenylpyridino[4,3-b]pyridin-4-yl)carboxamide;
N-[1-(2-methoxyphenyl)ethyl](2-phenylpyridino[4,3-b]pyridin-4-yl)carboxamide;
N-[1-(2-chlorophenyl)ethyl](2-phenylpyridino[4,3-b]pyridin-4-yl)carboxamide;
N-[1-(2-trifluoromethylphenyl)ethyl](2-phenylpyridino[4,3-b]pyridin-4-yl)carboxamide;
N-[1-(2,4-dichlorophenyl)ethyl](2-phenylpyridino[4,3-b]pyridin-4-yl)carboxamide;
N-[1-(4-methylphenyl)methyl](2-phenylpyridino[4,3-b]pyridin-4-yl)carboxamide;
N-[1-(3-methylphenyl)methyl](2-phenylpyridino[4,3-b]pyridin-4-yl)carboxamide;
N-[1-(4-fluorophenyl)methyl](2-phenylpyridino[4,3-b]pyridin-4-yl)carboxamide;
N-[1-(3-fluorophenyl)methyl](2-phenylpyridino[4,3-b]pyridin-4-yl)carboxamide;
N-[1-(4-methoxyphenyl)methyl](2-phenylpyridino[4,3-b]pyridin-4-yl)carboxamide;
N-[1-(3-methoxyphenyl)methyl](2-phenylpyridino[4,3-b]pyridin-4-yl)carboxamide;
N-[1-(4-chlorophenyl)methyl](2-phenylpyridino[4,3-b]pyridin-4-yl)carboxamide.
N-[1-(3-chlorophenyl)methyl](2-phenylpyridino[4,3-b]pyridin-4-yl)carboxamide;
N-[ 1-(4-trifluoromethylphenyl)methyl](2-phenylpyridino[4,3-b]pyridin-4-yl)carboxamide;
N-[1-(3-trifluoromethylphenyl)methyl](2-phenylpyridino[4,3-b]pyridin-4-yl)carboxamide;
N-[1-(4-bromophenyl)methyl](2-phenylpyridino[4,3-b]pyridin-4-yl)carboxamide;
4-ethyl-4-phenyl-2-(2-phenylpyridino[2,3-b]pyridin-4-yl)imidazoline;
4-isobutyl-4-phenyl-2-(2-phenylpyridino[2,3-b]pyridin-4-yl)imidazoline;
4-ethyl-4-phenyl-2-(2-phenylpyridino[4,3-b]pyridin-4-yl)imidazoline; or
4-isobutyl-4-phenyl-2-(2-phenylpyridino[4,3-b]pyridin-4-yl)imidazoline.

3. All stereoisomeric forms of a compound according to claim 1 and mixtures thereof.

4. A compound according to any one of Claims 1 to 3 for use in therapeutic treatment of a disease or disorder associated with pathogenic neurokinin-3 receptor activation.

5. A pharmaceutical composition comprising a compound as claimed in any one of Claims 1 to 4 combined with at least one pharmaceutically acceptable carrier or excipient.

6. The use of a compound as claimed in anyone of Claims 1 to 3 for the manufacture of a medicament for the treatment or prevention of a disease or disorder associated with pathogenic neurokinin-3 receptor activation.

7. The use of a compound as described in Claims 1 to 3 for the manufacture of a medicament for the treatment or prevention of anxiety, depression, obesity, pain, or chronic pulmonary obstructive disorder.

8. A method for localizing neurokinin-3 receptors in a tissue sample comprising: contacting with the sample a detectably-labeled compound of any one of Claims 1 to 3 under conditions that permit binding of the compound to neurokinin 3 receptors, washing the sample to remove unbound compound, and detecting the bound compound.

9. A method of inhibiting the binding of a neurokinin to a neurokinin-3 receptor, said method comprising contacting a compound of any one of Claims 1 to 3 with cells expressing such a receptor in the presence of a neurokinin, wherein the compound is present at a concentration sufficient to inhibit neurokinin binding cells expressing a cloned human NK-3 receptor *in vitro*.

10. A method for altering the signal-transducing activity of neurokinin-3 receptors, said method comprising exposing cells expressing such receptors to a compound according to any one of Claims 1 to 3 at a concentration sufficient to inhibit neurokinin binding cells expressing a cloned human neurokinin-3 receptor *in vitro.*

11. A packaged pharmaceutical composition comprising the pharmaceutical composition of Claim 5 in a container and instructions for using the composition to treat a patient suffering from a disorder responsive to neurokinin-3 receptor antagonism.

12. The packaged pharmaceutical composition of Claim 12, wherein said patient is suffering from anxiety, depression, schizophrenia, obesity or chronic pulmonary obstructive disorder.

13. A compound according to anyone of Claims 1 to 3 wherein in an assay of neurokinin-3 receptor binding performed as set out in Example IX of the description the compound exhibits an IC₅₀ of 1 micromolar or less.

## Patentansprüche

1. Verbindung der Formel: 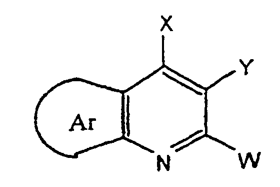 oder pharmazeutisch annehmbare, nicht toxische Salze oder pharmazeutisch annehmbare Solvate derselben, worin:
Y Wasserstoff, geradkettiges oder verzweigtes Niederalkyl mit 1-6 Kohlenstoffatomen, Halogen, Amino, Hydroxyl oder Niederalkoxy mit 1-6 Kohlenstoffatomen ist oder
Y geradkettiges oder verzweigtes Niederalkyl mit 1-6 Kohlenstoffatomen oder Niederalkoxy mit 1-6 Kohlenstoffatomen ist, von denen jedes auf der Alkylkette mit einer Amino- oder Mono- oder Dialkylaminogruppe substituiert ist, worin jedes Alkyl unabhängig ein Niederalkyl darstellt;
W Phenyl, Thienyl oder Pyridyl ist, von denen jedes mono- oder disubstituiert sein kann mit Halogen, Cyano, Trifluoromethyl, Trifluoromethoxy, Hydroxy, geradkettigem oder verzweigtem Niederalkyl mit 1-6 Kohlenstoffatomen, geradkettigem oder verzweigtem Niederalkoxy mit 1-6 Kohlenstoffatomen, Amino oder Mono- oder Dialkylamino, worin jedes Alkyl ein geradkettiges oder verzweigtes Niederalkyl mit 1-6 Kohlenstoffatomen bedeutet; oder
W Phenyl(C₁-C₆)alkyl, Thienyl(C₁-C₆)alkyl oder Pyridyl(C₁-C₆)alkyl ist, worin der aromatische Teil derselben wahlweise mono- oder disubstituiert ist mit Halogen, Cyano, Trifluoromethyl, Trifluoromethoxy, Hydroxy, geradkettigem oder verzweigtem Niederalkyl mit 1-6 Kohlenstoffatomen, geradkettigem oder verzweigtem Niederalkoxy mit 1-6 Kohlenstoffatomen, Amino oder Mono- oder Dialkylamino, worin jedes Alkyl geradkettig oder verzweigtes Niederalkyl mit 1-6 Kohlenstoffatomen bedeutet; oder
W Piperidinyl, Morpholinyl, Thiomorpholinyl, Pyrrolindinyl, Piperazinyl, Homopiperazinyl, Azabicyclo[3.2.2]nonanyl, Isoindolinyl ist, worin jedes von diesen unsubstituiert oder mono- oder disubstituiert mit Alkyl- oder Arylgruppen oder an einen aromatischen Ring kondensiert ist; oder
W Stickstoff darstellt, der mono- oder disubstituiert ist mit Wasserstoff, geradkettigem oder verzweigtem Alkyl, bestehend aus 1-8 Kohlenstoffatomen, Cycloalkyl, enthaltend 3-8 Kohlenstoffatome, Alkylketten mit 1-6 Kohlenstoffatomen, die in jeder Position an einen Arylring angebunden sind, Alkylketten mit 1-6 Kohlenstoffatomen, die in jeder Position an ein Cycloalkyl, enthaltend 3-8 Kohlenstoffatome, Phenyl, Thienyl, Pyridyl, Imidazolyl, Benzimidazolyl oder einen Heterozyklus mit aromatischem Charakter angebunden sind; oder
W Sauerstoff darstellt, der substituiert ist mit geradkettigem oder verzweigtem Alkyl mit 1-8 Kohlenstoffatomen, Cycloalkyl mit 3-8 Kohlenstoffatomen oder Phenyl, mono- oder disubstituiert mit Halogen, Cyano, Trifluoromethyl, Trifluoromethoxy, Hydroxy, geradkettigem oder verzweigtem Niederalkyl mit 1-6 Kohlenstoffatomen, Amino, Mono- oder Dialkylamino, worin jedes Alkyl geradkettiges oder verzweigtes Niederalkyl mit 1-6 Kohlenstoffatomen darstellt, oder geradkettigem oder verzweigtem Niederalkoxy mit 1-6 Kohlenstoffatomen; 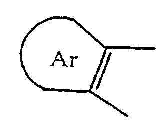 darstellt: 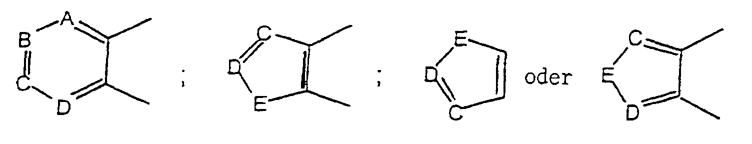
worin
A Stickstoff oder C-R₁ darstellt;
B Stickstoff oder C-R₂ darstellt;
C Stickstoff oder C-R₃ darstellt;
D Stickstoff oder C-R₄ darstellt, mit der Maßgabe, dass dann, wenn alle vier von A, B, C und D vorhanden sind, mindestens eines, jedoch nicht mehr als zwei von A, B, C und D Stickstoff darstellen; und
E Sauerstoff, Schwefel oder N-R₅ darstellt;
R₁ bis R₄ gleich oder verschieden sind und darstellen Wasserstoff, Halogen, Cyano, Trifluoromethyl, Trifluoromethoxy, Hydroxy, geradkettiges oder verzweigtes Niederalkyl mit 1-6 Kohlenstoffatomen, Amino, Mono- oder Dialkylamino, worin jedes Alkyl geradkettiges oder verzweigtes Niederalkyl mit 1-6 Kohlenstoffatomen darstellt, oder geradkettiges oder verzweigtes Niederalkoxy mit 1-6 Kohlenstoffatomen;
R₅ Wasserstoff oder geradkettiges oder verzweigtes Niederalkyl mit 1-6 Kohlenstoffatomen ist oder Phenyl, Thienyl oder Pyridyl darstellt, von denen jedes mono- oder disubstituiert sein kann mit Halogen, Cyano, Trifluoromethyl, Trifluoromethoxy, Hydroxy, geradkettigem oder verzweigtem Niederalkyl mit 1-6 Kohlenstoffatomen, Amino, Mono- oder Dialkylamino, worin jedes Alkyl geradkettiges oder verzweigtes Niederalkyl mit 1-6 Kohlenstoffatomen darstellt, oder geradkettigem oder verzweigtem Niederalkoxy mit 1-6 Kohlenstoffatomen;
X darstellt 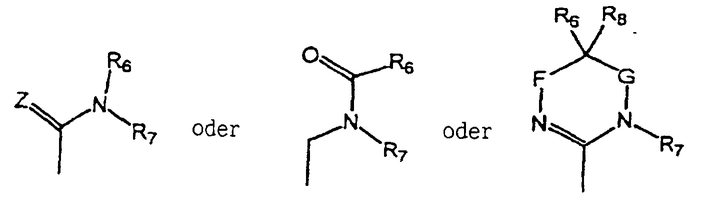
worin F Carbonyl, Methylen oder Ethylen darstellt;
G Methylen oder eine Bindung ist, mit der Maßgabe, dass G dann eine Bindung darstellt, wenn F Ethylen ist; und
Z Sauerstoff, Schwefel, N-C≡N oder zwei Wasserstoffatome darstellt;
R₆ Phenyl, Thienyl oder Pyridyl ist, von denen jedes mono- oder disubstituiert sein kann mit Halogen, Cyano, Trifluoromethyl, Trifluoromethoxy, Hydroxy, geradkettigem oder verzweigtem Niederalkyl mit 1-6 Kohlenstoffatomen, Amino, Mono- oder Dialkylamino, worin jedes Alkyl geradkettiges oder verzweigtes Niederalkyl mit 1-6 Kohlenstoffatomen ist, oder geradkettigem oder verzweigtem Niederalkoxy mit 1-6 Kohlenstoffatomen; oder
R₆ geradkettiges oder verzweigtes Alkyl mit 1-8 Kohlenstoffatomen oder Cycloalkyl, enthaltend 3-8 Kohlenstoffatome, darstellt, von denen jedes wahlweise mono- oder disubstituiert ist mit einem aromatischen Ring, worin jeder aromatische Ring wahlweise mono- oder disubstituiert ist, und zwar unabhängig, mit Halogen, Cyano, Trifluoromethyl, Trifluoromethoxy, Hydroxy, geradkettigem oder verzweigtem Niederalkyl mit 1-6 Kohlenstoffatomen, Amino, mono- oder disubstituiertem Amino, worin der Substituent auf dem Stickstoff entweder geradkettiges oder verzweigtes Alkyl mit 1-6 Kohlenstoffatomen darstellt, oder geradkettigem oder verzweigtem Alkoxy mit 1-6 Kohlenstoffatomen; oder
R₆ Cycloalkyl darstellt, enthaltend 5-8 Kohlenstoffatome, worin das Cycloalkyl an einen aromatischen Ring in benachbarter Position kondensiert ist;
R₇ und R₈ gleich oder verschieden sind und darstellen Wasserstoff oder geradkettiges oder verzweigtes Niederalkyl mit 1-6 Kohlenstoffatomen; oder R₆ und R₇ zusammengenommen einen 5, 6, 7 oder 8-gliedrigen Ring bilden, der monooder disubstituiert ist mit einer Arylgruppe oder einen fusionierten Arylring als Teil des 5, 6, 7 oder 8-gliedrigen Ringes, wie oben beschrieben, enthält, mit der Maßgabe, dass R₆ und R₇ zusammengenommen dann keinen Ring bilden, wenn G ein Methylen ist; oder
R₆ und R₈ zusammengenommen einen 5, 6, 7 oder 8-gliedrigen Ring bilden können, der mono- oder disubstituiert ist mit einer Arylgruppe oder einen kondensierten Arylring als Teil des 5, 6, 7 oder 8-gliedrigen Ringes enthält.

2. Verbindung gemäß Anspruch 1, bei der es sich handelt um
N-[(S)-1-Phenyl-1-propyl)(2-phenylpyridino[4,3-b]pyridin-4-yl)carboxamid;
N-[(R)-1-Phenyl-1-propyl)(2-phenylpyridino[4,3-b]pyridin-4-yl)carboxamid;
N-((S)-1-Phenyl-1-propyl)(2-phenylpyridino[2,3-b]pyridin-4-yl)carboxamid;
N-((R)-1-Phenyl-1-propyl)(2-phenylpyridino[2,3-b]pyridin-4-yl)carboxamid;
N-(Diphenylmethyl)-N-methyl-(2-phenylpyridino[2,3-b]pyridin-4-yl)carboxamid;
N-(Diphenylmethyl]-N-methyl-(2-phenylpyridino[4,3-b]pyridin-4-yl)carboxamid;
N-((S)-1-Phenyl-1-propyl)(2-phenylthieno[3,2-b]pyridin-4-yl)carboxamid;
N-[1-(Phenyl)-ethyl]-N-methyl-(2-phenylpyridino[2,3-b]pyridin-4-yl)carboxamid;
N- [1-(Phenyl)-butyl]-N-methyl-(2-phenylpyridino[2,3-b]pyridin-4-yl)carboxamid;
N-[1-(Phenyl)-pentyl]-N-methyl-(2-phenylpyridino[2,3-b]pyridin-4-yl)carboxamid;
N-[1-(Phenyl)-hexyl]-N-methyl-(2-phenylpyridino[2,3-b]pyridin-4-yl)carboxamid;
N-[1-(Diphenyl)-methyl]-N-methyl-(2-phenylpyridino[2,3-b]pyridin-4-yl) carboxamid;
N-[1-(1,2-Diphenyl)-ethyl]-N-methyl-(2-phenylpyridino[2,3-b]pyridin-4-yl)carboxamid;
N-[1-(1-Phenyl-2-cyclohexyl)-ethyl]-N-methyl-(2-phenylpyridino[2,3-b]pyridin-4-yl)carboxamid;
N-Indanyl-N-methyl-(2-phenylpyridino[2,3-b]pyridin-4-yl)carboxamid;
N-[1-(Phenyl)-isobutyl]-N-methyl-(2-phenylpyridino[2,3-b]pyridin-4-yl) carboxamid;
N-[1-(Phenyl)-2-methylbutyl]-N-methyl-(2-phenylpyridino[2,3-b]pyridin-4-yl) carboxamid;
1-Isobutyl-(2-1,2,3,4-tetrahydroisoquinolyl)(2-phenylpyridino[2,3-b]pyridin-4-yl) keton;
1-(2-Methylphenyl)(2-1,2,3,4-tetrahydroisoquinolyl)(2-phenylpyridino[2,3-b] pyridin-4-yl)keton;
1-(2-Chlorophenyl)(2-1,2,3,4-tetrahydroisoquinolyl)(2-phenylpyridino[2, 3-b] pyridin-4-yl)keton;
1-Methyl-(2-1,2,3,4-tetrahydroisoquinolyl)(2-phenylpyridino[2,3-b]pyridin-4-yl)keton;
1-Propyl-(2-1,2,3,4-tetrahydroisoquinolyl)(2-phenylpyridino[2,3-b]pyridin-4-yl) keton;
1-Butyl-(2-1,2,3,4-tetrahydroisoquinolyl)(2-phenylpyridino[2,3-b]pyridin-4-yl) keton;
1-Cyclopentyl-(2-1,2,3,4-tetrahydroisoquinolyl)(2-phenylpyridino[2,3-b]pyridin-4-yl)keton;
1-Pentyl-(2-1,2,3,4-tetrahydroisoquinolyl)(2-phenylpyridino[2, 3-b]pyridin-4-yl) keton;
1-Benzyl-(2-1,2,3,4-tetrahydroisoquinolyl)(2-phenylpyridino[2, 3-b]pyridin-4-yl) keton;
1-Cyclohexylmethyl-(2-1,2,3,4-tetrahydroisoquinolyl)(2-phenylpyridino[2, 3-b] pyridin-4-yl)keton;
1-Isopropyl-(2-1,2,3,4-tetrahydroisoquinolyl)(2-phenylpyridino[2,3-b]pyridin-4-yl) keton;
N-[1-(Phenyl)octyl]-N-methyl-(2-phenylpyridino[2,3-b]pyridin-4-yl)carboxamid; 1-(2-Methylbutyl)(2-1,2,3,4-tetrahydroisoquinolyl)(2-phenylpyridino[2,3-b] pyridin-4-yl)keton;
N-[1,3-(Diphenyl)propyl]-N-methyl-(2-phenylpyridino[2,3-b]pyridin-4-yl)carboxamid;
N-[1,4-(Diphenyl)butyl]-N-methyl-(2-phenylpyridino[2,3-b]pyridin-4-yl) carboxamid;
N-Benzyl-N-methyl-(2-phenylpyridino[2,3-b]pyridin-4-yl)carboxamid;
N-Benzyl-N-ethyl-(2-phenylpyridino[2,3-b]pyridin-4-yl)carboxamid;
N-Benzyl-N-isopropyl-(2-phenylpyridino[2,3-b]pyridin-4-yl)carboxamid;
N-Benzyl-N-butyl-(2-phenylpyridino[2,3-b]pyridin-4-yl)carboxamid;
N-Benzyl-N-isobutyl-(2-phenylpyridino[2,3-b]pyridin-4-yl)carboxarnid;
N-Benzyl-N-cyclopentyl-(2-phenylpyridino[2,3-b]pyridin-4-yl)carboxamid;
N-Benzyl-N-pentyl-(2-phenylpyridino[2,3-b]pyridin-4-yl)carboxamid;
N-Benzyl-N-cyclohexyl-(2-phenylpyridino[2,3-b]pyridin-4-yl)carboxamid;
N, N-Dibenzyl-(2-phenylpyridino[2,3-b]pyridin-4-yl)carboxamid;
N-Benzyl-N-1-methylpropyl-(2-phenylpyridino[2,3-b]pyridin-4-yl)carboxamid;
N-Benzyl-N-1-methylbutyl-(2-phenylpyridino[2,3-b]pyridin-4-yl)carboxamid;
N-Benzyl-N-4-methylbutyl-(2-phenylpyridino[2,3-b]pyridin-4-yl)carboxamid;
N-Benzyl-N-cyclopentylmethyl-(2-phenylpyridino[2,3-b]pyridin-4-yl)carboxamid;
N-Benzyl-N-hexyl-(2-phenylpyridino [2,3-b]pyridin-4-yl) carboxamid;
N-Benzyl-N-cyclohexylmethyl (2-phenylpyridino[2,3-b]pyridin-4-yl) carboxamid;
N-Benzyl-N-heptyl-(2-phenylpyridino[2,3-b]pyridin-4-yl)carboxamid;
N-Methyl-(2-phenylpyridino[2,3-b]pyridin-4-yl)carboxamid;
N-[1-(Phenylcyclopentyl)methyl]-N-methyl-(2-phenylpyridino[2,3-b]pyridin-4-yl)carboxamid;
N-[1-(Phenylcyclohexyl)methyl]-N-methyl-(2-phenylpyridino[2,3-b]pyridin-4-yl)carboxamid;
N-[1-(1-Phenyl-2-cyclopentyl)ethyl]-N-methyl-(2-phenylpyridino[2,3-b]pyridin-4-yl)carboxamid;
N-[1-(1,3-Diphenyl)propyl]-N-methyl-(2-phenylpyridino[2,3-b]pyridin-4-yl)carboxamid;
N-[ 1-(1,4-Diphenyl)butyl]-N-methyl-(2-phenylpyridino[2,3-b]pyridin-4-yl)carboxamid;
1-Phenyl-(2-1,2,3,4-tetrahydroisoquinolyl)(2-phenylpyridino[2,3-b]pyridin-4-yl)keton;
1-Cyclohexyl-(2-1,2,3,4-tetrahydroisoquinolyl)(2-phenylpyridino[2,3-b]pyridin-4-yl)keton;
1-Cyclopentylmethyl-(2-1,2,3,4-tetrahydroisoquinolyl)(2-phenylpyridino[2,3-b] pyridin-4-yl)keton;
1-Hexyl-(2-1,2,3,4-tetrahydroisoquinolyl)(2-phenylpyridino[2,3-b]pyridin-4-yl) keton;
1-(2-Fluorophenyl)(2-1,2,3,4-tetrahydroisoquinolyl)(2-phenylpyridino[2,3-b] pyridin-4-yl)keton;
1-(2-Trifluoromethylphenyl)(2-1,2,3,4-tetrahydroisoquinolyl)(2-phenylpyridino [2,3-b]pyridin-4-yl)keton;
1-(3-Methylphenyl)(2-1,2,3,4-tetrahydroisoquinolyl)(2-phenylpyridino[2,3-b] pyridin-4-yl)keton;
1-(4-Methylphenyl)(2-1,2,3,4-tetrahydroisoquinolyl)(2-phenylpyridino[2,3-b] pyridin-4-yl)keton;
1-(4-Fluorophenyl)(2-1,2,3,4-tetrahydroisoquinolyl)(2-phenylpyridino[2,3-b] pyridin-4-yl)keton;
1-Cycloheptyl-(2-1,2,3,4-tetrahydroisoquinolyl)(2-phenylpyridino[2,3-b]pyridin-4-yl)keton;
1-(3-Chlorophenyl)(2-1,2,3,4-tetrahydroisoquinolyl)(2-phenylpyridino[2,3-b] pyridin-4-yl)keton;
1-(4-Chlorophenyl)(2-1,2,3,4-tetrahydroisoquinolyl)(2-phenylpyridino[2,3-b] pyridin-4-yl)keton;
6,7-Dimethoxy-(2-1,2,3,4-tetrahydroisoquinolyl)(2-phenylpyridino[2,3-b]pyridin-4-yl)keton;
6,7-Dimethoxy-1-methyl-(2-1,2,3,4-tetrahydroisoquinolyl)(2-phenylpyridino[2,3-b] pyridin-4-yl)keton;
6,7-Dimethoxy-1-phenyl-(2-1,2,3,4-tetrahydroisoquinolyl)(2-phenylpyridino[2,3-b] pyridin-4-yl)keton;
6,7-Dimethoxy-3-methyl-(2-1,2,3,4-tetrahydroisoquinolyl)(2-phenylpyridino[2,3-b] pyridin-4-yl)keton;
2-[6,7-Dimethoxy-2-(2-1,2,3,4-tetrahydroisoquinolyl)(2-phenylpyridino[2,3-b] pyridin-4-yl)]-ethannitrilketon;
N-[1-(4-Bromophenyl)ethyl](2-phenylpyridino[2,3-b]pyridin-4-yl)carboxamid;
N-[1-(4-Methylphenyl)ethyl](2-phenylpyridino[2,3-b]pyridin-4-yl)carboxamid;
N-[1-(3-Methylphenyl)ethyl](2-phenylpyridino[2,3-b]pyridin-4-yl)carboxamid;
N-[1-(4-Fluorophenyl)ethyl](2-phenylpyridino[2,3-b]pyridin-4-yl)carboxamid;
N-[1-(3-Fluorophenyl)ethyl](2-phenylpyri dino[2,3-b]pyridin-4-yl)carboxamid;
N-[1-(4-Methoxyphenyl)ethyl](2-phenylpyridino[2,3-b]pyridin-4-yl)carboxamid;
N-[1-(3-Methoxyphenyl)ethyl](2-phenylpyridino[2,3-b]pyridin-4-yl)carboxamid;
N- [1-(4-Chlorophenyl)ethyl](2-phenylpyridino[2,3-b]pyridin-4-yl)carboxamid;
N-[1-(3-Chlorophenyl)ethyl] (2-phenylpyridino[2,3-b]pyridin-4-yl)carboxamid;
N-[1-(4-Trifluoromethylphenyl)ethyl](2-phenylpyridino[2,3-b]pyridin-4-yl)carboxamid;
N-[1-(3-Trifluoromethylphenyl)ethyl](2-phenylpyridino[2,3-b]pyridin-4-yl)carboxamid;
N-[ 1-(4-Methylphenyl)methyl]-N-methyl(2-phenylpyridino[2,3-b]pyridin-4-yl)carboxamid;
N-[1-(3-Methylphenyl)methyl]-N-methyl(2-phenylpyridino[2,3-b]pyridin-4-yl)carboxamid;
N-[1-(4-Fluorophenyl)methyl]-N-methyl(2-phenylpyridino[2,3-b]pyridin-4-yl)carboxamid;
N-[1-(3-Fluorophenyl)methyl]-N-methyl(2-phenylpyridino[2,3-b]pyridin-4-yl)carboxamid;
N-[1-(4-Methoxyphenyl)methyl]-N-methyl(2-phenylpyridino[2,3-b]pyridin-4-yl)carboxamid;
N-[1-(3-Methoxyphenyl)methyl]-N-methyl(2-phenylpyridino[2,3-b]pyridin-4-yl)carboxamid;
N-[1-(4-Chlorophenyl)methyl]-N-methyl(2-phenylpyridino[2,3-b]pyridin-4-yl) carboxamid;
N-[1-(3-Chlorophenyl)methyl]-N-methyl(2-phenylpyridino[2,3-b]pyridin-4-yl) carboxamid;
N-[1-(4-Trifluoromethylphenyl)methyl]-N-methyl(2-phenylpyridino[2,3-b]pyridin-4-yl)carboxamid;
N-[1-(3-Trifluoromethylphenyl)methyl]-N-methyl(2-phenylpyridino[2,3-b]pyridin-4-yl)carboxamid;
N-[1-(4-Bromophenyl)methyl]-N-methyl(2-phenylpyridino[2,3-b]pyridin-4-yl)carboxamid;
N-[1-(2-Methylphenyl)methyl](2-phenylpyridino[2,3-b]pyridin-4-yl)carboxamid;
N-[1-(2-Fluorophenyl)methyl](2-phenylpyridino[2,3-b]pyridin-4-yl)carboxamid;
N-[1-(2-Methoxyphenyl)methyl](2-phenylpyridino[2,3-b]pyridin-4-yl)carboxamid;
N-[1-(2-Chlorophenyl)methyl](2-phenylpyridino[2,3-b]pyridin-4-yl)carboxamid;
N-[1-(2-Trifluoromethylphenyl)methyl](2-phenylpyridino[2,3-b]pyridin-4-yl)carboxamid;
N-[1-(2-Methylphenyl)ethyl](2-phenylpyridino[2,3-b]pyridin-4-yl)carboxamid;
N-[1-(2-Fluorophenyl)ethyl](2-phenylpyridino[2,3-b]pyridin-4-yl)carboxamid;
N-[1-(2-Methoxyphenyl)ethyl](2-phenylpyridino[2,3-b]pyridin-4-yl)carboxamid;
N-[1-(2-Chlorophenyl)ethyl](2-phenylpyridino[2,3-b]pyridin-4-yl)carboxamid;
N-[1-(2-Trifluoromethylphenyl)ethyl](2-phenylpyridino[2,3-b]pyridin-4-yl)carboxamid;
N-[1-(2,4-Dichlorophenyl)ethyl](2-phenylpyridino[2,3-b]pyridin-4-yl)carboxamid;
N-[1-(4-Methylphenyl)methyl](2-phenylpyridino[2,3-b]pyridin-4-yl)carboxamid;
N-[1-(3-Methylphenyl)methyl](2-phenylpyridino[2,3-b]pyridin-4-yl)carboxamid;
N-[1-(4-Fluorophenyl)methyl](2-phenylpyridino[2,3-b]pyridin-4-yl)carboxamid;
N-[1-(3-Fluorophenyl)methyl](2-phenylpyridino[2,3-b]pyridin-4-yl)carboxamid;
N-[1-(4-Methoxyphenyl)methyl](2-phenylpyridino[2,3-b]pyridin-4-yl)carboxamid;
N-[1-(3-Methoxyphenyl)methyl](2-phenylpyridino[2,3-b]pyridin-4-yl)carboxamid;
N-[1-(4-Chlorophenyl)methyl](2-phenylpyridino[2,3-b]pyridin-4-yl)carboxamid;
N-[1-(3-Chlorophenyl)methyl](2-phenylpyridino[2,3-b]pyridin-4-yl)carboxamid;
N-[1-(4-Trifluoromethylphenyl)methyl](2-phenylpyridino[2,3-b]pyridin-4-yl)carboxamid;
N-[1-(3-Trifluoromethylphenyl)methyl](2-phenylpyridino[2,3-b]pyridin-4-yl) carboxamid;
N-[1-(4-Bromophenyl)methyl](2-phenylpyridino[2,3-b]pyridin-4-yl)carboxamid;
N-[1-(Phenyl)ethyl]-N-methyl(2-phenylpyridino[4,3-b]pyridin-4-yl)carboxamid;
N-[1-(Phenyl)butyl]-N-methyl(2-phenylpyridino[4,3-b]pyridin-4-yl)carboxamid;
N-[1-(Phenyl)pentyl]-N-methyl(2-phenylpyridino[4,3-b]pyridin-4-yl)carboxamid;
N-[1-(Phenyl)hexyl]-N-methyl(2-phenylpyridino[4,3-b]pyridin-4-yl)carboxamid;
N-[1-(Diphenyl)methyl]-N-methyl(2-phenylpyridino[4,3-b]pyridin-4-yl) carboxamid;
N-[1-(1,2-Diphenyl)ethyl]-N-methyl(2-phenylpyridino[4,3-b]pyridin-4-yl) carboxamid;
N- [1- (1-Phenyl-2-cyclohexyl) ethyl]-N-methyl (2-phenylpyridino [4,3-b] pyridin-4-yl)carboxamid;
N-Indanyl-N-methyl-(2-phenylpyridino[4,3-b]pyridin-4-yl)carboxamid;
N-[1-(Phenyl)isobutyl]-N-methyl-(2-phenylpyridino[4,3-b]pyridin-4-yl) carboxamid;
N-[1-(Phenyl)-2-methylbutyl]-N-methyl-(2-phenylpyridino[4,3-b]pyridin-4-yl) carboxamid;
1-Isobutyl-(2-1,2,3,4-tetrahydroisoquinolyl)(2-phenylpyridino[4,3-b]pyridin-4-yl) keton;
1-(2-Methylphenyl)(2-1,2,3,4-tetrahydroisoquinolyl)(2-phenylpyridino[4,3-b] pyridin-4-yl) keton;
1-(2-Chlorophenyl)(2-1,2,3,4-tetrahydroisoquinolyl)(2-phenylpyridino[4,3-b] pyridin-4-yl)keton;
1-Methyl-(2-1, 2,3,4-tetrahydroisoquinolyl)(2-phenylpyridino[4,3-b]pyridin-4-yl) keton;
1-Propyl-(2-1,2,3,4-tetrahydroisoquinolyl)(2-phenylpyridino[4,3-b]pyridin-4-yl) keton;
1-Butyl-(2-1,2,3,4-tetrahydroisoquinolyl)(2-phenylpyridino[4,3-b]pyridin-4-yl) keton;
1-Cyclopentyl-(2-1,2,3,4-tetrahydroisoquinolyl)(2-phenylpyridino[4,3-b]pyridin-4-yl)keton;
1-Pentyl-(2-1,2,3,4-tetrahydroisoquinolyl)(2-phenylpyridino[4,3-b]pyridin-4-yl) keton;
1-Benzyl-(2-1,2,3,4-tetrahydroisoquinolyl)(2-phenylpyridino[4,3-b]pyri din-4-yl) keton;
1-Cyclohexylmethyl-(2-1,2,3,4-tetrahydroisoquinolyl)(2-phenylpyridino[4,3-b] pyridin-4-yl)keton;
1-Isopropyl-(2-1, 2,3,4-tetrahydroisoquinolyl)(2-phenylpyridino[4,3-b]pyridin-4-yl) keton;
N-[1-(Phenyl)octyl]-N-methyl-(2-phenylpyridino[4,3-b]pyridin-4-yl)carboxamid; 1-(2-Methylbutyl)(2-1,2,3,4-tetrahydroisoquinolyl)(2-phenylpyridino[4,3-b] pyridin-4-yl)keton;
N-[1,3-(Diphenyl)propyl]-N-methyl-(2-phenylpyridino[4,3-b]pyridin-4-yl) carboxamid;
N-[1,4-(Diphenyl)butyl]-N-methyl-(2-phenylpyridino[4,3-b]pyridin-4-yl) carboxamid;
N-Benzyl-N-methyl-(2-phenylpyridino[4,3-b]pyridin-4-yl)carboxamid;
N-Benzyl-N-ethyl-(2-phenylpyridino[4,3-b]pyridin-4-yl)carboxamid;
N-Benzyl-N-isopropyl-(2-phenylpyridino[4,3-b]pyridin-4-yl)carboxamid;
N-Benzyl-N-butyl-(2-phenylpyridino[4,3-b]pyridin-4-yl)carboxamid;
N-Benzyl-N-isobutyl-(2-phenylpyridino[4,3-b]pyridin-4-yl)carboxamid;
N-Benzyl-N-cyclopentyl-(2-phenylpyridino[4,3-b]pyridin-4-yl)carboxamid;
N-Benzyl-N-pentyl-(2-phenylpyridino[4,3-b]pyridin-4-yl)carboxamid;
N-Methyl-(2-phenylpyridino[4,3-b]pyridin-4-yl)carboxamid;
N-[1-(Phenylcyclopentyl)methyl]-N-methyl-(2-phenylpyridino[4,3-b]pyridin-4-yl) carboxamid;
N-[1-(Phenylcyclohexyl)methyl]-N-methyl-(2-phenylpyridino[4,3-b]pyridin-4-yl)carboxamid;
N-[1-(1-Phenyl-2-cyclopentyl)ethyl]-N-methyl-(2-phenylpyridino[4,3-b]pyridin-4-yl)carboxamid;
N-[1-(1,3-Diphenyl)propyl]-N-methyl-(2-phenylpyridino[4,3-b]pyridin-4-yl) carboxamid;
N-[1-(1,4-Diphenyl)butyl]-N-methyl-(2-phenylpyridino[4,3-b]pyridin-4-yl) carboxamid;
1-Phenyl-(2-1,2,3,4-tetrahydroisoquinolyl)(2-phenylpyridino[4,3-b]pyridin-4-yl) keton;
1-Cyclohexyl-(2-1,2,3,4-tetrahydroisoquinolyl)(2-phenylpyridino[4,3-b]pyridin-4-yl)keton;
1-Cyclopentylmethyl-(2-1,2,3,4-tetrahydroisoquinoly])(2-phenylpyridino[4,3-b] pyridin-4-yl)keton;
1-Hexyl-(2-1,2,3,4-tetrahydroisoquinolyl)(2-phenylpyridino[4,3-b]pyridin-4-yl) keton;
1-(2-Fluorophenyl)(2-1,2,3,4-tetrahydroisoquinolyl)(2-phenylpyridino[4,3-b] pyridin-4-yl)keton;
1-(3-Methylphenyl)(2-1,2,3,4-tetrahydroisoquinolyl)(2-phenylpyridino[4,3-b] pyridin-4-yl)keton;
1-(4-Methylphenyl)(2-1,2,3,4-tetrahydroisoquinolyl)(2-phenylpyridino[4,3-b] pyridin-4-yl)keton;
1-(4-Fluorophenyl)(2-1,2,3,4-tetrahydroisoquinolyl)(2-phenylpyridino[4,3-b] pyridin-4-yl)keton;
1-Cycloheptyl-(2-1, 2,3,4-tetrahydroisoquinolyl)(2-phenylpyridino[4,3-b]pyridin-4-yl)keton;
1-(3-Chlorophenyl)(2-1, 2,3,4-tetrahydroisoquinolyl)(2-phenylpyridino[4,3-b] pyridin-4-yl)keton;
1-(4-Chlorophenyl)(2-1,2,3,4-tetrahydroisoquinolyl)(2-phenylpyridino[4,3-b] pyridin-4-yl)keton;
6,7-Dimethoxy-(2-1,2,3,4-tetrahydroisoquinolyl)(2-phenylpyridino[4,3-b]pyridin-4-yl)keton;
6,7-Dimethoxy-1-methyl-(2-1,2,3,4-tetrahydroisoquinolyl)(2-phenylpyridino[4,3-b] pyridin-4-yl)keton;
6,7-Dimethoxy-1-phenyl-(2-1,2,3,4-tetrahydroisoquinolyl)(2-phenylpyridino[4,3-b] pyridin-4-yl)keton;
6,7-Dimethoxy-3-methyl-(2-1,2,3,4-tetrahydroisoquinolyl)(2-phenylpyridino[4,3-b] pyridin-4-yl)keton;
2-[6,7-Dimethoxy-2-(2-1,2,3,4-tetrahydroisoquinolyl)(2-phenylpyridino[4,3-b] pyridin-4-yl)]-ethannitril-keton;
N-[1-(4-Bromophenyl)ethyl](2-phenylpyridino[4,3-b]pyridin-4-yl)carboxamid;
N-[1-(4-Methylphenyl)ethyl](2-phenylpyridino[4,3-b]pyridin-4-yl)carboxamid;
N-[1-(3-Methylphenyl)ethyl](2-phenylpyridino[4,3-b]pyridin-4-yl)carboxamid;
N-[1-(4-Fluorophenyl)ethyl](2-phenylpyridino[4,3-b]pyridin-4-yl)carboxamid;
N-[1-(3-Fluorophenyl)ethyl](2-phenylpyridino[4,3-b]pyridin-4-yl)carboxamid;
N-[1-(4-Methoxyphenyl)ethyl](2-phenylpyridino[4,3-b]pyridin-4-yl)carboxamid;
N-[1-(3-Methoxylphenyl)ethyl](2-phenylpyridino[4,3-b]pyridin-4-yl)carboxamid;
N-[1-(4-Chlorophenyl)ethyl](2-phenylpyridino[4,3-b]pyridin-4-yl)carboxamid;
N-[1-(3-Chlorophenyl)ethyl](2-phenylpyridino[4,3-b]pyridin-4-yl)carboxamid;
N-[1-(4-Trifluoromethylphenyl)ethyl](2-phenylpyridino[4,3-b]pyridin-4-yl) carboxamid;
N-[1-(3-Trifluoromethylphenyl)ethyl](2-phenylpyridino[4,3-b]pyridin-4-ylcarboxamid;
N-[1-(4-Methylphenyl)methyl]-N-methyl-[2-phenylpyridino [4,3-b]pyridin-4-yl)carboxamid;
N-[1-(3-Methylphenyl)methyl]-N-methyl-(2-phenylpyridino[4,3-b]pyridin-4-yl) carboxamid;
N-[1-(4-Fluorophenyl)methyl]-N-methyl-(2-phenylpyridino[4,3-b]pyridin-4-yl) carboxamid;
N-[1-(3-Fluorophenyl)methyl]-N-methyl-(2-phenylpyridino[4,3-b]pyridin-4-yl) carboxamid;
N-[1-(4-Methoxyphenyl)methyl]-N-methyl-(2-phenylpyridino[4,3-b]pyridin-4-yl) carboxamid;
N-[1-(3-Methoxyphenyl)methyl]-N-methyl-(2-phenylpyridino[4,3-b]pyridin-4-yl) carboxamid;
N-[1-(4-Chlorophenyl)methyl]-N-methyl-(2-phenylpyridino[4,3-b]pyridin-4-yl) carboxamid;
N-[1-(3-Chlorophenyl)methyl]-N-methyl-(2-pheny]pyridino[4,3-b]pyridin-4-yl) carboxamid;
N-[1-(4-Trifluoromethylphenyl)methyl]-N-methyl-(2-phenylpyridino[4, 3-b] pyridin-4-yl)carboxamid;
N-[1-(3-Trifluoromethylphenyl)methyl]-N-methyl-(2-phenylpyridino[4,3-b] pyridin-4-yl)carboxamid;
N-[1-(4-Bromophenyl)methyl]-N-methyl-(2-phenylpyridino[4,3-b]pyridin-4-yl) carboxamid;
N-[1-(2-Methylphenyl)methyl](2-phenylpyridino [4,3-b]pyridin-4-yl)carboxamid;
N-[1-(2-Fluorophenyl)methyl](2-phenylpyridino[4,3-b]pyridin-4-yl)carboxamid;
N-[1-(2-Methoxyphenyl)methyl](2-phenylpyridino[4,3-b]pyridin-4-yl)carboxamid;
N-[1-(2-Chlorophenyl)methyl](2-phenylpyridino[4,3-b]pyridin-4-yl)carboxamid;
N-[1-(2-Trifluoromethylphenyl)methyl](2-phenylpyridino[4,3-b]pyridin-4-yl) carboxamid;
N-[1-(2-Methylphenyl)ethyl](2-phenylpyridino[4,3-b]pyridin-4-yl)carboxamid;
N-[1-(2-Fluorophenyl)ethyl](2-phenylpyridino[4,3-b] pyridin-4-yl)carboxamid;
N-[1-(2-Methoxyphenyl)ethyl](2-phenylpyridino[4,3-b]pyridin-4-yl)carboxamid;
N-[1-(2-Chlorophenyl)ethyl](2-phenylpyridino[4,3-b]pyridin-4-yl)carboxamid;
N-[1-(2-Trifluoromethylphenyl)ethyl](2-phenylpyridino[4,3-b]pyridin-4-yl) carboxamid;
N-[1-(2,4-Dichlorophenyl)ethyl](2-phenylpyridino[4,3-b]pyridin-4-yl)carboxamid;
N-[1-(4-Methylphenyl)methyl](2-phenylpyridino[4,3-b]pyridin-4-yl)carboxamid;
N-[1-(3-Methylphenyl)methyl](2-phenylpyridino[4,3-b]pyridin-4-yl)carboxamid;
N-[1-(4-Fluorophenyl)methyl](2-phenylpyridino[4,3-b]pyridin-4-yl)carboxamid;
N-[1-(3-Fluorophenyl)methyl] (2-phenylpyridino [4,3-b]pyridin-4-yl)carboxamid;
N-[1-(4-Methoxyphenyl)methyl](2-phenylpyridino[4,3-b]pyridin-4-yl)carboxamid;
N-[1-(3-Methoxyphenyl)methyl](2-phenylpyridin [4,3-b]pyridin-4-yl)carboxamid;
N-[1-(4-Chlorophenyl)methyl](2-phenylpyridino[4,3-b]pyridin-4-yl)carboxamid;
N-[1-(3-Chlorophenyl)methyl] (2-phenylpyridino[4,3-b]pyridin-4-yl)carboxamid;
N-[1-(4-Trifluoromethylphenyl)methyl](2-phenylpyridino[4,3-b]pyridin-4-yl) carboxamid;
N-[1-(3-Trifluoromethylphenyl)methyl](2-phenylpyridino[4,3-b]pyridin-4-yl) carboxamid;
N-[1-(4-Bromophenyl)methyl](2-phenylpyridino[4,3-b]pyridin-4-yl)carboxamid;
4-Ethyl-4-phenyl-2-(2-phenylpyridino[2,3-b]pyridin-4-yl)imidazolin;
4-Isobutyl-4-phenyl-2-(2-phenylpyridino[2,3-b]pyridin-4-yl)imidazolin;
4-Ethyl-4-phenyl-2-(2-phenylpyridino[4,3-b]pyridin-4-yl)imidazolin; oder
4-Isobutyl-4-phenyl-2-(2-phenylpyridino[4,3-b]pyridin-4-yl)imidazolin.

3. Alle stereoisomeren Formen einer Verbindung gemäß Anspruch 1 und Mischungen derselben.

4. Verbindung gemäß einem der Ansprüche 1 bis 3 für die Verwendung bei der therapeutischen Behandlung einer Erkrankung oder Störung, die mit der patogenen Neurokinin-3-Rezeptor-Aktivierung assoziiert ist.

5. Pharmazeutische Zubereitung, umfassend eine Verbindung gemäß einem der Ansprüche 1 bis 4, kombiniert mit mindestens einem pharmazeutisch annehmbaren Träger oder Hilfsstoff.

6. Verwendung einer Verbindung, wie sie in einem der Ansprüche 1 bis 3 beansprucht ist, bei der Herstellung eines Medikaments zur Behandlung oder Vorbeugung einer Erkrankung oder Störung, verbunden mit der pathogenen Neurokinin-3-Rezeptor-Aktivierung.

7. Verwendung einer Verbindung, wie in den Ansprüchen 1 bis 3 beschrieben, bei der Herstellung eines Medikaments zur Behandlung oder Vorbeugung von Angstzuständen, Depression, Fettleibigkeit, Schmerz oder chronischen pulmonären obstruktiven Störungen.

8. Verfahren zur Lokalisation von Neurokinin-3-Rezeptoren in einer Gewebeprobe, umfassend das In-Kontakt-Bringen einer detektierbar markierten Verbindung nach einem der Ansprüche 1 bis 3 mit der Probe unter Bedingungen, die die Bindung der Verbindung an Neurokinin-3-Rezeptoren gestatten, Waschen der Probe zur Entfernung ungebundener Verbindung und Detektieren der gebundenen Verbindung.

9. Verfahren zum Inhibieren der Bindung eines Neurokinins an einen Neurokinin-3-Rezeptor, das Verfahren umfassend das In-Kontakt-Bringen einer Verbindung nach einem der Ansprüche 1 bis 3 mit Zellen, die einen solchen Rezeptor exprimieren, in Anwesenheit eines Neurokinins, worin die Verbindung in einer ausreichenden Konzentration vorhanden ist, um das Neurokinin am Binden von Zellen zu hindern, welche einen klonierten humanen NK-3-Rezeptor *in vitro* exprimieren.

10. Verfahren zum Ändern der Signal übermittelnden Aktivität von Neurokinin-3-Rezeptoren, das Verfahren umfassend das Exponieren von Zellen, welche solche Rezeptoren exprimieren, an eine Verbindung nach einem der Ansprüche 1 bis 3 in ausreichender Konzentration, um die Bindung des Neurokinins an die Zellen, die einen klonierten humanen Neurokinin-3-Rezeptor *in vitro* exprimieren, zu inhibieren.

11. Verpackte pharmazeutische Zubereitung, umfassend die pharmazeutische Zubereitung gemäß Anspruch 5 in einem Behälter und Instruktionen für die Anwendung der Zubereitung, um einen Patienten zu behandeln, der an einer Störung leidet, die auf Neurokinin-3-Rezeptor-Antagonismus anspricht.

12. Die verpackte pharmazeutische Zubereitung gemäß Anspruch 12, worin der Patient an Angstzuständen, Depression, Schizophrenie, Fettleibigkeit oder einer chronischen pulmonären obstruktiven Störung leidet.

13. Verbindung gemäß einem der Ansprüche 1 bis 3, worin die Verbindung in einem Test auf die Neurokinin-3-Rezeptor-Bindung, der wie in Beispiel IX der Beschreibung dargestellt ausgeführt wird, einen IC₅₀ von 1 Micromolar oder darunter aufweist.

## Revendications

1. Composé de formule : 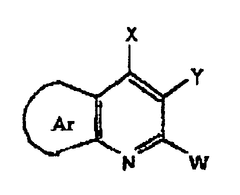 ou les sels non-toxiques pharmaceutiquement acceptables ou les solvates pharmaceutiquement acceptables de celui-ci, dans laquelle :
Y est
un hydrogène, un alkyle inférieur à chaîne linéaire ou ramifiée ayant de 1 à 6 atomes de carbone, un halogène, un amino, un hydroxyle ou un alcoxy inférieur ayant de 1 à 6 atomes de carbone ; ou
Y est
un alkyle inférieur à chaîne linéaire ou ramifiée ayant de 1 à 6 atomes de carbone, ou un alcoxy inférieur ayant de 1 à 6 atomes de carbone dont chacun est substitué sur la chaîne alkyle avec un amino ou un groupe mono ou dialkylamino où chaque alkyle est indépendamment un alkyle inférieur ;
W est
un phényle, un thiényle, ou un pyridyle, dont chacun peut être mono ou disubstitué avec un halogène, un cyano, un trifluorométhyle, un trifluorométhoxy, un hydroxy, un alkyle inférieur à chaîne linéaire ou ramifiée ayant de 1 à 6 atomes de carbone, un alcoxy inférieur à chaîne linéaire ou ramifiée ayant de 1 à 6 atomes de carbone, un amino, ou un mono ou dialkylamino où chaque alkyle est un alkyle inférieur à chaîne linéaire ou ramifiée ayant de 1 à 6 atomes de carbone ; ou
W est
un (alkyl en C₁ à C₆)-phényle, un (alkyl en C₁ à C₆)-thiényle, un (alkyl en C₁ à C₆)-pyridyle, où la partie aromatique de chacun est éventuellement mono ou disubstituée avec un halogène, un cyano, un trifluorométhyle, un trifluorométhoxy, un hydroxy, un alkyle inférieur à chaîne linéaire ou ramifiée ayant de 1 à 6 atomes de carbone, un alcoxy inférieur à chaîne linéaire ou ramifiée ayant de 1 à 6 atomes de carbone, un amino, ou un mono ou un diaklyamino où chaque alkyle est un alkyle inférieur à chaîne linéaire ou ramifiée ayant de 1 à 6 atomes de carbone ; ou
W est
pipéridinyle, morpholinyle, thiomorpholinyle, pyrrolindinyle, pipérazinyle, homopipérazinyle, azabicyclo[3.2.2]nonanyle, isoindolinyle, dont chacun est non substitué, ou mono ou disubstitué avec des groupes alkyle ou aryle, ou condensés avec un cycle aromatique ; ou
W est
un azote qui est mono ou disubstitué avec un hydrogène, une chaîne alkyle linéaire ou ramifiée constituée de 1 à 8 atomes de carbone, un cycloalkyle contenant de 3 à 8 atomes de carbone, une chaîne alkyle ayant de 1 à 6 atomes de carbone qui est attachée à n'importe quelle position à un cycle aryle, une chaîne alkyle ayant de 1 à 6 atomes de carbone qui est attachée à n'importe quelle position à un cycloalkyle contenant de 3 à 8 atomes de carbone, un phényle, un thiényle, un pyridyle, un imidazolyle, un benzimidazolyle, ou n'importe quel cycle ayant un caractère aromatique ; ou
W est
un oxygène qui est substitué avec
une chaîne alkyle linéaire ou ramifiée ayant de 1 à 8 atomes de carbone, un cycloalkyle ayant de 3 à 8 atomes de carbone, ou un phényl mono ou disubstitué avec un halogène, un cyano, un trifluorométhyle, un trifluorométhoxy, un hydroxy, un alkyle inférieur à chaîne linéaire ou ramifiée ayant de 1 à 6 atomes de carbone, un amino, un mono ou dialkylamino où chaque alkyle est une chaîne linéaire ou ramifiée ayant de 1 à 6 atomes de carbone, ou un alcoxy inférieur à chaîne linéaire ou ramifiée ayant de 1 à 6 atomes de carbone ; 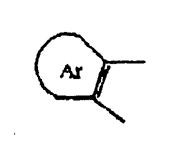 représente 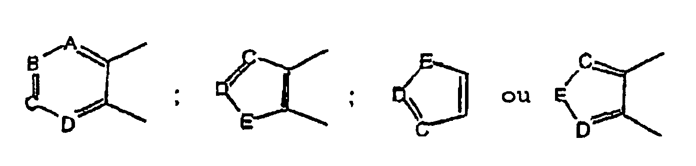
dans laquelle :
A représente un azote ou C-R₁
B représente un azote ou C-R₂ ;
C représente un azote ou C-R₃ ;
D représente un azote ou C-R₄ à la condition que si les quatre A, B, C et D sont présents, au moins un mais pas plus de 2 de A, B, C et D sont l'azote ; et
E représente un oxygène, un soufre ou N-R₅ ;
R₁ à R₄ sont identiques ou différents et représentent
un hydrogène, un halogène, un cyano, un trifluorométhyle, un trifluorométhoxy, un hydroxy, un alkyle inférieur à chaîne linéaire ou ramifiée ayant de 1 à 6 atomes de carbone, un amino, un mono ou un dialkylamino où chaque alkyle est un alkyle inférieur à chaîne linéaire ou ramifiée ayant de 1 à 6 atomes de carbone, ou un alcoxy inférieur à chaîne linéaire ou ramifiée ayant de 1 à 6 atomes de carbone :
R₅ est
un hydrogène ou un alkyle inférieur à chaîne linéaire ou ramifiée ayant de 1 à 6 atomes de carbone ; ou
un phényle, un thiényle, ou un pyridyle, dont chacun peut être mono ou disubstitué avec un halogène, un cyano, un trifluorométhyle, un trifluorométhoxy, un hydroxy, un alkyle inférieur à chaîne linéaire ou ramifiée ayant de 1 à 6 atomes de carbone, un amino, un mono ou dialkylamino où chaque alkyle est un alkyle inférieur à chaîne linéaire ou ramifiée ayant de 1 à 6 atomes de carbone, ou un alcoxy inférieur à chaîne linéaire ou ramifiée ayant de 1 à 6 atomes de carbone ;
X est 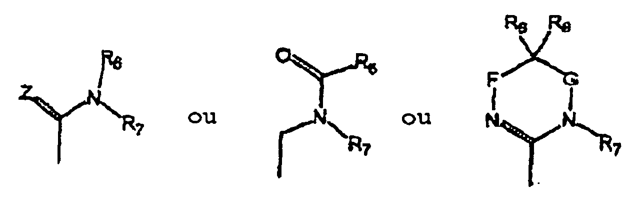
où
F est un carbonyle, un méthylène ou un éthylène ;
G est un méthylène ou une liaison à la condition que G soit une liaison quand F est un éthylène ; et
Z est un oxygène, un soufre, N-C≡N ou deux atomes d'hydrogène ;
R₆ est
un phényle, un thiényle, ou un pyridyle, dont chacun peut être mono ou disubstitué avec un halogène, un cyano, un trifluorméthyle, un trifluorométhoxy, un hydroxy, un alkyle inférieur à chaîne linéaire ou ramifiée ayant de 1 à 6 atomes de carbone, un amino, un mono ou dialkylamino où chaque alkyle est un alkyle inférieur linéaire ou ramifié ayant de 1 à 6 atomes de carbone, ou un alcoxy inférieur linéaire ou ramifié ayant de 1 à 6 atomes de carbone ; ou
R₆ est
un alkyle linéaire ou ramifié ayant de 1 à 8 atomes de carbone ou un cycloalkyle contenant de 3 à 8 atomes de carbone dont chacun est éventuellement mono ou disubstitué avec un cycle aromatique où chaque cycle aromatique est éventuellement mono ou disubstitué indépendamment avec un halogène, un cyano, un trifluorométhyle, un trifluorométhoxy, un hydroxy, un alkyle inférieur à chaîne linéaire ou ramifiée ayant de 1 à 6 atomes de carbone, un amino, un amino mono ou disubstitué où le substituant sur l'azote est un alkyle à chaîne linéaire ou ramifiée ayant de 1 à 6 atomes de carbone ou un alcoxy à chaîne linéaire ou ramifiée ayant de 1 à 6 atomes de carbone ; ou
R₆ est
un cycloalkyle contenant de 5 à 8 atomes de carbone dans lequel le cycloalkyle est condensé à un cycle aromatique à des positions adjacentes ;
R₇ et R₈ sont identiques ou différents et représentent
un hydrogène ou un alkyle inférieur à chaîne linéaire ou ramifiée ayant de 1 à 6 atomes de carbone ; ou
R₆ et R₇ pris ensemble forment un cycle à 5, 6, 7 ou 8 membres qui est mono ou disubstitué avec un groupe aryle ou contient un cycle aryle condensé comme partie du cycle à 5, 6, 7 ou 8 membres décrit ci-dessus, à la condition que R₆ et R₇ pris ensemble ne forment pas un cycle quand G est un méthylène ; ou
R₆ et R₈ pris ensemble peuvent former un cycle à 5, 6, 7 ou 8 membres qui est mono ou disubstitué avec un groupe aryle ou contient un cycle aryle condensé comme partie du cycle à 5, 6, 7 ou 8 membres.

2. Composé selon la revendication 1, qui est le N-[(S)-1-phényl-1-propyl)(2-phénylpyridino[4,3-b]pyridin-4-yl)carboxamide ;
le N-[(R)-1-phényl-1-propyl)(2-phénylpyridino[4,3-b]pyridin-4-yl)carboxamide ;
le N-((S)-1-phényl-1-propyl)(2-phénylpyridino[2,3-b]pyridin-4-yl)carboxamide ;
le N-((R)-1-phényl-1-propyl)(2-phénylpyridino[2,3-b]pyridin-4-yl)carboxamide ;
le N-(diphénylméthyl)N-méthyl(2-phénylpyridino[2,3-b]pyridin-4-yl)carboxamide ;
le N-(diphénylméthyl)N-méthyl(2-phénylpyridino[4,3-b]pyridin-4-yl)carboxamide ;
le N-((S)-1-phényl-1-propyl)(2-phénylthiéno[3,2-b]pyridin-4-yl)carboxamide ;
le N-[1-(phényl)éthyl)]-N-méthyl(2-phényl-pyridino[2,3-b]pyridin-4-yl)carboxamide ;
le N-[1-(phényl)butyl)-N-méthyl(2-phényl-pyridino[2,3-b]pyridin-4-yl)carboxamide ;
le N-[1-(phényl)pentyl)-N-méthyl(2-phényl-pyridino[2,3-b]pyridin-4-yl)carboxamide ;
le N-[1-(phényl)hexyl)-N-méthyl(2-phényl-pyridino[2,3-b]pyridin-4-yl)carboxamide ;
le N-[1-(diphényl)méthyl)-N-méthyl(2-phényl-pyridino[2,3-b]pyridin-4-yl)carboxamide ;
le N-[1-(1,2-diphényl)éthyl)-N-méthyl(2-phényl-pyridino[2,3-b]pyridin-4-yl)carboxamide ;
le N- [1-(1-phényl-2-cyclohexyl)éthyl)-N-méthyl (2-phénylpyridino[2,3-b]pyridin-4-yl)carboxamide ;
le N-indanyl-N-méthyl(2-phénylpyridino[2,3-b]pyridin-4-yl)carboxamide ;
le N-[1-(phényl)isobutyl)-N-méthyl(2-phényl-pyridino[2,3-b]pyridin-4-yl)carboxamide ;
le N-[1-(phényl)2-méthylbutyl)-N-méthyl(2-phényl-pyridino[2,3-b]pyridin-4-yl)carboxamide ;
la 1-isobutyl(2-1,2,3,4-tétrahydroisoquinolyl)(2-phénylpyridino[2,3-b]pyridin-4-yl)cétone ;
la 1-(2-méthylphényl)(2-1,2,3,4-tétrahydroisoquinolyl)(2-phénylpyridino[2,3-b]pyridin-4-yl)cétone ;
la 1-(2-chlorophényl)(2-1,2,3,4-tétrahydroisoquinolyl)(2-phénylpyridino[2,3-b]pyridin-4-yl)cétone ;
la 1-méthyl(2-1,2,3,4-tétrahydroisoquinolyl)(2-phénylpyridino[2,3-b]pyridin-4-yl)cétone ;
la 1-propyl(2-1,2,3,4-tétrahydroisoquinolyl)(2-phénylpyridino[2,3-b]pyridin-4-yl)cétone ;
la 1-butyl(2-1,2,3,4-tétrahydroisoquinolyl)(2-phénylpyridino[2,3-b]pyridin-4-yl)cétone ;
la 1-cyclopentyl(2-1,2,3,4-tétrahydroisoquinolyl)(2-phénylpyridino[2,3-b]pyridin-4-yl)cétone ;
la 1-pentyl(2-1,2,3,4-tétrahydroisoquinolyl)(2-phénylpyridino[2,3-b]pyridin-4-yl)cétone ;
la 1-benzyl(2-1,2,3,4-tétrahydroisoquinolyl)(2-phénylpyridino[2,3-b]pyridin-4-yl)cétone ;
la 1-cyclohexylméthyl(2-1,2,3,4-tétrahydroisoquinolyl)(2-phénylpyridino[2,3-b]pyridin-4-yl)cétone ;
la 1-isopropyl(2-1,2,3,4-tétrahydroisoquinolyl)(2-phénylpyridino[2,3-b]pyridin-4-yl)cétone ;
le N-[1-(phényl)octyl]-N-méthyl(2-phénylpyridino [2,3-b]pyridin-4-yl)carboxamide ;
la 1-(2-méthylbutyl)(2-1,2,3,4-tétrahydroisoquinolyl)(2-phénylpyridino[2,3-b]pyridin-4-yl)cétone ;
le N-[1,3-(diphényl)propyl]-N-méthyl(2-phényl-pyridino [2,3-b]pyridin-4-yl)carboxamide ;
le N-[1,4-(diphényl)butyl]-N-méthyl(2-phénylpyridino [2,3-b]pyridin-4-yl)carboxamide ;
le N-benzyl-N-méthyl(2-phénylpyridino[2,3-b]pyridin-4-yl)carboxamide ;
le N-benzyl-N-éthyl(2-phénylpyridino[2,3-b]pyridin-4-yl)carboxamide ;
le N-benzyl-N-isopropyl(2-phénylpyridino[2,3-b]pyridin-4-yl)carboxamide ;
le N-benzyl-N-butyl(2-phénylpyridino[2,3-b]pyridin-4-yl)carboxamide ;
le N-benzyl-N-isobutyl(2-phénylpyridino[2,3-b]pyridin-4-yl)carboxamide ;
le N-benzyl-N-cyclopentyl(2-phénylpyridino[2,3-b]pyridin-4-yl)carboxamide ;
le N-benzyl-N-pentyl(2-phénylpyridino[2,3-b]pyridin-4-yl)carboxamide ;
le N-benzyl-N-cyclohexyl(2-phénylpyridino[2,3-b]pyridin-4-yl)carboxamide ;
le N,N-dibenzyl-(2-phénylpyridino[2,3-b]pyridin-4-yl)carboxamide ;
le N-benzyl-N-1-méthylpropyl(2-phénylpyridino[2,3-b]pyridin-4-yl)carboxamide ;
le N-benzyl-N-1-méthylbutyl(2-phénylpyridino[2,3-b]pyridin-4-yl)carboxamide ;
le N-benzyl-N-4-méthylbutyl(2-phénylpyridino[2,3-b]pyridin-4-yl)carboxamide ;
le N-benzyl-cyclopentylméthyl(2-phénylpyridino[2,3-b]pyridin-4-yl)carboxamide ;
le N-benzyl-N-hexyl(2-phénylpyridino[2,3-b]pyridin-4-yl)carboxamide ;
le N-benzyl-N-cyclohexylméthyl(2-phénylpyridino[2,3-b]pyridin-4-yl)carboxamide ;
le N-benzyl-N-heptyl(2-phénylpyridino[2,3-b]pyridin-4-yl)carboxamide ;
le N-méthyl(2-phénylpyridino[2,3-b]pyridin-4-yl)carboxamide ;
le N-[1-(phénylcyclopentyl)méthyl]-N-méthyl(2-phénylpyridino[2,3-b]pyridin-4-yl)carboxamide ;
le N-[1-(phénylcyclohexyl)méthyl] -N-méthyl (2-phényl-pyridino[2,3-b]pyridin-4-yl)carboxamide ;
le N-[1-(1-phényl-2-cyclopentyl)éthyl]-N-méthyl(2-phénylpyridino[2,3-b]pyridin-4-yl)carboxamide ;
le N-[1-(1,3-diphényl)propyl]-N-méthyl(2-phényl-pyridino[2,3-b]pyridin-4-yl)carboxamide ;
le N-[1-(1,4-diphényl)butyl]-N-méthyl(2-phényl-pyridino[2,3-b]pyridin-4-yl)carboxamide ;
la 1-phényl(2-1,2,3,4-tétrahydroisoquinolyl)(2-phényl-pyridino[2,3-b]pyridin-4-yl)cétone ;
la 1-cyclohexyl(2-1,2,3,4-tétrahydroisoquinolyl)(2-phénylpyridino[2,3-b]pyridin-4-yl)cétone ;
la 1-cyclopentylméthyl(2-1,2,3,4-tétrahydroisoquinolyl)(2-phénylpyridino[2,3-b]pyridin-4-yl)cétone ;
la 1-hexyl(2-1,2,3,4-tétrahydroisoquinolyl)(2-phénylpyridino[2,3-b]pyridin-4-yl)cétone ;
la 1-(2-fluorophényl)(2-1,2,3,4-tétrahydroisoquinolyl)(2-phénylpyridino[2,3-b]pyridin-4-yl)cétone ;
la 1-(2-trifluorométhylphényl)(2-1,2,3,4-tétrahydroisoquinolyl)(2-phénylpyridino[2,3-b]pyridin-4-yl)cétone ;
la 1-(3-méthylphényl)(2-1,2,3,4-tétrahydroisoquinolyl)(2-phénylpyridino[2,3-b]pyridin-4-yl)cétone ;
la 1-(4-méthylphényl)(2-1,2,3,4-tétrahydroisoquinolyl)(2-phénylpyridino[2,3-b]pyridin-4-yl)cétone ;
la 1-(4-fluorophényl)(2-1,2,3,4-tétrahydroisoquinolyl)(2-phénylpyridino[2,3-b]pyridin-4-yl)cétone ;
la 1-cycloheptyl(2-1,2,3,4-tétrahydroisoquinolyl)(2-phénylpyridino[2,3-b]pyridin-4-yl)cétone ;
la 1-(3-chlorophényl)(2-1,2,3,4-tétrahydroisoquinolyl)(2-phénylpyridino[2,3-b]pyridin-4-yl)cétone ;
la 1-(4-chlorophényl)(2-1,2,3,4-tétrahydroisoquinolyl)(2-phénylpyridino[2,3-b]pyridin-4-yl)cétone ;
la 6,7-diméthoxy(2-1,2,3,4-tétrahydroisoquinolyl)(2-phénylpyridino[2,3-b]pyridin-4-yl)cétone ;
la 6,7-diméthoxy-1-méthyl(2-1,2,3,4-tétrahydroisoquinolyl)(2-phénylpyridino[2,3-b]pyridin-4-yl)cétone ;
la 6,7-diméthoxy-1-phényl(2-1,2,3,4-tétrahydroisoquinolyl)(2-phénylpyridino[2,3-b]pyridin-4-yl)cétone ;
la 6,7-diméthoxy-3-méthyl(2-1,2,3,4-tétrahydroisoquinolyl)(2-phénylpyridino[2,3-b]pyridin-4-yl)cétone ;
la 2-[6,7-diméthoxy-2-(2-1,2,3,4-tétrahydroisoquinolyl)(2-phénylpyridino[2,3-b]pyridin-4-yl)éthanenitrilecétone ;
le N-[1-(4-bromophényl)éthyl](2-phénylpyridino[2,3-b]pyridin-4-yl)carboxamide ;
le N-[1-(4-méthylphényl)éthyl](2-phénylpyridino [2,3-b]pyridin-4-yl)carboxamide ;
le N-[1-(3-méthylphényl)éthyl](2-phénylpyridino[2,3-b]pyridin-4-yl)carboxamide ;
le N- [1- (4-fluorophényl)éthyl] (2-phénylpyridino [2, 3-b]pyridin-4-yl)carboxamide ;
le N-[1-(3-fluorophényl)éthyl](2-phénylpyridino[2,3-b]pyridin-4-yl)carboxamide ;
le N-[1-(4-méthoxyphényl)éthyl](2-phénylpyridino [2,3-b]pyridin-4-yl)carboxamide ;
le N-[1-(3-méthoxyphényl)éthyl](2-phénylpyridino [2,3-b]pyridin-4-yl)carboxamide ;
le N-[1-(4-chlorophényl)éthyl](2-phénylpyridino[2,3-b]pyridin-4-yl)carboxamide ;
le N-[1-(3-chlorophényl)éthyl](2-phénylpyridino[2,3-b]pyridin-4-yl)carboxamide ;
le N-[1-(4-trifluorométhylphényl)éthyl](2-phényl-pyridino[2,3-b]pyridin-4-yl)carboxamide ;
le N-[1-(3-trifluorométhylphényl)éthyl](2-phényl-pyridino[2,3-b]pyridin-4-yl)carboxamide ;
le N-[1-(4-méthylphényl)méthyl]-N-méthyl(2-phényl-pyridino[2,3-b]pyridin-4-yl)carboxamide ;
le N-[1-(3-méthylphényl)méthyl]-N-méthyl(2-phényl-pyridino[2,3-b]pyridin-4-yl)carboxamide ;
le N- [1-(4-fluorophényl)méthyl]-N-méthyl(2-phényl-pyridino[2,3-b]pyridin-4-yl)carboxamide ;
le N- [1- (3-fluorophényl)méthyl] -N-méthyl (2-phénylpyridino[2,3-b]pyridin-4-yl)carboxamide ;
le N- [1-(4-méthoxyphényl)méthyl]-N-méthyl(2-phényl-pyridino[2,3-b]pyridin-4-yl)carboxamide ;
le N-[1-(3-méthoxyphényl)méthyl]-N-méthyl(2-phényl-pyridino[2,3-b]pyridin-4-yl)carboxamide ;
le N- [1-(4-chlorophényl)méthyl]-N-méthyl(2-phényl-pyridino[2,3-b]pyridin-4-yl)carboxamide ;
le N-[1-(3-chlorophényl)méthyl]-N-méthyl(2-phényl-pyridino[2,3-b]pyridin-4-yl)carboxamide ;
le N-[1-(4-trifluorométhylphényl)méthyl]-N-méthyl(2-phénylpyridino[2,3-b]pyridin-4-yl)carboxamide ;
le N- [1-(3-trifluorométhylphényl)méthyl] -N-méthyl (2-phénylpyridino[2,3-b]pyridin-4-yl)carboxamide ;
le N-[1-(4-bromophényl)méthyl]-N-méthyl(2-phényl-pyridino[2,3-b]pyridin-4-yl)carboxamide ;
le N-[1-(2-méthylphényl)méthyl](2-phénylpyridino [2,3-b]pyridin-4-yl)carboxamide ;
le N-[1-(2-fluorophényl)méthyl](2-phénylpyridino [2,3-b]pyridin-4-yl)carboxamide ;
le N-[1-(2-méthoxyphényl)méthyl](2-phénylpyridino [2,3-b]pyridin-4-yl)carboxamide ;
le N-[1-(2-chlorophényl)méthyl](2-phénylpyridino [2,3-b]pyridin-4-yl)carboxamide ;
le N-[1-(2-trifluorométhylphényl)méthyl](2-phényl-pyridino[2,3-b]pyridin-4-yl)carboxamide ;
le N-[1-(2-méthylphényl)éthyl](2-phénylpyridino[2,3-b]pyridin-4-yl)carboxamide ;
le N-[1-(2-fluorophényl)éthyl](2-phénylpyridino[2,3-b]pyridin-4-yl)carboxamide ;
le N-[1-(2-méthoxyphényl)éthyl](2-phénylpyridino [2,3-b]pyridin-4-yl)carboxamide ;
le N-[1-(2-chlorophényl)éthyl] (2-phénylpyridino[2,3-b]pyridin-4-yl)carboxamide ;
le N- [1- (2-trifluorométhylphényl)éthyl](2-phényl-pyridino[2,3-b]pyridin-4-yl)carboxamide ;
le N-[1-(2,4-dichlorophényl)éthyl](2-phénylpyridino [2,3-b]pyridin-4-yl)carboxamide ;
le N-[1-(4-méthylphényl)méthyl](2-phénylpyridino [2,3-b]pyridin-4-yl)carboxamide ;
le N- [1-(3-méthylphényl)méthyl](2-phénylpyridino [2,3-b]pyridin-4-yl)carboxamide ;
le N-[1-(4-fluorophényl)méthyl](2-phénylpyridino [2,3-b]pyridin-4-yl)carboxamide ;
le N-[1-(3-fluorophényl)méthyl](2-phénylpyridino [2,3-b]pyridin-4-yl)carboxamide ;
le N-[1-(4-méthoxyphényl)méthyl](2-phénylpyridino [2,3-b]pyridin-4-yl)carboxamide ;
le N-[1-(3-méthoxyphényl)méthyl](2-phénylpyridino [2,3-b]pyridin-4-yl)carboxamide ;
le N-[1-(4-chlorophényl)méthyl](2-phénylpyridino [2,3-b]pyridin-4-yl)carboxamide ;
le N-[1-(3-chlorophényl)méthyl](2-phényl-pyridino[2,3-b]pyridin-4-yl)carboxamide ;
le N-[1-(4-trifluorométhylphényl)méthyl](2-phényl-pyridino[2,3-b]pyridin-4-yl)carboxamide ;
le N-[1-(3-trifluorométhylphényl)méthyl](2-phényl-pyridino[2,3-b]pyridin-4-yl)carboxamide ;
le N-[1-(4-bromophényl)méthyl](2-phénylpyridino[2,3-b]pyridin-4-yl)carboxamide ;
le N-[1-(phényl)éthyl]-N-méthyl(2-phénylpyridino [4,3-b]pyridin-4-yl)carboxamide ;
le N-[1-(phényl)butyl]-N-méthyl(2-phénylpyridino [4,3-b]pyridin-4-yl)carboxamide ;
le N-[1-(phényl)pentyl]-N-méthyl(2-phénylpyridino [4,3-b]pyridin-4-yl)carboxamide ;
le N-[1-(phényl)hexyl]-N-méthyl(2-phénylpyridino [4,3-b]pyridin-4-yl)carboxamide ;
le N-[1-(diphényl)méthyl]-N-méthyl(2-phénylpyridino [4,3-b]pyridin-4-yl)carboxamide ;
le N- [1- (1,2-diphényl)éthyl]-N-méthyl (2-phényl-pyridino[4,3-b]pyridin-4-yl)carboxamide ;
le N- [1-(1-phényl-2-cyclohexyl)éthyl]-N-méthyl(2-phénylpyridino[4,3-b]pyridin-4-yl)carboxamide ;
le N-indanyl-N-méthyl(2-phénylpyridino[4,3-b]pyridin-4-yl)carboxamide ;
le N-[1-(phényl)isobutyl]-N-méthyl(2-phénylpyridino [4,3-b]pyridin-4-yl)carboxamide ;
le N-[1-(phényl)2-méthylbutyl]-N-méthyl(2-phényl-pyridino[4,3-b]pyridin-4-yl)carboxamide ;
la 1-isobutyl(2-1,2,3,4-tétrahydroisoquinolyl)(2-phénylpyridino[4,3-b]pyridin-4-yl)cétone ;
la 1-(2-méthylphényl)(2-1,2,3,4-tétrahydroisoquinolyl)(2-phénylpyridino[4,3-b]pyridin-4-yl)cétone ;
la 1-(2-chlorophényl)(2-1,2,3,4-tétrahydroisoquinolyl)(2-phénylpyridino[4,3-b]pyridin-4-yl)cétone ;
la 1-méthyl(2-1,2,3,4-tétrahydroisoquinolyl)(2-phénylpyridino[4,3-b]pyridin-4-yl)cétone ;
la 1-propyl(2-1,2,3,4-tétrahydroisoquinolyl)(2-phénylpyridino[4,3-b]pyridin-4-yl)cétone ;
la 1-butyl(2-1,2,3,4-tétrahydroisoquinolyl)(2-phénylpyridino[4,3-b]pyridin-4-yl)cétone ;
la 1-cyclopentyl(2-1,2,3,4-tétrahydroisoquinolyl)(2-phénylpyridino[4,3-b]pyridin-4-yl)cétone ;
la 1-pentyl(2-1,2,3,4-tétrahydroisoquinolyl)(2-phénylpyridino[4,3-b]pyridin-4-yl)cétone ;
la 1-benzyl(2-1,2,3,4-tétrahydroisoquinolyl)(2-phénylpyridino[4,3-b]pyridin-4-yl)cétone ;
la 1-cyclohexylméthyl(2-1,2,3,4-tétrahydroisoquinolyl)(2-phénylpyridino[4,3-b]pyridin-4-yl)cétone ;
la 1-isopropyl(2-1,2,3,4-tétrahydroisoquinolyl)(2-phénylpyridino[4,3-b]pyridin-4-yl)cétone ; et
le N-[1-(phényl)octyl]-N-méthyl(2-phénylpyridino [4,3-b]pyridin-4-yl)carboxamide.
composé selon la revendication 1, qui est choisi dans le groupe constitué par :
la 1-(2-méthylbutyl)(2-1,2,3,4-tétrahydroisoquinolyl)(2-phénylpyridino[4,3-b]pyridin-4-yl)cétone ;
le N- [1,3-(diphényl)propyl]-N-méthyl(2-phénylpyridino[4,3-b]pyridin-4-yl)carboxamide ;
le N- [1,4-(diphényl)butyl]-N-méthyl(2-phénylpyridino[4,3-b]pyridin-4-yl)carboxamide ;
le N-benzyl-N-méthyl(2-phénylpyridino[4,3-b]pyridin-4-yl)carboxamide ;
le N-benzyl-N-éthyl(2-phénylpyridino[4,3-b]pyridin-4-yl)carboxamide ;
le N-benzyl-N-isopropyl(2-phénylpyridino[4,3-b]pyridin-4-yl)carboxamide ;
le N-benzyl-N-butyl(2-phénylpyridino[4,3-b]pyridin-4-yl)carboxamide ;
le N-benzyl-N-isobutyl(2-phénylpyridino[4,3-b]pyridin-4-yl)carboxamide ;
le N-benzyl-N-cyclopentyl(2-phénylpyridino[4,3-b]pyridin-4-yl)carboxamide ;
le N-benzyl-N-pentyl(2-phénylpyridino[4,3-b]pyridin-4-yl)carboxamide ;
le N-méthyl(2-phénylpyridino[4,3-b]pyridin-4-yl)carboxamide ;
le N-[1-(phénylcyclopentyl)méthyl]-N-méthyl(2-phénylpyridino[4,3-b]pyridin-4-yl)carboxamide ;
le N-[1-(phénylcyclohexyl)méthyl]-N-méthyl(2-phénylpyridino[4,3-b]pyridin-4-yl)carboxamide ;
le N-[1-(1-phényl-2-cyclopentyl)éthyl]-N-méthyl(2-phénylpyridino[4,3-b]pyridin-4-yl)carboxamide ;
le N-[(1,3-diphényl)propyl]-N-méthyl(2-phénylpyridino[4,3-b]pyridin-4-yl)carboxamide ;
le N-[(1,4-diphényl)butyl]-N-méthyl(2-phénylpyridino[4,3-b]pyridin-4-yl)carboxamide ;
la 1-phényl(2-1,2,3,4-tétrahydroisoquinolyl)(2-phénylpyridino[4,3-b]pyridin-4-yl)cétone ;
la 1-cyclohexyl(2-1,2,3,4-tétrahydroisoquinolyl)(2-phénylpyridino[4,3-b]pyridin-4-yl)cétone ;
la 1-cyclopentylméthyl(2-1,2,3,4-tétrahydroisoquinolyl)(2-phénylpyridino[4,3-b]pyridin-4-yl)cétone ;
la 1-hexyl(2-1,2,3,4-tétrahydroisoquinolyl)(2-phénylpyridino[4,3-b]pyridin-4-yl)cétone ;
la 1-(2-fluorophényl)(2-1,2,3,4-tétrahydroisoquinolyl)(2-phénylpyridino[4,3-b]pyridin-4-yl)cétone ;
la 1-(3-méthylphényl)(2-1,2,3,4-tétrahydroisoquinolyl)(2-phénylpyridino[4,3-b]pyridin-4-yl)cétone ;
la 1-(4-méthylphényl)(2-1,2,3,4-tétrahydroisoquinolyl)(2-phénylpyridino[4,3-b]pyridin-4-yl)cétone ;
la 1-(4-fluorophényl)(2-1,2,3,4-tétrahydroisoquinolyl)(2-phénylpyridino[4,3-b]pyridin-4-yl)cétone ;
la 1-cycloheptyl(2-1,2,3,4-tétrahydroisoquinolyl)(2-phénylpyridino[4,3-b]pyridin-4-yl)cétone ;
la 1-(3-chlorophényl)(2-1,2,3,4-tétrahydroisoquinolyl)(2-phénylpyridino[4,3-b]pyridin-4-yl)cétone ;
la 1-(4-chlorophényl)(2-1,2,3,4-tétrahydroisoquinolyl)(2-phénylpyridino[4,3-b]pyridin-4-yl)cétone ;
la 6,7-diméthoxy(2-1,2,3,4-tétrahydroisoquinolyl)(2-phénylpyridino[4,3-b]pyridin-4-yl)cétone ;
la 6,7-diméthoxy-1-méthyl(2-1,2,3,4-tétrahydroisoquinolyl)(2-phénylpyridino[4,3-b]pyridin-4-yl)cétone ;
la 6,7-diméthoxy-1-phényl(2-1,2,3,4-tétrahydroisoquinolyl)(2-phénylpyridino[4,3-b]pyridin-4-yl)cétone ;
la 6,7-diméthoxy-3-méthyl(2-1,2,3,4-tétrahydroisoquinolyl)(2-phénylpyridino[4,3-b]pyridin-4-yl)cétone ;
la 2-[6,7-diméthoxy-2-(2-1,2,3,4-tétrahydroisoquinolyl) (2-phénylpyridino[4,3-b]pyridin-4-yl)éthane nitrile cétone ;
le N-[1-(4-bromophényl)éthyl](2-phénylpyridino[4,3-b]pyridin-4-yl)carboxamide ;
le N-[1-(4-méthylphényl)éthyl](2-phénylpyridino[4,3-b] pyridin-4-yl)carboxamide ;
le N- [1-(3-méthylphényl)éthyl](2-phénylpyridino[4,3-b]pyridin-4-yl)carboxamide ;
le N-[1-(4-fluorophényl)éthyl](2-phénylpyridino[4,3-b]pyridin-4-yl)carboxamide ;
le N- [1-(3-fluorophényl)éthyl](2-phénylpyridino[4,3-b]pyridin-4-yl)carboxamide ;
le N-[1-(4-méthoxyphényl)éthyl](2-phénylpyridino [4,3-b]pyridin-4-yl)carboxamide ;
le N-[1-(3-méthoxyphényl)éthyl](2-phénylpyridino [4,3-b]pyridin-4-yl)carboxamide ;
le N-[1-(4-chlorophényl)éthyl](2-phénylpyridino[4,3-b]pyridin-4-yl)carboxamide ;
le N-[1-(3-chlorophényl)éthyl] (2-phénylpyridino[4,3-b]pyridin-4-yl)carboxamide ;
le N-[1-(4-trifluorométhylphényl)éthyl](2-phényl-pyridino[4,3-b]pyridin-4-yl)carboxamide ;
le N-[1-(3-trifluorométhylphényl)éthyl](2-phényl-pyridino[4,3-b]pyridin-4-yl)carboxamide ;
le N-[1-(4-méthylphényl)méthyl]-N-méthyl(2-phényl-pyridino[4,3-b]pyridin-4-yl)carboxamide ;
le N-[1-(3-méthylphényl)méthyl]-N-méthyl(2-phényl-pyridino[4,3-b]pyridin-4-yl)carboxamide ;
le N-[1-(4-fluorophényl)méthyl]-N-méthyl(2-phényl-pyridino[4,3-b]pyridin-4-yl)carboxamide ;
le N-[1-(3-fluorophényl)méthyl]-N-méthyl (2-phényl-pyridino[4,3-b]pyridin-4-yl)carboxamide ;
le N-[1-(4-méthoxyphényl)méthyl]-N-méthyl(2-phényl-pyridino[4,3-b]pyridin-4-yl)carboxamide ;
le N-[1-(3-méthoxyphényl)méthyl]-N-méthyl (2-phényl-pyridino[4,3-b]pyridin-4-yl)carboxamide ;
le N-[1-(4-chlorophényl)méthyl]-N-méthyl(2-phényl-pyridino[4,3-b]pyridin-4-yl)carboxamide ;
le N-[1-(3-chlorophényl)méthyl]-N-méthyl(2-phényl-pyridino[4,3-b]pyridin-4-yl)carboxamide ;
le N-[1-(4-trifluorométhylphényl)méthyl]-N-méthyl (2-phénylpyridino[4,3-b]pyridin-4-yl)carboxamide ;
le N-[1-(3-trifluorométhylphényl)méthyl]-N-méthyl(2-phénylpyridino[4,3-b]pyridin-4-yl)carboxamide ;
le N-[1-(4-bromophényl)méthyl]-N-méthyl (2-phényl-pyridino[4,3-b]pyridin-4-yl)carboxamide ;
le N-[1-(2-méthylphényl)méthyl](2-phénylpyridino [4,3-b]pyridin-4-yl)carboxamide ;
le N-[1-(2-fluorophényl)méthyl](2-phénylpyridino [4,3-b]pyridin-4-yl)carboxamide ;
le N-[1-(2-méthoxyphényl)méthyl](2-phénylpyridino [4,3-b]pyridin-4-yl)carboxamide ;
le N-[1-(2-chlorophényl)méthyl](2-phénylpyridino [4,3-b]pyridin-4-yl)carboxamide ;
le N-[1-(2-trifluorométhylphényl)méthyl](2-phényl-pyridino[4,3-b]pyridin-4-yl)carboxamide ;
le N-[1-(2-méthylphényl)éthyl](2-phénylpyridino[4,3-b]pyridin-4-yl)carboxamide ;
le N-[1-(2-fluorophényl)éthyl](2-phénylpyridino[4,3-b]pyridin-4-yl)carboxamide ;
le N-[1-(2-méthoxyphényl)éthyl](2-phénylpyridino [4,3-b]pyridin-4-yl)carboxamide ;
le N-[1-(2-chlorophényl)éthyl](2-phénylpyridino[4,3-b]pyridin-4-yl)carboxamide ;
le N-[1-(2-trifluorométhylphényl)éthyl](2-phénylpyridino[4,3-b]pyridin-4-yl)carboxamide ;
le N-[1-(2,4-dichlorophényl)éthyl](2-phénylpyridino [4,3-b]pyridin-4-yl)carboxamide ;
le N-[1-(4-méthylphényl)méthyl](2-phénylpyridino [4,3-b]pyridin-4-yl)carboxamide ;
le N-[1-(3-méthylphényl)méthyl](2-phénylpyridino [4,3-b]pyridin-4-yl)carboxamide ;
le N-[1-(4-fluorophényl)méthyl](2-phénylpyridino [4,3-b]pyridin-4-yl)carboxamide ;
le N-[1-(3-fluorophényl)méthyl](2-phénylpyridino [4,3-b]pyridin-4-yl)carboxamide ;
le N-[1-(4-méthoxyphényl)méthyl](2-phénylpyridino[4,3-b]pyridin-4-yl)carboxamide ;
le N-[1-(3-méthoxyphényl)méthyl](2-phénylpyridino [4,3-b]pyridin-4-yl)carboxamide ;
le N-[1-(4-chlorophényl)méthyl](2-phénylpyridino [4,3-b]pyridin-4-yl)carboxamide ;
le N-[1-(3-chlorophényl)méthyl](2-phénylpyridino [4,3-b]pyridin-4-yl)carboxamide ;
le N-[1-(4-trifluorométhylphényl)méthyl](2-phényl-pyridino[4,3-b]pyridin-4-yl)carboxamide ;
le N-[1-(3-trifluorométhylphényl)méthyl](2-phényl-pyridino[4,3-b]pyridin-4-yl)carboxamide ;
le N-[1-(4-bromophényl)méthyl](2-phénylpyridino[4,3-b]pyridin-4-yl)carboxamide ;
la 4-éthyl-4-phényl-2-(2-phénylpyridino[2,3-b]pyridin-4-yl)imidazoline ;
la 4-isobutyl-4-phényl-2-(2-phénylpyridino[2,3-b]pyridin-4-yl) imidazoline ;
la 4-éthyl-4-phényl-2-(2-phénylpyridino[4,3-b]pyridin-4-yl)imidazoline ; ou
la 4-isobutyl-4-phényl-2-(2-phénylpyridino[4,3-b]pyridin-4-yl) imidazoline.

3. Toutes formes stéréoisomères d'un composé selon la revendication 1 et leurs mélanges.

4. Composé selon l'une quelconque des revendications 1 à 3, pour une utilisation dans le traitement thérapeutique d'une maladie ou d'un trouble associé à l'activation pathogène du récepteur de la neurokinine-3.

5. Composition pharmaceutique comprenant un composé selon l'une quelconque des revendications 1 à 4, combinée avec au moins un véhicule ou excipient pharmaceutiquement acceptable.

6. Utilisation d'un composé selon l'une quelconque des revendications 1 à 3, pour la fabrication d'un médicament pour le traitement ou la prévention d'une maladie ou d'un trouble associé à l'activation pathogène de la neurokinine-3.

7. Utilisation d'un composé comme décrit dans les revendications 1 à 3, pour la fabrication d'un médicament pour le traitement ou la prévention de l'anxiété, de la dépression, de l'obésité, de la douleur, ou d'un trouble obstructif pulmonaire chronique.

8. Procédé pour localiser les récepteurs de la neurokinine-3 dans un échantillon de tissu comprenant : la mise en contact avec l'échantillon d'un composé marqué de manière détectable selon l'une quelconque des revendications 1 à 3 dans des conditions qui permettent la liaison du composé aux récepteurs de neurokinine 3, le lavage de l'échantillon pour éliminer le composé non lié, et la détection du composé lié.

9. Procédé d'inhibition de la liaison d'une neurokinine à un récepteur de neurokinine 3, ledit procédé comprenant la mise en contact d'un composé selon l'une quelconque des revendications 1 à 3 avec des cellules exprimant ce récepteur en présence d'une neurokinine, dans lequel le composé est présent à une concentration suffisante pour inhiber la liaison de la neurokinine aux cellules exprimant un récepteur NK-3 humain cloné *in vitro*.

10. Procédé pour altérer l'activité de transduction de signal des récepteurs de la neurokinine-3, ledit procédé comprenant l'exposition des cellules exprimant ces récepteurs à un composé selon l'une quelconque des revendications 1 à 3 à une concentration suffisante pour inhiber la liaison de la neurokinine aux cellules exprimant un récepteur de neurokinine-3 humain cloné *in vitro*.

11. Composition pharmaceutique conditionnée comprenant la composition selon la revendication 5, dans un conteneur et les instructions pour utiliser la composition pour traiter un patient souffrant d'un trouble répondant à l'antagonisme du récepteur de la neurokinine-3.

12. Composition pharmaceutique conditionnée selon la revendication 11, dans laquelle ledit patient souffre d'anxiété, de dépression, de schizophrénie, d'obésité ou de trouble obstructif pulmonaire chronique.

13. Composé selon l'une quelconque des revendications 1 à 3, dans lequel, dans un essai de liaison du récepteur de neurokinine-3 effectué comme établi dans l'exemple IX de la description, le composé présente une CI₅₀ de 1 micromolaire ou inférieure.
